(19) **Europäisches Patentamt / European Patent Office / Office européen des brevets**

(11) **EP 3 642 334 B1**

(12) **EUROPEAN PATENT SPECIFICATION**

(45) Date of publication and mention of the grant of the patent:
**27.12.2023 Bulletin 2023/52**

(21) Application number: **18821213.8**

(22) Date of filing: **25.05.2018**

(51) International Patent Classification (IPC):
*A61K 48/00* (2006.01)   *C12N 15/10* (2006.01)
*C12N 15/11* (2006.01)   *C12N 15/90* (2006.01)
*C12N 9/22* (2006.01)

(52) Cooperative Patent Classification (CPC):
**C12N 15/102; C12N 9/22; C12N 15/111;
C12N 15/90; A61K 48/0016; C12N 2310/20**

(86) International application number:
**PCT/US2018/034779**

(87) International publication number:
**WO 2018/236548 (27.12.2018 Gazette 2018/52)**

(54) **NUCLEIC ACID-GUIDED NUCLEASES**

NUKLEINSÄURE-GEFÜHRTE NUKLEASEN

NUCLÉASES GUIDÉES PAR ACIDE NUCLÉIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.06.2017 US 201715632001
23.06.2017 US 201715631989**

(43) Date of publication of application:
**29.04.2020 Bulletin 2020/18**

(60) Divisional application:
**21167880.0 / 3 916 086**

(73) Proprietor: **Inscripta, Inc.
Pleasanton, CA 94588 (US)**

(72) Inventors:
• **KIM, Juhan
Pleasanton
CA 94566 (US)**
• **GILL, Ryan T.
Denver,
Colorado 80207 (US)**
• **GARST, Andrew
Boulder,
Colorado 80301 (US)**
• **LIPSCOMB, Tanya Elizabeth Warnecke
Boulder,
Colorado 80303 (US)**

(74) Representative: **Mewburn Ellis LLP
Aurora Building
Counterslip
Bristol BS1 6BX (GB)**

(56) References cited:
**WO-A1-2014/093701    WO-A1-2014/093701
WO-A1-2017/096041    WO-A1-2017/096041
WO-A1-2017/223538    US-A1- 2016 208 243
US-A1- 2016 208 243    US-A1- 2018 230 460
US-B1- 9 982 279**

• **BIN LI ET AL: "Engineering CRISPR-Cpf1 crRNAs and mRNAs to maximize genome editing efficiency", NATURE BIOMEDICAL ENGINEERING, vol. 1, no. 5, 10 May 2017 (2017-05-10), pages 1-21, XP055564263, DOI: 10.1038/s41551-017-0066**
• **LI; B et al.: "Engineering CRISPR-Cpf1 crRNAs and mRNAs to maximize genome editing efficiency", Nature Biomedical Engineering, vol. 1, no. 5, 10 May 2017 (2017-05-10), pages 1-21, XP055564263,**

Remarks:
The complete document including Reference Table(s) and the Sequence Listing(s) can be downloaded from the EPO website

**Description**

**BACKGROUND OF THE DISCLOSURE**

Nucleic acid-guided nucleases have become important tools for research and genome engineering. The applicability of these tools can be limited by the sequence specificity requirements, expression, or delivery issues.

**SUMMARY OF THE DISCLOSURE**

The invention is defined by the appended claims.

Figure 1A depicts a partial sequence alignment MAD1-8 (SEQ ID NO: 1-8) and MAD10-12 (SEQ ID NO: 10-12). Figure 1A discloses SEQ ID NO: 721, residues 703-707 of SEQ ID NO: 1, residues 625-629 of SEQ ID NO: 2, residues 587-591 of SEQ ID NO: 3, residues 654-658 of SEQ ID NO: 4, residues 581-585 of SEQ ID NO: 5, residues 637-641 of SEQ ID NO: 6, residues 590-594 of SEQ ID NO: 7, residues 645-649 of SEQ ID NO: 8, SEQ ID NO: 395, residues 619-623 of SEQ ID NO: 10 and residues 603-607 of SEQ ID NO: 12, all respectively, in order of appearance.

Figure 1B depicts a phylogenetic tree of nucleases including MAD 1-8.

Figure 2 depicts an example protein expression construct. Figure 2 discloses "6X-His" as SEQ ID NO: 376.

Figure 3 depicts an example editing cassette and target codons representing the editing sequences SEQ ID NOS 396-398, respectively, in order of appearance.

Figure 4 depicts an example screening or selection experiment workflow.

Figure 5A depicts an example protein expression construct.

Figure 5B depicts an example editing cassette.

Figure 5C depicts an example screening or selection experiment workflow.

Figure 6A depicts an example protein expression construct.

Figure 6B depicts an example editing cassette.

Figure 6C depicts an example screening or selection experiment workflow.

Figure 7 depicts example data from a functional nuclease complex screening or selection experiment.

Figure 8 depicts example data from a targetable nuclease complex-based editing experiment.

Figure 9 depicts example data from a targetable nuclease complex-based editing experiment.

Figures 10A and 10B depict example data from a targetable nuclease complex-based editing experiment.

Figure 11 depicts an example sequence alignment of select sequences from an editing experiment. Figure 11 discloses SEQ ID NOS 399-401, 400, 400, 400, 400, 400, 402, 399-400 and 400, respectively, in order of appearance.

Figure 12 depicts example data from a targetable nuclease complex-based editing experiment.

Figure 13 depicts an example alignment of scaffold sequences. Figure 13 discloses SEQ ID NOS 403-415, respectively, in order of appearance.

Figure 14A-14B depict example data from a primer validation experiment.

Figure 15 depicts example data from a targetable nuclease complex-based editing experiment.

Figure 16 depicts example validation data comparing results from two different assays.

Figure 17A-17C depict an example trackable genetic engineering workflow, including a plasmid comprising an editing cassette and a recording cassette, and downstream sequencing of barcodes in order to identify the incorporated edit or mutation. Figure 17B discloses SEQ ID NOS 416-417, respectively, in order of appearance.

Figures 18 depicts an example trackable genetic engineering workflow, including iterative rounds of engineering with a different editing cassette and recorder cassette with unique barcode (BC) at each round, which can be followed by selection and tracking to confirm the successful engineering step at each round.

Figure 19 depicts an example recursive engineering workflow.

Figure 20 depicts an example subject expression cassette and Figure 20B depicts an example target sequence in a target gene.

Figures 21A and 21B depict examples of measured cell and colony growth data.

Figures 22A and 22B depict example data comparing input and 15 hour time point library quantification.

Figures 23A and 23B depict depletion score data from an example experiment.

Figure 23C depicts target sequences and editing efficiencies from example experiments. Figure 23C discloses SEQ ID NOS 419-423, respectively, in order of appearance.

Figures 24A, 24B and 24C depict depletion score data from an example experiment.

Figure 25 depicts a cartoon representation of subject assays used to determine PAM specificity and off-target effects of subject nuclease systems.

Figures 26A, 26B, and 26C depict depletion score data from example experiments.

Figures 27A and 27B depict depletion score data from example experiments.

Figures 28A and 28B depict depletion score data from example experiments.

Figures 29A and 29B depict depletion score data from example experiments.

Figures 30A and 30B depict depletion score data from example experiments.

Figures 31A, 31B, and 31C depict depletion score data from example experiments.

Figures 32A, 32B, 32C, 32D, 32E, 32F, 32G, and 32H depict depletion score data (negative enrichment scores) from example experiments.

Figures 32I, 32J, 32K, 32L, 32M, 32N, 32O, and 32P depict depletion score data from example experiments.

Figure 33 depicts an example construct and experimental design for determining PAM specificity and guide scaffold sequence characterization and optimization of subject nuclease systems.

Figures 34A and 34B depicts guide nucleic acid scaffold sequence alignments for example MAD crRNA sequences. Figure 34A discloses SEQ ID NOS 639-666, respectively, in order of appearance. Figure 34B discloses SEQ ID NOS 667-675, respectively, in order of appearance.

Figures 35A, 35B, and 35C depict example data characterizing preferred crRNA loop sequences.

Figures 36A, 36B, 36C, 36D, 36E and 36F depict example depletion score data characterizing preferred crRNA loop sequences for MAD7, MAD2 and MAD4, respectively.

Figure 37 depicts depletion score data from example experiments characterizing crRNA stem loop sequence preference.

Figure 38A depicts example expression constructs for use in mammalian cells.

Figure 38B depicts example guide nucleic acid sequences. Figure 38B discloses SEQ ID NOS 677-678, respectively, in order of appearance.

Figures 39A and 39B depict example target sites targeted by the indicated gRNA sequences within the indicated target gene.

Figures 39C and 39D summarize PAM and target sequences of the indicated guide nucleic acids. Figure 38C discloses SEQ ID NOS 679-688, respectively, in order of appearance. Figure 39D discloses SEQ ID NOS 689-698, respectively, in order of appearance.

Figure 40 depicts cleavage (cutting) efficiencies from an *in vitro* cutting assay.

Figure 41 depicts MAD7-dependent (two constructs) indel formation in mammalian HEK293T cells for two genes targeted by three difference guide nucleic acids with two different lengths of scaffold (42- or 56-mer).

## DETAILED DESCRIPTION OF THE DISCLOSURE

The present disclosure provides nucleic acid-guided nucleases and methods of use. Often, the subject nucleic-acid guided nucleases are part of a targetable nuclease system comprising a nucleic acid-guided nuclease and a guide nucleic acid. A subject targetable nuclease system can be used to cleave, modify, and/or edit a target polynucleotide sequence, often referred to as a target sequence. A subject targetable nuclease system refers collectively to transcripts and other elements involved in the expression of or directing the activity of genes, which may include sequences encoding a subject nucleic acid-guided nuclease protein and a guide nucleic acid as disclosed herein.

Methods, systems, vectors, polynucleotides, and compositions described herein may be used in various applications including altering or modifying synthesis of a gene product, such as a protein, polynucleotide cleavage, polynucleotide editing, polynucleotide splicing; trafficking of target polynucleotide, tracing of target polynucleotide, isolation of target polynucleotide, visualization of target polynucleotide, etc.. Aspects of the invention also encompass methods and uses of the compositions and systems described herein in genome engineering, e.g. for altering or manipulating the expression of one or more genes or the one or more gene products, in prokaryotic, archaeal, or eukaryotic cells, *in vitro, in vivo* or ex vivo.

## Nucleic acid-guided nucleases

Bacterial and archaeal targetable nuclease systems have emerged as powerful tools for precision genome editing. However, naturally occurring nucleases have some limitations including expression and delivery challenges due to the nucleic acid sequence and protein size. Targetable nucleases that require PAM recognition are also limited in the sequences they can target throughout a genetic sequence. Other challenges include processivity, target recognition specificity and efficiency, and nuclease acidity efficiency, which often effect genetic editing efficiency.

Non-naturally occurring targetable nucleases and non-naturally occurring targetable nuclease systems can address many of these challenges and limitations.

Disclosed herein are non-naturally targetable nuclease systems. Such targetable nuclease systems are engineered to address one or more of the challenges described above and can be referred to as engineered nuclease systems. Engineered nuclease systems can comprise one or more of an engineered nuclease, such as an engineered nucleic acid-guided nuclease, an engineered guide nucleic acid, an engineered polynucleotides encoding said nuclease, or an engineered polynucleotides encoding said guide nucleic acid. Engineered nucleases, engineered guide nucleic acids, and engineered polynucleotides encoding the engineered nuclease or engineered guide nucleic acid are not naturally occurring and are not found in nature. It follows that engineered nuclease systems including one or more of these elements are non-naturally occurring.

Non-limiting examples of types of engineering that can be done to obtain a non-naturally occurring nuclease system are as follows. Engineering can include codon optimization to facilitate expression or improve expression in a host cell, such as a heterologous host cell. Engineering can reduce the size or molecular weight of the nuclease in order to facilitate expression or delivery. Engineering can alter PAM selection in order to change PAM specificity or to broaden the range of recognized PAMs. Engineering can alter, increase, or decrease stability, processivity, specificity, or efficiency of a targetable nuclease system. Engineering can alter, increase, or decrease protein stability. Engineering can alter, increase, or decrease processivity of nucleic acid scanning. Engineering can alter, increase, or decrease target sequence specificity. Engineering can alter, increase, or decrease nuclease activity. Engineering can alter, increase, or decrease editing efficiency. Engineering can alter, increase, or decrease transformation efficiency. Engineering can alter, increase, or decrease nuclease or guide nucleic acid expression.

Examples of non-naturally occurring nucleic acid sequences which are disclosed herein include sequences codon optimized for expression in bacteria, such as *E. coli* (e.g., SEQ ID NO: 41-60), sequences codon optimized for expression in single cell eukaryotes, such as yeast (e.g., SEQ ID NO: 127-146), sequences codon optimized for expression in multi cell eukaryotes, such as human cells (e.g., SEQ ID NO: 147-166), polynucleotides used for cloning or expression of any sequences disclosed herein (e.g., SEQ ID NO: 61-80), plasmids comprising nucleic acid sequences (e.g., SEQ ID NO: 21-40) operably linked to a heterologous promoter or nuclear localization signal or other heterologous element, proteins generated from engineered or codon optimized nucleic acid sequences (e.g., SEQ ID NO: 1-20), or engineered guide nucleic acids comprising any one of SEQ ID NO: 84-107. Such non-naturally occurring nucleic acid sequences can be amplified, cloned, assembled, synthesized, generated from synthesized oligonucleotides or dNTPs, or otherwise obtained using methods known by those skilled in the art.

Disclosed herein are nucleic acid-guided nucleases. Subject nucleases are functional *in vitro,* or in prokaryotic, archaeal, or eukaryotic cells for *in vitro, in vivo,* or ex vivo applications. Suitable nucleic acid-guided nucleases can be from an organism from a genus which includes but is not limited to Thiomicrospira, Succinivibrio, Candidatus, Porphyromonas, Acidaminococcus, Acidomonococcus, Prevotella, Smithella, Moraxella, Synergistes, Francisella, Leptospira, Catenibacterium, Kandleria, Clostridium, Dorea, Coprococcus, Enterococcus, Fructobacillus, Weissella, Pediococcus, Corynebacter, Sutterella, Legionella, Treponema, Roseburia, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma, Alicyclobacillus, Brevibacilus, Bacillus, Bacteroidetes, Brevibacilus, Carnobacterium, Clostridiaridium, Clostridium, Desulfonatronum, Desulfovibrio, Helcococcus, Leptotrichia, Listeria, Methanomethyophilus, Methylobacterium, Opitutaceae, Paludibacter, Rhodobacter, Sphaerochaeta, Tuberibacillus, Oleiphilus, Omnitrophica, Parcubacteria, and Campylobacter. Species of organism of such a genus can be as otherwise herein discussed. Suitable nucleic acid-guided nucleases can be from an organism from a genus or unclassified genus within a kingdom which includes but is not limited to Firmicute, Actinobacteria, Bacteroidetes, Proteobacteria, Spirochates, and Tenericutes. Suitable nucleic acid-guided nucleases can be from an organism from a genus or unclassified genus within a phylum which includes but is not limited to Erysipelotrichia, Clostridia, Bacilli, Actinobacteria, Bacteroidetes, Flavobacteria, Alphaproteobacteria, Betaproteobacteria, Gammaproteobacteria, Deltaproteobacteria, Epsilonproteobacteria, Spirochaetes, and Mollicutes. Suitable nucleic acid-guided nucleases can be from an organism from a genus or unclassified genus within an order which includes but is not limited to Clostridiales, Lactobacillales, Actinomycetales, Bacteroidales, Flavobacteriales, Rhizobiales, Rhodospirillales, Burkholderiales, Neisseriales, Legionellales, Nautiliales, Campylobacterales, Spirochaetales, Mycoplasmatales, and Thiotrichales. Suitable nucleic acid-guided nucleases can be from an organism from a genus or unclassified genus within a family which includes but is not limited to Lachnospiraceae, Enterococcaceae, Leuconostocaceae, Lactobacillaceae, Streptococcaceae, Peptostreptococcaceae, Staphylococcaceae, Eubacteriaceae, Corynebacterineae, Bacteroidaceae, Flavobacterium, Cryomoorphaceae, Rhodobiaceae, Rhodospirillaceae, Acetobacteraceae, Sutterellaceae, Neisseriaceae, Legionellaceae, Nautiliaceae, Campylobacteraceae, Spirochaetaceae, Mycoplasmataceae, Pisciririckettsiaceae, and Francisellaceae. Other nucleic acid-guided nucleases have been describe in US Patent Application Publication No. US20160208243 filed December 18, 2015, US Application Publication No. US20140068797 filed March 15, 2013, US Patent No. US8697359 filed October 15, 2013, and Zetsche et al., Cell 2015 Oct 22; 163(3):759-71.

Some nucleic acid-guided nucleases suitable for use in the methods, systems, and compositions of the present disclosure include those derived from an organism such as, but not limited to, Thiomicrospira sp. XS5, Eubacterium rectale, Succinivibrio dextrinosolvens, Candidatus Methanoplasma termitum, Candidatus Methanomethylophilus alvus, Porphyromonas crevioricanis, Flavobacterium branchiophilum, Acidaminococcus Sp., Acidomonococcus sp., Lachnospiraceae bacterium COE1, Prevotella brevis ATCC 19188, Smithella sp. SCADC, Moraxella bovoculi, Synergistes jonesii, Bacteroidetes oral taxon 274, Francisella tularensis, Leptospira inadai serovar Lyme str. 10, Acidomonococcus sp. crystal structure (5B43) S. mutans, S. agalactiae, S. equisimilis, S. sanguinis, S. pneumonia; C. jejuni, C. coli; N. salsuginis, N. tergarcus; S. auricularis, S. carnosus; N. meningitides, N. gonorrhoeae; L. monocytogenes, L. ivanovii; C. botulinum, C. difficile, C. tetani, C. sordellii; Francisella tularensis 1, Prevotella albensis, Lachnospiraceae bacterium MC2017 1, Butyrivibrio proteoclasticus, Butyrivibrio proteoclasticus B316, Peregrinibacteria bacterium GW2011_GWA2_33_10, Parcubacteria bacterium GW2011_GWC2_44_17, Smithella sp. SCADC, Acidaminococcus sp. BV3L6, Lachnospiraceae bacterium MA2020, Candidatus Methanoplasma termitum, Eubacterium eligens, Moraxella bovoculi 237, Leptospira inadai, Lachnospiraceae bacterium ND2006, Porphyromonas crevioricanis 3, Prevotella disiens, Porphyromonas macacae, Catenibacterium sp. CAG:290, Kandleria vitulina, Clostridiales bacterium KA00274, Lachnospiraceae bacterium 3-2, Dorea longicatena, Coprococcus catus GD/7, Enterococcus columbae DSM 7374, Fructobacillus sp. EFB-N1, Weissella halotolerans, Pediococcus acidilactici, Lactobacillus curvatus, Streptococcus pyogenes, Lactobacillus versmoldensis, Filifactor alocis ATCC 35896, Alicyclobacillus acidoterrestris, Alicyclobacillus acidoterrestris ATCC 49025, Desulfovibrio inopinatus, Desulfovibrio inopinatus DSM 10711, Oleiphilus sp. Oleiphilus sp. HI0009, Candidtus kefeldibacteria, Parcubacteria CasY.4, Omnitrophica WOR 2 bacterium GWF2, Bacillus sp. NSP2.1, and Bacillus thermoamylovorans.

In the disclosure, the nucleic acid-guided nuclease comprises SEQ ID NO: 7.

In some cases, the nucleic acid-guided nuclease is encoded by the nucleic acid sequence of SEQ ID NO: 27.

In some instances, a nucleic acid-guided nuclease disclosed herein is encoded on a nucleic acid sequence. Such a nucleic acid can be codon optimized for expression in a desired host cell. Suitable host cells can include, as non-limiting examples, prokaryotic cells such as *E. coli, P. aeruginosa, B. subtilus,* and *V. natriegens,* and eukaryotic cells such as *S. cerevisiae,* plant cells such as *Arabidopsis thaliana* or tobacco plant cells, insect cells, nematode cells, amphibian cells, fish cells, or mammalian cells, including human cells.

A nucleic acid sequence encoding a nucleic acid-guided nuclease can be codon optimized for expression in gram positive bacteria, *e.g., Bacillus subtilis,* or gram negative bacteria, *e.g., E. coli.* In some cases, the nucleic acid-guided nuclease is encoded by the nucleic acid sequence comprising at least about 85%, 90%, 95%, greater than 95% sequence identity to SEQ ID NO: 47 or 203-222. In some cases, the nucleic acid-guided nuclease is encoded by the nucleic acid sequence of SEQ ID NO: 47 or 203-222.

A nucleic acid sequence encoding a nucleic acid-guided nuclease can be codon optimized for expression in a species of yeast, *e.g., S. cerevisiae.* In some cases, the nucleic acid-guided nuclease is encoded by the nucleic acid sequence comprising at least about 50%, 60%, 65%, 70%, 75%, 80%, 85%, 90%, 95%, greater than 95% sequence identity to SEQ ID NO: 133 or 183-202. In some cases, the nucleic acid-guided nuclease is encoded by the nucleic acid sequence of SEQ ID NO: 133 or 183-202.

A nucleic acid sequence encoding a nucleic acid-guided nuclease can be codon optimized for expression in mammalian cells. In some cases, the nucleic acid-guided nuclease is encoded by the nucleic acid sequence comprising at least about 85%, 90%, 95%, greater than 95% sequence identity to SEQ ID NO: 153 or 243-262. In some cases, the nucleic acid-guided nuclease is encoded by the nucleic acid sequence of SEQ ID NO: 153 or 243-262.

A nucleic acid sequence encoding a nucleic acid-guided nuclease can be codon optimized for expression in plant cells. In some cases, the nucleic acid-guided nuclease is encoded by the nucleic acid sequence comprising at least about 85%, 90%, 95%, greater than 95% sequence identity to SEQ ID NO: 223-242. In some cases, the nucleic acid-guided nuclease is encoded by the nucleic acid sequence of SEQ ID NO: 223-242.

A nucleic acid sequence encoding a nucleic acid-guided nuclease can be operably linked to a promoter. Such nucleic acid sequences can be linear or circular. The nucleic acid sequences can be comprised on a larger linear or circular nucleic acid sequences that comprises additional elements such as an origin of replication, selectable or screenable marker, terminator, other components of a targetable nuclease system, such as a guide nucleic acid, or an editing or recorder cassette as disclosed herein. These larger nucleic acid sequences can be recombinant expression vectors, as are described in more detail later.

**Guide nucleic acid**

In general, a guide nucleic acid can complex with a compatible nucleic acid-guided nuclease and can hybridize with a target sequence, thereby directing the nuclease to the target sequence. A subject nucleic acid-guided nuclease capable of complexing with a guide nucleic acid can be referred to as a nucleic acid-guided nuclease that is compatible with the guide nucleic acid. Likewise, a guide nucleic acid capable of complexing with a nucleic acid-guided nuclease can be referred to as a guide nucleic acid that is compatible with the nucleic acid-guided nucleases.

A guide nucleic acid can be DNA. A guide nucleic acid can be RNA. A guide nucleic acid can comprise both DNA and RNA. A guide nucleic acid can comprise modified of non-naturally occurring nucleotides. In cases where the guide nucleic acid comprises RNA, the RNA guide nucleic acid can be encoded by a DNA sequence on a polynucleotide molecule such as a plasmid, linear construct, or editing cassette as disclosed herein.

A guide nucleic acid can comprise a guide sequence. A guide sequence is a polynucleotide sequence having sufficient complementarity with a target polynucleotide sequence to hybridize with the target sequence and direct sequence-specific binding of a complexed nucleic acid-guided nuclease to the target sequence. The degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences. In some embodiments, a guide sequence is about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length. In some embodiments, a guide sequence is less than about 75, 50, 45, 40, 35, 30, 25, 20 nucleotides in length. Preferably the guide sequence is 10-30 nucleotides long. The guide sequence can be 15-20 nucleotides in length. The guide sequence can be 15 nucleotides in length. The guide sequence can be 16 nucleotides in length. The guide sequence can be 17 nucleotides in length. The guide sequence can be 18 nucleotides in length. The guide sequence can be 19 nucleotides in length. The guide sequence can be 20 nucleotides in length.

A guide nucleic acid can comprise a scaffold sequence. In general, a "scaffold sequence" includes any sequence that has sufficient sequence to promote formation of a targetable nuclease complex, wherein the targetable nuclease complex comprises a nucleic acid-guided nuclease and a guide nucleic acid comprising a scaffold sequence and a guide sequence. Sufficient sequence within the scaffold sequence to promote formation of a targetable nuclease complex may include a degree of complementarity along the length of two sequence regions within the scaffold sequence, such as one or two sequence regions involved in forming a secondary structure. In some cases, the one or two sequence regions are comprised or encoded on the same polynucleotide. In some cases, the one or two sequence regions are comprised or encoded on separate polynucleotides. Optimal alignment may be determined by any suitable alignment algorithm, and may further account for secondary structures, such as self-complementarity within either the one or two sequence regions. In some embodiments, the degree of complementarity between the one or two sequence regions along the length of the shorter of the two when optimally aligned is about or more than about 25%, 30%, 40%, 50%, 60%, 70%, 80%, 90%, 95%, 97.5%, 99%, or higher. In some embodiments, at least one of the two sequence regions is about or more than about 5, 6, 7, 8, 9, 10, 11, 12, 13, 14, 15, 16, 17, 18, 19, 20, 25, 30, 40, 50, or more nucleotides in length.

A scaffold sequence of a subject guide nucleic acid can comprise a secondary structure. A secondary structure can comprise a pseudoknot region. In some cases, binding kinetics of a guide nucleic acid to a nucleic acid-guided nuclease is determined in part by secondary structures within the scaffold sequence. In some cases, binding kinetics of a guide nucleic acid to a nucleic acid-guided nuclease is determined in part by nucleic acid sequence with the scaffold sequence. An example of a pseudoknot region within a scaffold sequence is depicted in Figure 49C.

A scaffold sequence can comprise a loop sequence. A loop sequence can comprise 1 or more nucleotides. In some examples, the loop sequence comprises 4 nucleotides. In some examples, the loop sequence comprises 5 nucleotides. A loop sequence can be a region of the scaffold sequence that is not hybridized with another sequence or is not hybridized with another sequence within the scaffold sequence. An example of a loop sequence within a scaffold sequence is depicted in Figure 49C.

A scaffold sequence can comprise the sequence of any one of SEQ ID NO: 84-107 or 172-182. A scaffold sequence can comprise the sequence of any one of SEQ ID NO: 84-103. A scaffold sequence can comprise the sequence of any one of SEQ ID NO: 84-91 or 93-95. A scaffold sequence can comprise the sequence of any one of SEQ ID NO: 88, 93, 94, or 95. A scaffold sequence can comprise the sequence of SEQ ID NO: 88. A scaffold sequence can comprise the sequence of SEQ ID NO: 93. A scaffold sequence can comprise the sequence of SEQ ID NO: 94. A scaffold sequence can comprise the sequence of SEQ ID NO: 95. A scaffold sequence can comprise a sequence or consensus sequence depicted in Figure 49A, 49B, or 49C.

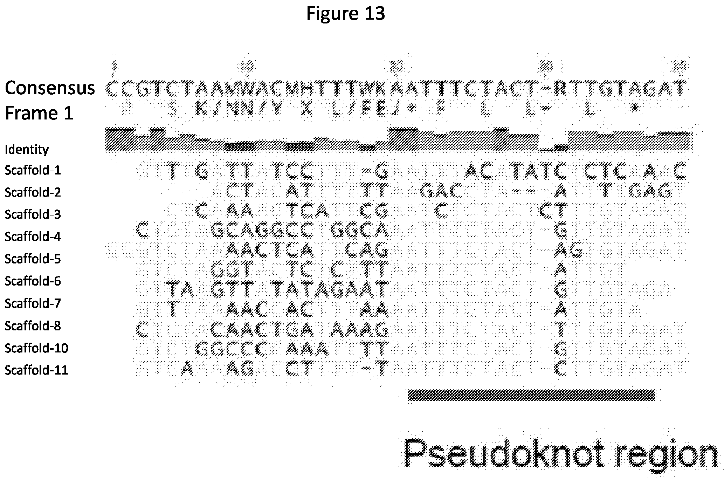

In some aspects, the disclosure provides a nuclease that binds to a guide nucleic acid comprising a conserved scaffold sequence. For example, the nucleic acid-guided nucleases for use in the present disclosure can bind to a conserved pseudoknot region as shown in Figure 13. Specifically, the nucleic acid-guided nucleases for use in the present disclosure can bind to a guide nucleic acid comprising a conserved pseudoknot region as shown in Figure 13. Certain nucleic acid-guided nucleases for use in the present disclosure can bind to a pseudoknot region having at least 75%, 80%, 85%, 90%, 95%, or 100% sequence identity to the pseudoknot region of Scaffold-1 (SEQ ID NO: 172). Other nucleic acid-guided nucleases for use in the present disclosure can bind to a pseudoknot region having at least 75%, 80%, 85%, 90%, 95%, or 100% sequence identity to the pseudoknot region of Scaffold-3 (SEQ ID NO: 173). Still other nucleic acid-guided nucleases for use in the present disclosure can bind to a pseudoknot region having at least 75%, 80%, 85%, 90%, 95%, or 100% sequence identity to the pseudoknot region of Scaffold-4 (SEQ ID NO: 174). Other nucleic acid-guided nucleases for use in the present disclosure can bind to a pseudoknot region having at least 75%, 80%, 85%, 90%, 95%, or 100% sequence identity to the pseudoknot region of Scaffold-5 (SEQ ID NO: 175). Other nucleic acid-guided nucleases for use in the present disclosure can bind to a pseudoknot region having at least 75%, 80%, 85%, 90%, 95%, or 100% sequence identity to the pseudoknot region of Scaffold-6 (SEQ ID NO: 176). Still other nucleic acid-guided nucleases for use in the present disclosure can bind to a pseudoknot region having at least 75%, 80%, 85%, 90%, 95%, or 100% sequence identity to the pseudoknot region of Scaffold-7 (SEQ ID NO: 177). Other nucleic acid-guided nucleases for use in the present disclosure can bind to a pseudoknot region having at least 75%, 80%, 85%, 90%, 95%, or 100% sequence identity to the pseudoknot region of Scaffold-8 (SEQ ID NO: 178). Other nucleic acid-guided nucleases for use in the present disclosure can bind to a pseudoknot region having at least 75%, 80%, 85%, 90%, 95%, or 100% sequence identity to the pseudoknot region of Scaffold-10 (SEQ ID NO: 179). Still other nucleic acid-guided nucleases for use in the present disclosure can bind to a pseudoknot region having at least 75%, 80%, 85%, 90%, 95%, or 100% sequence identity to the pseudoknot region of Scaffold-11 (SEQ ID NO: 180). Certain nucleic acid-guided nucleases for use in the present disclosure can bind to a pseudoknot region having at least 75%, 80%, 85%, 90%, 95%, or 100% sequence identity to the pseudoknot region of Scaffold-12 (SEQ ID NO: 181). Certain nucleic acid-guided nucleases for use in the present disclosure can bind to a pseudoknot region having at least 75%, 80%, 85%, 90%, 95%, or 100% sequence identity to the pseudoknot region of Scaffold-25 (SEQ ID NO: 182).

Certain nucleic acid-guided nucleases for use in the present disclosure can bind to a scaffold sequence having at least 75%, 80%, 85%, 90%, 95%, or 100% sequence identity to of any one of SEQ ID NO: 84-107 or 172-182. Certain nucleic acid-guided nucleases for use in the present disclosure can bind to a scaffold sequence having at least 75%, 80%, 85%, 90%, 95%, or 100% sequence identity to of any one of the sequences depicted in Figures 49A, 49B, or 49C.

Certain nucleic acid-guided nucleases for use in the present disclosure can bind to a scaffold sequence having a loop sequence depicted in Figures 49A, 49B, or 49C. Certain nucleic acid-guided nucleases for use in the present disclosure can bind to a scaffold sequence having a loop sequence of UAUU, UUU, UGUU, UCUU, UCLTLTU, or UAGU.

Certain nucleic acid-guided nucleases for use in the present disclosure can bind to a scaffold sequence comprising the RNA version of SEQ ID NO: 181, wherein T is replaced with U. In some cases, the scaffold sequence comprises at least one mutation compared to the RNA version of SEQ ID NO: 181. For example, the scaffold sequence can comprise an altered sequence in the loop sequence of the scaffold sequence. For example, the loop sequence can comprise UAUU, UUU, UGUU, UCUU, UCUUU, or UAGU.

A guide nucleic acid can comprise the sequence of any one of SEQ ID NO: 84-107. A guide nucleic acid can comprise the sequence of any one of SEQ ID NO: 84-103. A guide nucleic acid can comprise the sequence of any one of SEQ ID NO: 84-91 or 93-95. A guide nucleic acid can comprise the sequence of any one of SEQ ID NO: 88, 93, 94, or 95. A guide nucleic acid can comprise the sequence of SEQ ID NO: 88. A guide nucleic acid can comprise the sequence of SEQ ID NO: 93. A guide nucleic acid can comprise the sequence of SEQ ID NO: 94. A guide nucleic acid can comprise the sequence of SEQ ID NO: 95.

In aspects of the invention the terms "guide nucleic acid" refers to one or more polynucleotides comprising 1) a guide sequence capable of hybridizing to a target sequence and 2) a scaffold sequence capable of interacting with or complexing with an nucleic acid-guided nuclease as described herein. A guide nucleic acid may be provided as one or more nucleic acids. In specific embodiments, the guide sequence and the scaffold sequence are provided as a single polynucleotide.

A guide nucleic acid can be compatible with a nucleic acid-guided nuclease when the two elements can form a functional targetable nuclease complex capable of cleaving a target sequence. Often, a compatible scaffold sequence for a compatible guide nucleic acid can be found by scanning sequences adjacent to a native nucleic acid-guided nuclease locus. In other words, native nucleic acid-guided nucleases can be encoded on a genome within proximity to a corresponding compatible guide nucleic acid or scaffold sequence.

Nucleic acid-guided nucleases can be compatible with guide nucleic acids that are not found within the nucleases endogenous host. Such orthogonal guide nucleic acids can be determined by empirical testing. Orthogonal guide nucleic acids can come from different bacterial species or be synthetic or otherwise engineered to be non-naturally occurring.

Orthogonal guide nucleic acids that are compatible with a common nucleic acid-guided nuclease can comprise one or more common features. Common features can include sequence outside a pseudoknot region. Common features can include a pseudoknot region. Common features can include a primary sequence or secondary structure.

A guide nucleic acid can be engineered to target a desired target sequence by altering the guide sequence such that the guide sequence is complementary to the target sequence, thereby allowing hybridization between the guide sequence and the target sequence. A guide nucleic acid with an engineered guide sequence can be referred to as an engineered guide nucleic acid. Engineered guide nucleic acids are often non-naturally occurring and are not found in nature.

Compatible guide nucleic acids or scaffold sequences for a subject nuclease can be identified or tested using a screening assay as disclosed herein. In general, a library of vectors can be generated as disclosed herein, said vector comprising a target sequence adjacent to a PAM sequence. Said vector can comprise a selectable marker or screenable marker. Said vector can comprise a guide nucleic acid sequence to be tested. Said vector can comprise a barcode or other unique identifier that allows identification of the guide nucleic acid to be tested. Said guide nucleic acid can comprise a targeting sequence capable of targeting the target sequence of the vector. Said guide nucleic acid can comprise a scaffold sequence. In general, within a library of such vector, the scaffold sequence would vary between the different vectors such that numerous different scaffold sequences could be screened or tested within a single experiment or a high throughput manner. In some cases, a stem loop sequence within the scaffold sequence is varied between the different vectors such that numerous different scaffold sequences could be screened or tested within a single experiment or a high throughput manner. In some cases, the vector library comprises a variety of different PAM sequences adjacent to the target sequence. The vector library can then be introduced into host cells, said host cells comprising a subject nuclease. Said subject nuclease can be expressed from the same or different vector that is introduced into the cell either concomitantly, prior to, or subsequent to the guide nucleic acid vector. In other cases, the subject nuclease can be introduced to the cell as a mRNA transcript. In other cases, the subject nuclease can be introduces to the cell as a protein. Without wishing to be bound by theory, within each cell, the guide nucleic acid would be expressed. If the expressed guide nucleic acid is compatible with the subject nuclease, then the guide nucleic acid would complex with the subject nuclease and target the nuclease to the target sequence, and then the nuclease would cleave the target sequence. This cleavage event would cause the host cell to lose the vector comprising the target sequence or to lose the function of the selectable marker or screenable marker, and therefore the host cell would die under a selection or be lost during screening. On the other hand, if the guide nucleic acid is not compatible with the subject nuclease, then the target sequence would not be cleaved, and thus the host cell would maintain the selectable or screenable marker. By comparing the input vectors to the selected or screened for vectors from the surviving or selected output host cells, one can identify vectors that have been depleted. By sequencing or analyzing the barcode or unique identifier of the input vectors and output vectors, one can identify the barcodes or unique identifiers that have been depleted, which would allow for identification of the guide nucleic acids that were depleted. The depleted guide nucleic acids would include those that are compatible with the subject nuclease.

In some cases, when performing the guide nucleic acid screening or testing assay described herein, the assay can also be used to identify or screen for PAM sequences that are compatible with the subject nuclease. In such cases, the vectors within the vector library can comprise a barcode or unique identifier adjacent to the PAM. By comparing the input vectors from the output vectors, one can identify the PAM sequences that have been depleted. The depleted PAM sequences would include those that are compatible with the subject nuclease. In some cases, by use of the assays described herein, compatible guide nucleic acids and PAM sequences for a subject nuclease can be identified or tested within a single experiment or screen or in a high throughput manner.

Depletion can refer to a decrease in the subject sequence relative to a starting frequency or a reference sequence frequency. Depletion is the converse of enrichment and can therefore also be expressed as a negative enrichment value. Depletion can be calculated using any known method in the field. In some cases, depletion scores can be calculated by calculating the frequency of each sequence or construct within the experimental data and compare the subject frequency to a control, wherein the depletion is the level of change of the subject frequency to the control or reference frequency. In some cases, a threshold frequency is used in order to reduce noise, for example there may be a cutoff of at least 50 counts before the data will be used in the depletion score calculation. A mean and standard deviation is calculated for sequences that show no change relative to a control or reference frequency. This mean and standard deviation can be used to derive z-scores that can yield p-values of <0.05 to infer a significance threshold. In some cases, in order to correct for potential over-estimation of a depletion score, a cut-off is used and depletion scores that pass that threshold are considered depleted. In some cases, the threshold can be $\log_2$ -1, $\log_2$ -2, $\log_2$ -3, $\log_2$ -4, or $\log_2$ -5.

In some examples, a depletion scores can be calculated as the log2 depletion score using the following equation: W = log2(Fx,f/Fx,i); where Fx,f is the frequency of cassette X at the final time point and Fx,i is the initial frequency of cassette X, and W is the absolute fitness of each variant. Frequencies were determined by dividing the read counts for each variant by the total experimental counts including those that were lost to filtering. Count weighted averages of multiple replicates can be used to infer the average fitness score of each mutation as follows:

$$\mathrm{Wavg} = (\Sigma^{N}_{i=1} \, \mathrm{counts}_i * W_i) \, / \, (\Sigma^{N}_{i=1} \, \mathrm{counts}_i)$$

. Calculated scores can be referred to as a depletion score when the calculated value is negative, and can also be referred to as an enrichment score if the calculated value is positive.

**Targetable nuclease system**

Disclosed herein are targetable nuclease systems. A targetable nuclease system can comprise a nucleic acid-guided nuclease and a compatible guide nucleic acid. A targetable nuclease system can comprise a nucleic acid-guided nuclease or a polynucleotide sequence encoding the nucleic acid-guided nuclease. A targetable nuclease system can comprise a guide nucleic acid or a polynucleotide sequence encoding the guide nucleic acid.

In general, a targetable nuclease system as disclosed herein is characterized by elements that promote the formation of a targetable nuclease complex at the site of a target sequence, wherein the targetable nuclease complex comprises a nucleic acid-guided nuclease and a guide nucleic acid.

A guide nucleic acid together with a nucleic acid-guided nuclease forms a targetable nuclease complex which is capable of binding to a target sequence within a target polynucleotide, as determined by the guide sequence of the guide nucleic acid.

In general, to generate a double stranded break, in most cases a targetable nuclease complex binds to a target sequence as determined by the guide nucleic acid, and the nuclease has to recognize a protospacer adjacent motif (PAM) sequence adjacent to the target sequence.

A targetable nuclease complex can comprise a nucleic acid-guided nuclease of SEQ ID NO: 7 and a compatible guide nucleic acid. In any of these cases, the guide nucleic acid can comprise a scaffold sequence compatible with the nucleic acid-guided nuclease. In any of these cases, the scaffold sequence can be a native scaffold sequence or a heterologous scaffold sequence. In any of these cases, the guide nucleic acid can further comprise a guide sequence. The guide sequence can be engineered to target any desired target sequence. The guide sequence can be engineered to be complementary to any desired target sequence. The guide sequence can be engineered to hybridize to any desired target sequence.

A targetable nuclease complex can comprise a nucleic acid-guided nuclease of SEQ ID NO: 7 and a compatible guide nucleic acid. A targetable nuclease complex can comprise a nucleic acid-guided nuclease of SEQ ID NO: 7 and a compatible guide nucleic acid comprising any one of SEQ ID NO: 84-107 or 172-182. A targetable nuclease complex can comprise a nucleic acid-guided nuclease of SEQ ID NO: 7 and a compatible guide nucleic acid comprising any one of SEQ ID NO: 88, 93, 94, or 95. A targetable nuclease complex can comprise a nucleic acid-guided nuclease of SEQ ID NO: 7 and a compatible guide nucleic acid comprising SEQ ID NO: 88. A targetable nuclease complex can comprise a nucleic acid-guided nuclease of SEQ ID NO: 7 and a compatible guide nucleic acid comprising SEQ ID NO: 93. A targetable nuclease complex can comprise a nucleic acid-guided nuclease of SEQ ID NO: 7 and a compatible guide nucleic acid comprising SEQ ID NO: 94. A targetable nuclease complex can comprise a nucleic acid-guided nuclease of SEQ ID NO: 7 and a compatible guide nucleic acid comprising SEQ ID NO: 95. A targetable nuclease complex can comprise a nucleic acid-guided nuclease of SEQ ID NO: 7 and a compatible guide nucleic acid comprising SEQ ID NO: 172. A targetable nuclease complex can comprise a nucleic acid-guided nuclease of SEQ ID NO: 7 and a compatible guide nucleic acid comprising SEQ ID NO: 173. A targetable nuclease complex can comprise a nucleic acid-guided nuclease of SEQ ID NO: 7 and a compatible guide nucleic acid comprising SEQ ID NO: 174. A targetable nuclease complex can comprise a nucleic acid-guided nuclease of SEQ ID NO: 7 and a compatible guide nucleic acid comprising SEQ ID NO: 175. A targetable nuclease complex can comprise a nucleic acid-guided nuclease of SEQ ID NO: 7 and a compatible guide nucleic acid comprising SEQ ID NO: 176. A targetable nuclease complex can comprise a nucleic acid-guided nuclease of SEQ ID NO: 7 and a compatible guide nucleic acid comprising SEQ ID NO: 177. A targetable nuclease complex can comprise a nucleic acid-guided nuclease of SEQ ID NO: 7 and a compatible guide nucleic acid comprising SEQ ID NO: 178. A targetable nuclease complex can comprise a nucleic acid-guided nuclease of SEQ ID NO: 7 and a compatible guide nucleic acid comprising SEQ ID NO: 179. A targetable nuclease complex can comprise a nucleic acid-guided nuclease of SEQ ID NO: 7 and a compatible guide nucleic acid comprising SEQ ID NO: 180. A targetable nuclease complex can comprise a nucleic acid-guided nuclease of SEQ ID NO: 7 and a compatible guide nucleic acid comprising SEQ ID NO: 181. A targetable nuclease complex can comprise a nucleic acid-guided nuclease of SEQ ID NO: 7 and a compatible guide nucleic acid comprising SEQ ID NO: 182. In any of these cases, the guide nucleic acid can further comprise a guide sequence. The guide sequence can be engineered to target any desired target sequence. The guide sequence can be engineered to be complementary to any desired target sequence. The guide sequence can be engineered to hybridize to any desired target sequence.

A targetable nuclease complex can comprise a subject targetable nuclease and a guide nucleic acid. In some cases, the guide nucleic acid is selected based on its compatibility with the subject nuclease. Methods of determining and selecting compatible guide nucleic acids are disclosed herein and described in more detail elsewhere. In some cases, the guide nucleic acid is selected based on the cleavage efficiency it confers to the targetable nuclease complex. Cleavage efficiency can be the frequency of cutting a target nucleic acid. In some cases, the guide nucleic acid is selected based on the targeting efficiency it confers to the targetable nuclease complex. Targeting efficiency can be the frequency of targeting an intended target nucleic acid. In some cases, the guide nucleic acid is selected based on the cleavage specificity it confers to the targetable nuclease complex. Cleavage specificity can be the frequency of cleaving the intended target sequence compared to cleaving unintended target sequences. In some cases, the guide nucleic acid is selected based on the targeting specificity it confers to the targetable nuclease complex. Targeting specificity can be the frequency of targeting the intended target sequence compared to targeting unintended target sequences.

Characteristics such as cleavage specificity, cleavage efficiency, targeting specificity, or targeting efficiency can be determined based on characteristics of the guide nucleic acid. For example, the scaffold sequence, pseudoknot region, or loop sequence can each or in combination affect the targeting or cleavage characteristics of a subject nuclease complex. In some examples, portions of the scaffold sequence that interact with the nuclease affect the cleavage or targeting specificity or efficiency of the complex. In some cases, the guide sequence (sometimes referred to as the targeting sequence) of the guide nucleic acid affects the cleavage or targeting specificity or efficiency of the complex. In some examples, selection of the target sequence affects the cleavage or targeting specificity or efficiency of the complex. For example, mismatches between the guide sequence and the target sequence can affect cleavage or targeting specificity or efficiency of the complex. In some cases, the location of the mismatch relative to the PAM can affect cleavage or targeting specificity or efficiency of the complex. In some cases, the number of mismatches or spacing of the mismatches relative to each other can affect cleavage or targeting specificity or efficiency of the complex. In any of these cases, the recited parameter can either increase or decrease the targeting specificity, targeting efficiency, cleavage specificity, or cleavage efficiency of the subject targetable nuclease complex.

A target sequence of a targetable nuclease complex can be any polynucleotide endogenous or exogenous to a prokaryotic or eukaryotic cell, or *in vitro.* For example, the target sequence can be a polynucleotide residing in the nucleus of the eukaryotic cell. A target sequence can be a sequence coding a gene product (e.g., a protein) or a non-coding sequence (e.g., a regulatory polynucleotide or a junk DNA). Without wishing to be bound by theory, it is believed that the target sequence should be associated with a PAM; that is, a short sequence recognized by a targetable nuclease complex. The precise sequence and length requirements for a PAM differ depending on the nucleic acid-guided nuclease used, but PAMs are typically 2-5 base pair sequences adjacent the target sequence. Examples of PAM sequences are given in the examples section below, and the skilled person will be able to identify further PAM sequences for use with a given nucleic acid-guided nuclease. Further, engineering of the PAM Interacting (PI) domain may allow programming of PAM specificity, improve target site recognition fidelity, and increase the versatility of a nucleic acid-guided nuclease genome engineering platform. Nucleic acid-guided nucleases may be engineered to alter their PAM specificity, for example as described in Kleinstiver B P et al. Engineered CRISPR-Cas9 nucleases with altered PAM specificities. Nature. 2015 Jul. 23; 523 (7561): 481-5. doi: 10.1038/nature14592.

A PAM site is a nucleotide sequence in proximity to a target sequence. In most cases, a nucleic acid-guided nuclease can only cleave a target sequence if an appropriate PAM is present. PAMs are nucleic acid-guided nuclease-specific and can be different between two different nucleic acid-guided nucleases. A PAM can be 5' or 3' of a target sequence. A PAM can be upstream or downstream of a target sequence. A PAM can be 1, 2, 3, 4, 5, 6, 7, 8, 9, 10 or more nucleotides in length. Often, a PAM is between 2-6 nucleotides in length.

PAM sites compatible with a subject nuclease or targetable nuclease complex can be identified using methods disclosed herein. In general, a library of vectors can be generated as disclosed herein, said vector comprising a target sequence adjacent to a PAM sequence. Within a library of such vector, the PAM sequence can vary between the different vectors such that numerous different PAM sequences can be screened or tested within a single experiment or in a high throughput manner. Said vector can comprise a barcode or other unique identifier that allows identification of the PAM to be tested. Said vector can comprise a selectable marker or screenable marker. Said vector can comprise a guide nucleic acid sequence. Said guide nucleic acid can comprise a targeting sequence capable of targeting the target sequence of the vector. Said guide nucleic acid can comprise a scaffold sequence. In some cases, the vector library comprises a variety of different guide nucleic acid sequence or scaffold sequences. The vector library can then be introduced into host cells, said host cells comprising a subject nuclease. Said subject nuclease can be expressed from the same or different vector that is introduced into the cell either concomitantly, prior to, or subsequent to the guide nucleic acid vector. In other cases, the subject nuclease can be introduced to the cell as a mRNA transcript. In other cases, the subject nuclease can be introduces to the cell as a protein. Without wishing to be bound by theory, within each cell, the guide nucleic acid would be expressed and would complex with the subject nuclease. Then the guide nucleic acid can target the nuclease to the target sequence. In some cases, if the PAM adjacent to the target sequence is compatible with the subject nuclease, then the subject nuclease can cleave the target sequence. This cleavage event would cause the host cell to lose the vector comprising the target sequence or to lose the function of the selectable marker or screenable marker, and therefore the host cell would die under a selection or be lost during screening. On the other hand, if the PAM is not compatible with the subject nuclease, then the target sequence would not be cleaved, and thus the host cell would maintain the selectable or screenable marker. By comparing the input vectors to the selected or screened for vectors from the surviving or selected output host cells, one can identify vectors that have been depleted. By sequencing or analyzing the barcode or unique identifier of the input vectors and output vectors, one can identify the barcodes or unique identifiers that have been depleted, which would allow for identification of the PAM sequences that were depleted. The depleted PAM sequences would include those that are compatible with the subject nuclease.

In some cases, when performing the PAM screening or testing assay described herein, the assay can also be used to identify or screen for guide nucleic acid sequences that are compatible with the subject nuclease. In such cases, the vectors within the vector library can comprise a barcode or unique identifier adjacent to the guide nucleic acid. By comparing the input vectors from the output vectors, one can identify the guide nucleic acid sequences that have been depleted. The depleted guide nucleic acid sequences would include those that are compatible with the subject nuclease. In some cases, by use of the assays described herein, compatible guide nucleic acids and PAM sequences for a subject nuclease can be identified or tested within a single experiment or screen or in a high throughput manner. Methods for calculating and assessing depletion as described previously are applicable to calculation and assessment of depletion in these cases as well.

In some examples, a PAM can be provided on a separate oligonucleotide. In such cases, providing PAM on an oligonucleotide allows cleavage of a target sequence that otherwise would not be able to be cleave because no adjacent PAM is present on the same polynucleotide as the target sequence.

Polynucleotide sequences encoding a component of a targetable nuclease system can comprise one or more vectors. In general, the term "vector" refers to a nucleic acid molecule capable of transporting another nucleic acid to which it has been linked. Vectors include, but are not limited to, nucleic acid molecules that are single-stranded, double-stranded, or partially double-stranded; nucleic acid molecules that comprise one or more free ends, no free ends (e.g. circular); nucleic acid molecules that comprise DNA, RNA, or both; and other varieties of polynucleotides known in the art. One type of vector is a "plasmid," which refers to a circular double stranded DNA loop into which additional DNA segments can be inserted, such as by standard molecular cloning techniques. Another type of vector is a viral vector, wherein virally-derived DNA or RNA sequences are present in the vector for packaging into a virus (e.g. retroviruses, replication defective retroviruses, adenoviruses, replication defective adenoviruses, and adeno-associated viruses). Viral vectors also include polynucleotides carried by a virus for transfection into a host cell. Certain vectors are capable of autonomous replication in a host cell into which they are introduced (e.g. bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors." Common expression vectors of utility in recombinant DNA techniques are often in the form of plasmids. Further discussion of vectors is provided herein.

Recombinant expression vectors can comprise a nucleic acid of the invention in a form suitable for expression of the nucleic acid in a host cell, which means that the recombinant expression vectors include one or more regulatory elements, which may be selected on the basis of the host cells to be used for expression, that is operatively-linked to the nucleic acid sequence to be expressed. Within a recombinant expression vector, "operably linked" is intended to mean that the nucleotide sequence of interest is linked to the regulatory element(s) in a manner that allows for expression of the nucleotide sequence (e.g. in an *in vitro* transcription/translation system or in a host cell when the vector is introduced into the host cell). With regards to recombination and cloning methods, mention is made of U.S. patent application Ser. No. 10/815,730, published Sep. 2, 2004 as US 2004-0171156 A1.

In some cases, a regulatory element is operably linked to one or more elements of a targetable nuclease system so as to drive expression of the one or more components of the targetable nuclease system.

In some cases, a vector comprises a regulatory element operably linked to a polynucleotide sequence encoding a nucleic acid-guided nuclease. The polynucleotide sequence encoding the nucleic acid-guided nuclease can be codon optimized for expression in particular cells, such as prokaryotic or eukaryotic cells. Eukaryotic cells can be yeast, fungi, algae, plant, animal, or human cells. Eukaryotic cells may be those of or derived from a particular organism, such as a mammal, including but not limited to human, mouse, rat, rabbit, dog, or non-human mammal including non-human primate.

In general, codon optimization refers to a process of modifying a nucleic acid sequence for enhanced expression in the host cells of interest by replacing at least one codon (e.g. about or more than about 1, 2, 3, 4, 5, 10, 15, 20, 25, 50, or more codons) of the native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the "Codon Usage Database" available at www.kazusa.orjp/codon/ (visited Jul. 9, 2002), and these tables can be adapted in a number of ways. See Nakamura, Y., et al. "Codon usage tabulated from the international DNA sequence databases: status for the year 2000" Nucl. Acids Res. 28:292 (2000). Computer algorithms for codon optimizing a particular sequence for expression in a particular host cell are also available, such as Gene Forge (Aptagen; Jacobus, Pa.), are also available. In some cases, one or more codons (e.g. 1, 2, 3, 4, 5, 10, 15, 20, 25, 50, or more, or all codons) in a sequence encoding an engineered nuclease correspond to the most frequently used codon for a particular amino acid.

In some cases, a vector encodes a nucleic acid-guided nuclease comprising one or more nuclear localization sequences (NLSs), such as about or more than about 1, 2, 3, 4, 5, 6, 7, 8, 9, 10, or more NLSs. In some cases, the engineered nuclease comprises about or more than about 1, 2, 3, 4, 5, 6, 7, 8, 9, 10, or more NLSs at or near the amino-terminus, about or more than about 1, 2, 3, 4, 5, 6, 7, 8, 9, 10, or more NLSs at or near the carboxy-terminus, or a combination of these (e.g. one or more NLS at the amino-terminus and one or more NLS at the carboxy terminus). When more than one NLS is present, each may be selected independently of the others, such that a single NLS may be present in more than one copy and/or in combination with one or more other NLSs present in one or more copies. In a preferred case, the engineered nuclease comprises at most 6 NLSs. In some cases, an NLS is considered near the N- or C-terminus when the nearest amino acid of the NLS is within about 1, 2, 3, 4, 5, 10, 15, 20, 25, 30, 40, 50, or more amino acids along the polypeptide chain from the N- or C-terminus. Non-limiting examples of NLSs include an NLS sequence derived from: the NLS of the SV40 virus large T-antigen, having the amino acid sequence PKKKRKV (SEQ ID NO: 111); the NLS from nucleoplasmin (e.g. the nucleoplasmin bipartite NLS with the sequence KRPAATKKAGQAKKKK (SEQ ID NO:112)); the c-myc NLS having the amino acid sequence PAAKRVKLD (SEQ ID NO:113) or RQRRNELKRSP (SEQ ID NO:114); the hRNPA1 M9 NLS having the sequence NQSSNFGPMKGGNFGGRSSGPYGGGGGQYFAKPRNQGGY (SEQ ID NO: 115); the sequence RMRIZFKNKGKDTAELRRRVEVSVELRKAKKDEQILKRRNV (SEQ ID NO:1 116) of the IBB domain from importin-alpha; the sequences VSRKRPRP (SEQ ID NO:117) and PPKKARED (SEQ ID NO:118) of the myoma T protein; the sequence PQPKKKPL (SEQ ID NO:119) of human p53; the sequence SALIKKKKKMAP (SEQ ID NO:120) of mouse c-abl IV; the sequences DRLRR (SEQ ID NO:121) and PKQKKRK (SEQ ID NO:122) of the influenza virus NS1; the sequence RKLKKKIKKL (SEQ ID NO:123) of the Hepatitis virus delta antigen; the sequence REKKKFLKRR (SEQ ID NO: 124) of the mouse Mx1 protein; the sequence KRKGDEVDGVDEVAKKKSKK (SEQ ID NO: 125) of the human poly(ADP-ribose) polymerase; and the sequence RKCLQAGMNLEARKTKK (SEQ ID NO: 126) of the steroid hormone receptors (human) glucocorticoid.

In general, the one or more NLSs are of sufficient strength to drive accumulation of the nucleic acid-guided nuclease in a detectable amount in the nucleus of a eukaryotic cell. In general, strength of nuclear localization activity may derive from the number of NLSs, the particular NLS(s) used, or a combination of these factors. Detection of accumulation in the nucleus may be performed by any suitable technique. For example, a detectable marker may be fused to the nucleic acid-guided nuclease, such that location within a cell may be visualized, such as in combination with a means for detecting the location of the nucleus (e.g. a stain specific for the nucleus such as DAPI). Cell nuclei may also be isolated from cells, the contents of which may then be analyzed by any suitable process for detecting protein, such as immunohistochemistry, Western blot, or enzyme activity assay. Accumulation in the nucleus may also be determined indirectly, such as by an assay for the effect of the nucleic acid-guided nuclease complex formation (e.g. assay for DNA cleavage or mutation at the target sequence, or assay for altered gene expression activity affected by targetable nuclease complex formation and/or nucleic acid-guided nuclease activity), as compared to a control not exposed to the nucleic acid-guided nuclease or targetable nuclease complex, or exposed to a nucleic acid-guided nuclease lacking the one or more NLSs.

A nucleic acid-guided nuclease and one or more guide nucleic acids can be delivered either as DNA or RNA. Delivery of an nucleic acid-guided nuclease and guide nucleic acid both as RNA (unmodified or containing base or backbone modifications) molecules can be used to reduce the amount of time that the nucleic acid-guided nuclease persist in the cell. This may reduce the level of off-target cleavage activity in the target cell. Since delivery of a nucleic acid-guided nuclease as mRNA takes time to be translated into protein, it might be advantageous to deliver the guide nucleic acid several hours following the delivery of the nucleic acid-guided nuclease mRNA, to maximize the level of guide nucleic acid available for interaction with the nucleic acid-guided nuclease protein. In other cases, the nucleic acid-guided nuclease mRNA and guide nucleic acid are delivered concomitantly. In other examples, the guide nucleic acid is delivered sequentially, such as 0.5, 1, 2, 3, 4, or more hours after the nucleic acid-guided nuclease mRNA.

In situations where guide nucleic acid amount is limiting, it may be desirable to introduce a nucleic acid-guided nuclease as mRNA and guide nucleic acid in the form of a DNA expression cassette with a promoter driving the expression of the guide nucleic acid. This way the amount of guide nucleic acid available will be amplified via transcription.

Guide nucleic acid in the form of RNA or encoded on a DNA expression cassette can be introduced into a host cell comprising a nucleic acid-guided nuclease encoded on a vector or chromosome. The guide nucleic acid may be provided in the cassette one or more polynucleotides, which may be contiguous or non-contiguous in the cassette. In specific cases, the guide nucleic acid is provided in the cassette as a single contiguous polynucleotide.

A variety of delivery systems can be used to introduce a nucleic acid-guided nuclease (DNA or RNA) and guide nucleic acid (DNA or RNA) into a host cell. These include the use of yeast systems, lipofection systems, microinjection systems, biolistic systems, virosomes, liposomes, immunoliposomes, polycations, lipid:nucleic acid conjugates, virions, artificial virions, viral vectors, electroporation, cell permeable peptides, nanoparticles, nanowires (Shalek et al., Nano Letters, 2012), exosomes. Molecular trojan horses liposomes (Pardridge et al., Cold Spring Harb Protoc; 2010; doi:10.1101/pdb.prot5407) may be used to deliver an engineered nuclease and guide nuclease across the blood brain barrier.

In some cases, an editing template is also provided. An editing template may be a component of a vector as described herein, contained in a separate vector, or provided as a separate polynucleotide, such as an oligonucleotide, linear polynucleotide, or synthetic polynucleotide. In some cases, an editing template is on the same polynucleotide as a guide nucleic acid. In some embodiments, an editing template is designed to serve as a template in homologous recombination, such as within or near a target sequence nicked or cleaved by a nucleic acid-guided nuclease as a part of a complex as disclosed herein. A editing template polynucleotide may be of any suitable length, such as about or more than about 10, 15, 20, 25, 50, 75, 100, 150, 200, 500, 1000, or more nucleotides in length. In some cases, the editing template polynucleotide is complementary to a portion of a polynucleotide comprising the target sequence. When optimally aligned, an editing template polynucleotide might overlap with one or more nucleotides of a target sequences (e.g. about or more than about 1, 5, 10, 15, 20, 25, 30, 35, 40, or more nucleotides). In some cases, when a editing template sequence and a polynucleotide comprising a target sequence are optimally aligned, the nearest nucleotide of the template polynucleotide is within about 1, 5, 10, 15, 20, 25, 50, 75, 100, 200, 300, 400, 500, 1000, 5000, 10000, or more nucleotides from the target sequence.

In many examples, an editing template comprises at least one mutation compared to the target sequence. An editing template can comprise an insertion, deletion, modification, or any combination thereof compared to the target sequence. Examples of some editing templates are described in more detail in a later section.

In some aspects, the disclosure provides methods comprising delivering one or more polynucleotides, such as or one or more vectors or linear polynucleotides as described herein, one or more transcripts thereof, and/or one or proteins transcribed therefrom, to a host cell. In some aspects, the disclosure further provides cells produced by such methods, and organisms comprising or produced from such cells. In some cases, an engineered nuclease in combination with (and optionally complexed with) a guide nucleic acid is delivered to a cell.

Conventional viral and non-viral based gene transfer methods can be used to introduce nucleic acids in cells, such as prokaryotic cells, eukaryotic cells, mammalian cells, or target tissues. Such methods can be used to administer nucleic acids encoding components of an engineered nucleic acid-guided nuclease system to cells in culture, or in a host organism. Non-viral vector delivery systems include DNA plasmids, RNA (e.g. a transcript of a vector described herein), naked nucleic acid, and nucleic acid complexed with a delivery vehicle, such as a liposome. Viral vector delivery systems include DNA and RNA viruses, which have either episomal or integrated genomes after delivery to the cell. For a review of gene therapy procedures, see Anderson, Science 256:808-813 (1992); Nabel & Feigner, TIBTECH 11:211-217 (1993); Mitani & Caskey, TIBTECH 11:162-166 (1993); Dillon. TIBTECH 11:167-175 (1993); Miller, Nature 357:455-460 (1992); Van Brunt, Biotechnology 6(10):1149-1154 (1988); Vigne, Restorative Neurology and Neuroscience 8:35-36 (1995); Kremer & Perricaudet, British Medical Bulletin 51(1):31-44 (1995); Haddada et al., in Current Topics in Microbiology and Immunology Doerfler and Bohm (eds) (1995); and Yu et al., Gene Therapy 1:13-26 (1994).

Methods of non-viral delivery of nucleic acids include lipofection, microinjection, biolistics, virosomes, liposomes, immunoliposomes, polycation or lipid:nucleic acid conjugates, naked DNA, artificial virions, and agent-enhanced uptake of DNA. Lipofection is described in e.g., U.S. Pat. Nos. 5,049,386, 4,946,787; and 4,897,355) and lipofection reagents are sold commercially (e.g., Transfectam™ and Lipofectin™). Cationic and neutral lipids that are suitable for efficient receptor-recognition lipofection of polynucleotides include those of Felgner, WO 91/17424; WO 91/16024. Delivery can be to cells (e.g. *in vitro* or ex vivo administration) or target tissues (e.g. *in vivo* administration).

The preparation of lipid:nucleic acid complexes, including targeted liposomes such as immunolipid complexes, is well known to one of skill in the art (see, e.g., Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5:647-654 (1994); Gao et al., Gene Therapy 2:710-722 (1995); Ahmad et al., Cancer Res. 52:4817-4820 (1992); U.S. Pat. Nos. 4,186,183, 4,217,344, 4,235,871, 4,261,975, 4,485,054, 4,501,728, 4,774,085, 4,837,028, and 4,946,787).

The use of RNA or DNA viral based systems for the delivery of nucleic acids take advantage of highly evolved processes for targeting a virus to specific cells in culture or in the host and trafficking the viral payload to the nucleus or host cell genome. Viral vectors can be administered directly to cells in culture, patients (*in vivo*), or they can be used to treat cells *in vitro,* and the modified cells may optionally be administered to patients (ex vivo). Conventional viral based systems could include retroviral, lentivirus, adenoviral, adeno-associated and herpes simplex virus vectors for gene transfer. Integration in the host genome is possible with the retrovirus, lentivirus, and adeno-associated virus gene transfer methods, often resulting in long term expression of the inserted transgene. Additionally, high transduction efficiencies have been observed in many different cell types and target tissues.

The tropism of a retrovirus can be altered by incorporating foreign envelope proteins, expanding the potential target population of target cells. Lentiviral vectors are retroviral vectors that are able to transduce or infect non-dividing cells and typically produce high viral titers. Selection of a retroviral gene transfer system would therefore depend on the target tissue. Retroviral vectors are comprised of cis-acting long terminal repeats with packaging capacity for up to 6-10 kb of foreign sequence. The minimum cis-acting LTRs are sufficient for replication and packaging of the vectors, which are then used to integrate the therapeutic gene into the target cell to provide permanent transgene expression. Widely used retroviral vectors include those based upon murine leukemia virus (MuLV), gibbon ape leukemia virus (GaLV), Simian Immuno deficiency virus (SIV), human immuno deficiency virus (HIV), and combinations thereof (see, e.g., Buchscher et al., J. Virol. 66:2731-2739 (1992); Johann et al., J. Virol. 66: 1635-1640 (1992); Sommnerfelt et al., Virol. 176:58-59 (1990); Wilson et al., J. Virol. 63:2374-2378 (1989); Miller et al., J. Virol. 65:2220-2224 (1991); PCT/US94/05700).

In applications where transient expression is preferred, adenoviral based systems may be used. Adenoviral based vectors are capable of very high transduction efficiency in many cell types and do not require cell division. With such vectors, high titer and levels of expression have been obtained. This vector can be produced in large quantities in a relatively simple system.

Adeno-associated virus ("AAV") vectors may also be used to transduce cells with target nucleic acids, e.g., in the *in vitro* production of nucleic acids and peptides, and for *in vivo* and ex vivo gene therapy procedures (see, e.g., West et al., Virology 160:38-47 (1987); U.S. Pat. No. 4,797,368; WO 93/24641; Kotin, Human Gene Therapy 5:793-801 (1994); Muzyczka, J. Clin. Invest. 94:1351 (1994). Construction of recombinant AAV vectors are described in a number of publications, including U.S. Pat. No. 5,173,414; Tratschin et al., Mol. Cell. Biol. 5:3251-3260 (1985); Tratschin, et al., Mol. Cell. Biol. 4:2072-2081 (1984); Hermonat & Muzyczka, PNAS 81:6466-6470 (1984); and Samulski et al., J. Virol. 63:03822-3828 (1989).

In some cases, a host cell is transiently or non-transiently transfected with one or more vectors, linear polynucleotides, polypeptides, nucleic acid-protein complexes, or any combination thereof as described herein. In some cases, a cell in transfected *in vitro,* in culture, or ex vivo. In some cases, a cell is transfected as it naturally occurs in a subject. In some cases, a cell that is transfected is taken from a subject. In some cases, the cell is derived from cells taken from a subject, such as a cell line.

In some cases, a cell transfected with one or more vectors, linear polynucleotides, polypeptides, nucleic acid-protein complexes, or any combination thereof as described herein is used to establish a new cell line comprising one or more transfection-derived sequences. In some cases, a cell transiently transfected with the components of an engineered nucleic acid-guided nuclease system as described herein (such as by transient transfection of one or more vectors, or transfection with RNA), and modified through the activity of an engineered nuclease complex, is used to establish a new cell line comprising cells containing the modification but lacking any other exogenous sequence.

In some cases, one or more vectors described herein are used to produce a non-human transgenic cell, organism, animal, or plant. In some cases, the transgenic animal is a mammal, such as a mouse, rat, or rabbit. Methods for producing transgenic cells, organisms, plants, and animals are known in the art, and generally begin with a method of cell transformation or transfection, such as described herein.

Off-target cleavage events can be analyzed using methods disclosed herein. Off-target cleavage events can be cleavage events that occur at a nucleic acid sequence location other than the intended target sequence, thereby making them unintended target sequence. In general, a library of vectors can be generated as disclosed herein, said vector comprising a target sequence or unintended target sequence adjacent to a PAM sequence. Within a library of such vector, the target sequence or unintended target sequence can vary between the different vectors such that numerous different target sequences or unintended target sequences can be screened or tested within a single experiment or in a high throughput manner. Said vector can comprise a barcode or other unique identifier that allows identification of the target sequence or unintended target sequence to be tested. Said vector can comprise a selectable marker or screenable marker. Said vector can comprise a guide nucleic acid sequence. Said guide nucleic acid can comprise a targeting sequence capable of targeting compatible target sequences within the vector. Said guide nucleic acid can comprise a scaffold sequence. The vector library can then be introduced into host cells, said host cells comprising a subject nuclease. Said subject nuclease can be expressed from the same or different vector that is introduced into the cell either concomitantly, prior to, or subsequent to the guide nucleic acid vector. In other cases, the subject nuclease can be introduced to the cell as a mRNA transcript. In other cases, the subject nuclease can be introduces to the cell as a protein. Without wishing to be bound by theory, within each cell, the guide nucleic acid would be expressed and would complex with the subject nuclease. Then the guide nucleic acid can target the nuclease to compatible target sequences. In some cases, if the guide nucleic acid is able to hybridize with the target sequence or unintended target sequence, then the subject nuclease can cleave the target sequence or unintended target sequence. This cleavage event would cause the host cell to lose the vector comprising the target sequence or to lose the function of the selectable marker or screenable marker, and therefore the host cell would die under a selection or be lost during screening. On the other hand, if the guide nucleic acid is not able to hybridize to the unintended target sequence, then the unintended target sequence would not be cleaved, and thus the host cell would maintain the selectable or screenable marker. By comparing the input vectors to the selected or screened for vectors from the surviving or selected output host cells, one can identify vectors that have been depleted. By sequencing or analyzing the barcode or unique identifier of the input vectors and output vectors, one can identify the barcodes or unique identifiers that have been depleted, which would allow for identification of the target sequences or unintended target sequences that were depleted. The depleted target sequences or unintended target sequences would include those that were able to hybridize to the guide nucleic acid and therefore were able to be cleaved by the subject nuclease. Depleted unintended target sequences would this comprise the off-target cleavage events or the off-target sequences that were able to be cleaved by the subject nuclease system.

Unintended target sequences to be tested or screened in off-target assays disclosed herein can be variants of a known target sequence that is able to be targeted by a subject guide nucleic acid. For example, using the known target sequence of interest, the unintended target sequences can be designed to comprise insertions, deletions, rearrangements, or other sequence alterations compared to the known target sequence. Such sequence alterations can comprise 1, 2, 3, 4, 5, 6, 7, 8, 9, 10, or more nucleotides. Altered nucleotides can be contiguous or non-contiguous.

Methods for calculating and assessing depletion as described previously are applicable to calculation and assessment of depletion in these cases as well.

Other off-target assessing assays involved sequencing the genome of the host organism in order to identify the off-target cleavage events, which are often identifiable due to mutations in the sequence such as insertion, deletion, and mutation of one or more nucleotides. These other methods are thus limited by the host cell genome sequence when assessing the off-target effects of the subject nuclease system. The subject assays disclosed herein allow for a much more robust and high throughput method of identifying off-target effects for virtually any sequence and is not limited by the host cell's genome sequence.

**Methods of use**

In the context of formation of an engineered nuclease complex, "target sequence" refers to a sequence to which a guide sequence is designed to have complementarity, where hybridization between a target sequence and a guide sequence promotes the formation of an engineered nuclease complex. A target sequence may comprise any polynucleotide, such as DNA, RNA, or a DNA-RNA hybrid. A target sequence can be located in the nucleus or cytoplasm of a cell. A target sequence can be located *in vitro* or in a cell-free environment.

Typically, formation of an engineered nuclease complex comprising a guide nucleic acid hybridized to a target sequence and complexed with one or more engineered nucleases as disclosed herein results in cleavage of one or both strands in or near (e.g. within 1, 2, 3, 4, 5, 6, 7, 8, 9, 10, 20, 50, or more base pairs from) the target sequence. Cleavage can occur within a target sequence, 5' of the target sequence, upstream of a target sequence, 3' of the target sequence, or downstream of a target sequence.

In some cases, one or more vectors driving expression of one or more components of a targetable nuclease system are introduced into a host cell or *in vitro* such formation of a targetable nuclease complex at one or more target sites. For example, a nucleic acid-guided nuclease and a guide nucleic acid could each be operably linked to separate regulatory elements on separate vectors. Alternatively, two or more of the elements expressed from the same or different regulatory elements, may be combined in a single vector, with one or more additional vectors providing any components of the targetable nuclease system not included in the first vector. Targetable nuclease system elements that are combined in a single vector may be arranged in any suitable orientation, such as one element located 5' with respect to ("upstream" of) or 3' with respect to ("downstream" of) a second element. The coding sequence of one element may be located on the same or opposite strand of the coding sequence of a second element, and oriented in the same or opposite direction. In some cases, a single promoter drives expression of a transcript encoding a nucleic acid-guided nuclease and one or more guide nucleic acids. In some cases, a nucleic acid-guided nuclease and one or more guide nucleic acids are operably linked to and expressed from the same promoter. In other cases, one or more guide nucleic acids or polynucleotides encoding the one or more guide nucleic acids are introduced into a cell or *in vitro* environment already comprising a nucleic acid-guided nuclease or polynucleotide sequence encoding the nucleic acid-guided nuclease.

When multiple different guide sequences are used, a single expression construct may be used to target nuclease activity to multiple different, corresponding target sequences within a cell or *in vitro.* For example, a single vector may comprise about or more than about 1, 2, 3, 4, 5, 6, 7, 8, 9, 10, 15, 20, or more guide sequences. In some cases, about or more than about 1, 2, 3, 4, 5, 6, 7, 8, 9, 10, or more such guide-sequence-containing vectors may be provided, and optionally delivered to a cell or *in vitro.*

Methods and compositions disclosed herein may comprise more than one guide nucleic acid, wherein each guide nucleic acid has a different guide sequence, thereby targeting a different target sequence. In such cases, multiple guide nucleic acids can be using in multiplexing, wherein multiple targets are targeted simultaneously. Additionally or alternatively, the multiple guide nucleic acids are introduced into a population of cells, such that each cell in a population received a different or random guide nucleic acid, thereby targeting multiple different target sequences across a population of cells. In such cases, the collection of subsequently altered cells can be referred to as a library.

Methods and compositions disclosed herein may comprise multiple different nucleic acid-guided nucleases, each with one or more different corresponding guide nucleic acids, thereby allowing targeting of different target sequences by different nucleic acid-guided nucleases. In some such cases, each nucleic acid-guided nuclease can correspond to a distinct plurality of guide nucleic acids, allowing two or more non-overlapping, partially overlapping, or completely overlapping multiplexing events.

In some cases, the nucleic acid-guided nuclease has DNA cleavage activity or RNA cleavage activity. In some cases, the nucleic acid-guided nuclease directs cleavage of one or both strands at the location of a target sequence, such as within the target sequence and/or within the complement of the target sequence. In some cases, the nucleic acid-guided nuclease directs cleavage of one or both strands within about 1, 2, 3, 4, 5, 6, 7, 8, 9, 10, 15, 20, 25, 50, 100, 200, 500, or more base pairs from the first or last nucleotide of a target sequence.

In some cases, a nucleic acid-guided nuclease may form a component of an inducible system. The inducible nature of the system would allow for spatiotemporal control of gene editing or gene expression using a form of energy. The form of energy may include but is not limited to electromagnetic radiation, sound energy, chemical energy, light energy, temperature, and thermal energy. Examples of inducible system include tetracycline inducible promoters (Tet-On or Tet-Off), small molecule two-hybrid transcription activations systems (FKBP, ABA, etc), or light inducible systems (Phytochrome, LOV domains, or cryptochorome). In one case the nucleic acid-guided nuclease may be a part of a Light Inducible Transcriptional Effector (LITE) to direct changes in transcriptional activity in a sequence-specific manner. The components of a light inducible system may include a nucleic acid-guided nuclease, a light-responsive cytochrome heterodimer (e.g. from Arabidopsis thaliana), and a transcriptional activation/repression domain. Further examples of inducible DNA binding proteins and methods for their use are provided in U.S. 61/736,465 and U.S. 61/721,283. An inducible system can be temperature inducible such that the system is turned on or off by increasing or decreasing the temperature. In some temperature inducible systems, increasing the temperature turns the system on. In some temperature inducible systems, increasing the temperature turns the system off.

In some aspects, the disclosure provides for methods of modifying a target sequence *in vitro,* or in a prokaryotic or eukaryotic cell, which may be *in vivo,* ex vivo, or *in vitro.* In some cases, the method comprises sampling a cell or population of cells such as prokaryotic cells, or those from a human or non-human animal or plant (including micro-algae), and modifying the cell or cells. Culturing may occur at any stage *in vitro* or ex vivo. The cell or cells may even be re-introduced into the host, such as a non-human animal or plant (including micro-algae). For re-introduced cells it is particularly preferred that the cells are stem cells.

In some cases, the method comprises allowing a targetable nuclease complex to bind to the target sequence to effect cleavage of said target sequence, thereby modifying the target sequence, wherein the targetable nuclease complex comprises a nucleic acid-guided nuclease complexed with a guide nucleic acid wherein the guide sequence of the guide nucleic acid is hybridized to a target sequence within a target polynucleotide.

In some aspects, the disclosure provides a method of modifying expression of a target polynucleotide in *in vitro* or in a prokaryotic or eukaryotic cell. In some cases, the method comprises allowing an targetable nuclease complex to bind to a target sequence with the target polynucleotide such that said binding results in increased or decreased expression of said target polynucleotide; wherein the targetable nuclease complex comprises an nucleic acid-guided nuclease complexed with a guide nucleic acid, and wherein the guide sequence of the guide nucleic acid is hybridized to a target sequence within said target polynucleotide. Similar considerations apply as above for methods of modifying a target polynucleotide. In fact, these sampling, culturing and re-introduction options apply across the aspects of the present invention.

In some aspects, the disclosure provides methods for using one or more elements of an engineered targetable nuclease system. A targetable nuclease complex of the disclosure provides an effective means for modifying a target sequence within a target polynucleotide. A targetable nuclease complex of the disclosure has a wide variety of utility including modifying (e.g., deleting, inserting, translocating, inactivating, activating) a target sequence in a multiplicity of cell types. As such a targetable nuclease complex of the invention has a broad spectrum of applications in, e.g., biochemical pathway optimization, genome-wide studies, genome engineering, gene therapy, drug screening, disease diagnosis, and prognosis. An exemplary targetable nuclease complex comprises a nucleic acid-guided nuclease as disclosed herein complexed with a guide nucleic acid, wherein the guide sequence of the guide nucleic acid can hybridize to a target sequence within the target polynucleotide. A guide nucleic acid can comprise a guide sequence linked to a scaffold sequence. A scaffold sequence can comprise one or more sequence regions with a degree of complementarity such that together they form a secondary structure. In some cases, the one or more sequence regions are comprised or encoded on the same polynucleotide. In some cases, the one or more sequence regions are comprised or encoded on separate polynucleotides.

Provided herein are methods of cleaving a target polynucleotide. The method comprises cleaving a target polynucleotide using a targetable nuclease complex that binds to a target sequence within a target polynucleotide and effect cleavage of said target polynucleotide. Typically, the targetable nuclease complex of the invention, when introduced into a cell, creates a break (e.g., a single or a double strand break) in the target sequence. For example, the method can be used to cleave a target gene in a cell, or to replace a wildtype sequence with a modified sequence.

The break created by the targetable nuclease complex can be repaired by a repair processes such as the error prone non-homologous end joining (NHEJ) pathway, the high fidelity homology-directed repair (HDR), or by recombination pathways. During these repair processes, an editing template can be introduced into the genome sequence. In some methods, the HDR or recombination process is used to modify a target sequence. For example, an editing template comprising a sequence to be integrated flanked by an upstream sequence and a downstream sequence is introduced into a cell. The upstream and downstream sequences share sequence similarity with either side of the site of integration in the chromosome, target vector, or target polynucleotide.

An editing template can be DNA or RNA, e.g., a DNA plasmid, a bacterial artificial chromosome (BAC), a yeast artificial chromosome (YAC), a viral vector, a linear piece of DNA, a PCR fragment, oligonucleotide, synthetic polynucleotide, a naked nucleic acid, or a nucleic acid complexed with a delivery vehicle such as a liposome or poloxamer.

An editing template polynucleotide can comprise a sequence to be integrated (e.g., a mutated gene). A sequence for integration may be a sequence endogenous or exogenous to the cell. Examples of a sequence to be integrated include polynucleotides encoding a protein or a non-coding RNA (e.g., a microRNA). Thus, the sequence for integration may be operably linked to an appropriate control sequence or sequences. Alternatively, the sequence to be integrated may provide a regulatory function. Sequence to be integrated may be a mutated or variant of an endogenous wildtype sequence. Alternatively, sequence to be integrated may be a wildtype version of an endogenous mutated sequence. Additionally or alternatively, sequenced to be integrated may be a variant or mutated form of an endogenous mutated or variant sequence.

Upstream and downstream sequences in an editing template polynucleotide can be selected to promote recombination between the target polynucleotide of interest and the editing template polynucleotide. The upstream sequence can be a nucleic acid sequence having sequence similarity with the sequence upstream of the targeted site for integration. Similarly, the downstream sequence can be a nucleic acid sequence having similarity with the sequence downstream of the targeted site of integration. The upstream and downstream sequences in an editing template can have 75%, 80%, 85%, 90%, 95%, or 100% sequence identity with the targeted polynucleotide. Preferably, the upstream and downstream sequences in the editing template polynucleotide have about 95%, 96%, 97%, 98%, 99%, or 100% sequence identity with the targeted polynucleotide. In some methods, the upstream and downstream sequences in the editing template polynucleotide have about 99% or 100% sequence identity with the targeted polynucleotide.

An upstream or downstream sequence may comprise from about 20 bp to about 2500 bp, for example, about 50, 100, 200, 300, 400, 500, 600, 700, 800, 900, 1000, 1100, 1200, 1300, 1400, 1500, 1600, 1700, 1800, 1900, 2000, 2100, 2200, 2300, 2400, or 2500 bp. In some methods, the exemplary upstream or downstream sequence has about 15 bp to about 50 bp, about 30 bp to about 100 bp, about 200 bp to about 2000 bp, about 600 bp to about 1000 bp, or more particularly about 700 bp to about 1000 bp.

In some methods, the editing template polynucleotide may further comprise a marker. Such a marker may make it easy to screen for targeted integrations. Examples of suitable markers include restriction sites, fluorescent proteins, or selectable markers. The exogenous polynucleotide template of the invention can be constructed using recombinant techniques (see, for example, Green and Sambrook et al., 2014 and Ausubel et al., 2017).

In an exemplary method for modifying a target polynucleotide by integrating an editing template polynucleotide, a double stranded break is introduced into the genome sequence by an engineered nuclease complex, the break can be repaired via homologous recombination using an editing template such that the template is integrated into the target polynucleotide. The presence of a double-stranded break can increase the efficiency of integration of the editing template.

Disclosed herein are methods for modifying expression of a polynucleotide in a cell. Some methods comprise increasing or decreasing expression of a target polynucleotide by using a targetable nuclease complex that binds to the target polynucleotide.

In some methods, a target polynucleotide can be inactivated to effect the modification of the expression in a cell. For example, upon the binding of a targetable nuclease complex to a target sequence in a cell, the target polynucleotide is inactivated such that the sequence is not transcribed, the coded protein is not produced, or the sequence does not function as the wild-type sequence does. For example, a protein or microRNA coding sequence may be inactivated such that the protein is not produced.

In some methods, a control sequence can be inactivated such that it no longer functions as a regulatory sequence. As used herein, "regulatory sequence" can refer to any nucleic acid sequence that effects the transcription, translation, or accessibility of a nucleic acid sequence. Examples of regulatory sequences include, a promoter, a transcription terminator, and an enhancer.

An inactivated target sequence may include a deletion mutation (i.e., deletion of one or more nucleotides), an insertion mutation (i.e., insertion of one or more nucleotides), or a nonsense mutation (i.e., substitution of a single nucleotide for another nucleotide such that a stop codon is introduced). In some methods, the inactivation of a target sequence results in "knockout" of the target sequence.

An altered expression of one or more target polynucleotides associated with a signaling biochemical pathway can be determined by assaying for a difference in the mRNA levels of the corresponding genes between the test model cell and a control cell, when they are contacted with a candidate agent. Alternatively, the differential expression of the sequences associated with a signaling biochemical pathway is determined by detecting a difference in the level of the encoded polypeptide or gene product.

To assay for an agent-induced alteration in the level of mRNA transcripts or corresponding polynucleotides, nucleic acid contained in a sample is first extracted according to standard methods in the art. For instance, mRNA can be isolated using various lytic enzymes or chemical solutions according to the procedures set forth in Green and Sambrook (2014), or extracted by nucleic-acid-binding resins following the accompanying instructions provided by the manufacturers. The mRNA contained in the extracted nucleic acid sample is then detected by amplification procedures or conventional hybridization assays (e.g. Northern blot analysis) according to methods widely known in the art or based on the methods exemplified herein.

For purpose of this disclosure, amplification means any method employing a primer and a polymerase capable of replicating a target sequence with reasonable fidelity. Amplification may be carried out by natural or recombinant DNA polymerases such as TaqGold™, T7 DNA polymerase, Klenow fragment of *E. coli* DNA polymerase, and reverse transcriptase. A preferred amplification method is PCR. In particular, the isolated RNA can be subjected to a reverse transcription assay that is coupled with a quantitative polymerase chain reaction (RT-PCR) in order to quantify the expression level of a sequence associated with a signaling biochemical pathway.

Detection of the gene expression level can be conducted in real time in an amplification assay. In one aspect, the amplified products can be directly visualized with fluorescent DNA-binding agents including but not limited to DNA intercalators and DNA groove binders. Because the amount of the intercalators incorporated into the double-stranded DNA molecules is typically proportional to the amount of the amplified DNA products, one can conveniently determine the amount of the amplified products by quantifying the fluorescence of the intercalated dye using conventional optical systems in the art. DNA-binding dye suitable for this application include SYBR green, SYBR blue, DAPI, propidium iodine, Hoeste, SYBR gold, ethidium bromide, acridines, proflavine, acridine orange, acriflavine, fluorcoumanin, ellipticine, daunomycin, chloroquine, distamycin D, chromomycin, homidium, mithramycin, ruthenium polypyridyls, anthramycin, and the like.

In another aspect, other fluorescent labels such as sequence specific probes can be employed in the amplification reaction to facilitate the detection and quantification of the amplified products. Probe-based quantitative amplification relies on the sequence-specific detection of a desired amplified product. It utilizes fluorescent, target-specific probes (e.g., TaqMan™ probes) resulting in increased specificity and sensitivity. Methods for performing probe-based quantitative amplification are well established in the art and are taught in U.S. Pat. No. 5,210,015.

In yet another aspect, conventional hybridization assays using hybridization probes that share sequence homology with sequences associated with a signaling biochemical pathway can be performed. Typically, probes are allowed to form stable complexes with the sequences associated with a signaling biochemical pathway contained within the biological sample derived from the test subject in a hybridization reaction. It will be appreciated by one of skill in the art that where antisense is used as the probe nucleic acid, the target polynucleotides provided in the sample are chosen to be complementary to sequences of the antisense nucleic acids. Conversely, where the nucleotide probe is a sense nucleic acid, the target polynucleotide is selected to be complementary to sequences of the sense nucleic acid.

Hybridization can be performed under conditions of various stringency, for instance as described herein. Suitable hybridization conditions for the practice of the present invention are such that the recognition interaction between the probe and sequences associated with a signaling biochemical pathway is both sufficiently specific and sufficiently stable. Conditions that increase the stringency of a hybridization reaction are widely known and published in the art. See, for example, (Green and Sambrook, et al., (2014); Nonradioactive in Situ Hybridization Application Manual, Boehringer Mannheim, second edition). The hybridization assay can be formed using probes immobilized on any solid support, including but are not limited to nitrocellulose, glass, silicon, and a variety of gene arrays. A preferred hybridization assay is conducted on high-density gene chips as described in U.S. Pat. No. 5,445,934.

For a convenient detection of the probe-target complexes formed during the hybridization assay, the nucleotide probes are conjugated to a detectable label. Detectable labels suitable for use in the present invention include any composition detectable by photochemical, biochemical, spectroscopic, immunochemical, electrical, optical or chemical means. A wide variety of appropriate detectable labels are known in the art, which include fluorescent or chemiluminescent labels, radioactive isotope labels, enzymatic or other ligands. In preferred embodiments, one will likely desire to employ a fluorescent label or an enzyme tag, such as digoxigenin, .beta.-galactosidase, urease, alkaline phosphatase or peroxidase, avidin/biotin complex.

Detection methods used to detect or quantify the hybridization intensity will typically depend upon the label selected above. For example, radiolabels may be detected using photographic film or a phosphoimager. Fluorescent markers may be detected and quantified using a photodetector to detect emitted light. Enzymatic labels are typically detected by providing the enzyme with a substrate and measuring the reaction product produced by the action of the enzyme on the substrate; and finally colorimetric labels are detected by simply visualizing the colored label.

An agent-induced change in expression of sequences associated with a signaling biochemical pathway can also be determined by examining the corresponding gene products. Determining the protein level typically involves a) contacting the protein contained in a biological sample with an agent that specifically bind to a protein associated with a signaling biochemical pathway; and (b) identifying any agent protein complex so formed. In one aspect of this embodiment, the agent that specifically binds a protein associated with a signaling biochemical pathway is an antibody, preferably a monoclonal antibody.

The reaction can be performed by contacting the agent with a sample of the proteins associated with a signaling biochemical pathway derived from the test samples under conditions that will allow a complex to form between the agent and the proteins associated with a signaling biochemical pathway. The formation of the complex can be detected directly or indirectly according to standard procedures in the art. In the direct detection method, the agents are supplied with a detectable label and unreacted agents may be removed from the complex; the amount of remaining label thereby indicating the amount of complex formed. For such method, it is preferable to select labels that remain attached to the agents even during stringent washing conditions. It is preferable that the label does not interfere with the binding reaction. In the alternative, an indirect detection procedure may use an agent that contains a label introduced either chemically or enzymatically. A desirable label generally does not interfere with binding or the stability of the resulting agent polypeptide complex. However, the label is typically designed to be accessible to an antibody for an effective binding and hence generating a detectable signal.

A wide variety of labels suitable for detecting protein levels are known in the art. Non-limiting examples include radioisotopes, enzymes, colloidal metals, fluorescent compounds, bioluminescent compounds, and chemiluminescent compounds.

The amount of agent:polypeptide complexes formed during the binding reaction can be quantified by standard quantitative assays. As illustrated above, the formation of agent polypeptide complex can be measured directly by the amount of label remained at the site of binding. In an alternative, the protein associated with a signaling biochemical pathway is tested for its ability to compete with a labeled analog for binding sites on the specific agent. In this competitive assay, the amount of label captured is inversely proportional to the amount of protein sequences associated with a signaling biochemical pathway present in a test sample.

A number of techniques for protein analysis based on the general principles outlined above are available in the art. They include but are not limited to radioimmunoassays, ELISA (enzyme linked immunoradiometric assays), "sandwich" immunoassays, immunoradiometric assays, in situ immunoassays (using e.g., colloidal gold, enzyme or radioisotope labels), western blot analysis, immunoprecipitation assays, immunofluorescent assays, and SDS-PAGE.

Antibodies that specifically recognize or bind to proteins associated with a signaling biochemical pathway are preferable for conducting the aforementioned protein analyses. Where desired, antibodies that recognize a specific type of post-translational modifications (e.g., signaling biochemical pathway inducible modifications) can be used. Post-translational modifications include but are not limited to glycosylation, lipidation, acetylation, and phosphorylation. These antibodies may be purchased from commercial vendors. For example, anti-phosphotyrosine antibodies that specifically recognize tyrosine-phosphorylated proteins are available from a number of vendors including Invitrogen and Perkin Elmer. Anti-phosphotyrosine antibodies are particularly useful in detecting proteins that are differentially phosphorylated on their tyrosine residues in response to an ER stress. Such proteins include but are not limited to eukaryotic translation initiation factor 2 alpha (eIF-2.alpha.). Alternatively, these antibodies can be generated using conventional polyclonal or monoclonal antibody technologies by immunizing a host animal or an antibody-producing cell with a target protein that exhibits the desired post-translational modification.

In practicing a subject method, it may be desirable to discern the expression pattern of a protein associated with a signaling biochemical pathway in different bodily tissue, in different cell types, and/or in different subcellular structures. These studies can be performed with the use of tissue-specific, cell-specific or subcellular structure specific antibodies capable of binding to protein markers that are preferentially expressed in certain tissues, cell types, or subcellular structures.

An altered expression of a gene associated with a signaling biochemical pathway can also be determined by examining a change in activity of the gene product relative to a control cell. The assay for an agent-induced change in the activity of a protein associated with a signaling biochemical pathway will dependent on the biological activity and/or the signal transduction pathway that is under investigation. For example, where the protein is a kinase, a change in its ability to phosphorylate the downstream substrate(s) can be determined by a variety of assays known in the art. Representative assays include but are not limited to immunoblotting and immunoprecipitation with antibodies such as anti-phosphotyrosine antibodies that recognize phosphorylated proteins. In addition, kinase activity can be detected by high throughput chemiluminescent assays such as AlphaScreen™ (available from Perkin Elmer) and eTag™ assay (Chan-Hui, et al. (2003) Clinical Immunology 111: 162-174).

Where the protein associated with a signaling biochemical pathway is part of a signaling cascade leading to a fluctuation of intracellular pH condition, pH sensitive molecules such as fluorescent pH dyes can be used as the reporter molecules. In another example where the protein associated with a signaling biochemical pathway is an ion channel, fluctuations in membrane potential and/or intracellular ion concentration can be monitored. A number of commercial kits and high-throughput devices are particularly suited for a rapid and robust screening for modulators of ion channels. Representative instruments include FLIPR™ (Molecular Devices, Inc.) and VIPR (Aurora Biosciences). These instruments can detect reactions in over 1000 sample wells of a microplate simultaneously and providing real-time measurement and functional data within a second or even a fraction thereof.

In practicing any of the methods disclosed herein, a suitable vector can be introduced to a cell, tissue, organism, or a non-human embryo via one or more methods known in the art, including without limitation, microinjection, electroporation, sonoporation, biolistics, calcium phosphate-mediated transfection, cationic transfection, liposome transfection, dendrimer transfection, heat shock transfection, nucleofection transfection, magnetofection, lipofection, impalefection, optical transfection, proprietary agent-enhanced uptake of nucleic acids, and delivery via liposomes, immunoliposomes, virosomes, or artificial virions. In some methods, the vector or vectors may be introduced into a cell by nucleofection.

**Target sequence**

A target polynucleotide of a targetable nuclease complex can be any polynucleotide endogenous or exogenous to the host cell. For example, the target polynucleotide can be a polynucleotide residing in the nucleus of the eukaryotic cell, the genome of a prokaryotic cell, or an extrachromosomal vector of a host cell. The target polynucleotide can be a sequence coding a gene product (e.g., a protein) or a non-coding sequence (e.g., a regulatory polynucleotide or a junk DNA).

Examples of target polynucleotides include a sequence associated with a signaling biochemical pathway, e.g., a signaling biochemical pathway-associated gene or polynucleotide. Examples of target polynucleotides include a disease associated gene or polynucleotide. A "disease-associated" gene or polynucleotide refers to any gene or polynucleotide which is yielding transcription or translation products at an abnormal level or in an abnormal form in cells derived from a disease-affected tissues compared with tissues or cells of a non-disease control. It may be a gene that becomes expressed at an abnormally high level; it may be a gene that becomes expressed at an abnormally low level, where the altered expression correlates with the occurrence and/or progression of the disease. A disease-associated gene also refers to a gene possessing mutation(s) or genetic variation that is directly responsible or is in linkage disequilibrium with a gene(s) that is responsible for the etiology of a disease. The transcribed or translated products may be known or unknown, and may be at a normal or abnormal level.

This disclosure also relates to methods and compositions related to knocking out genes, editing genes, altering genes, amplifying genes, and repairing particular mutations. Altering genes may also mean the epigenetic manipulation of a target sequence. This may be the chromatin state of a target sequence, such as by modification of the methylation state of the target sequence (i.e. addition or removal of methylation or methylation patterns or CpG islands), histone modification, increasing or reducing accessibility to the target sequence, or by promoting 3D folding. It will be appreciated that where reference is made to a method of modifying a cell, organism, or mammal including human or a non-human mammal or organism by manipulation of a target sequence in a genomic locus of interest, this may apply to the organism (or mammal) as a whole or just a single cell or population of cells from that organism (if the organism is multicellular). In the case of humans, for instance, Applicants envisage, inter alia, a single cell or a population of cells and these may preferably be modified ex vivo and then re-introduced. In this case, a biopsy or other tissue or biological fluid sample may be necessary. Stem cells are also particularly preferred in this regard. But, of course, *in vivo* embodiments are also envisaged. And the invention is especially advantageous as to HSCs.

The functionality of a targetable nuclease complex can be assessed by any suitable assay. For example, the components of a targetable nuclease system sufficient to form a targetable nuclease complex, including a guide nucleic acid and nucleic acid-guided nuclease, can be provided to a host cell having the corresponding target sequence, such as by transfection with vectors encoding the components of the engineered nuclease system, followed by an assessment of preferential cleavage within the target sequence. Similarly, cleavage of a target sequence may be evaluated in a test tube by providing the target sequence and components of a targetable nuclease complex. Other assays are possible, and will occur to those skilled in the art. A guide sequence can be selected to target any target sequence. In some cases, the target sequence is a sequence within a genome of a cell. Exemplary target sequences include those that are unique in the target genome.

**Editing cassette**

Disclosed herein are compositions and methods for editing a target polynucleotide sequence. Such compositions include polynucleotides containing one or more components of targetable nuclease system. Polynucleotide sequences for use in these methods can be referred to as editing cassettes.

An editing cassette can comprise one or more primer sites. Primer sites can be used to amplify an editing cassette by using oligonucleotide primers comprising reverse complementary sequences that can hybridize to the one or more primer sites. An editing cassette can comprise two or more primer times. Sometimes, an editing cassette comprises a primer site on each end of the editing cassette, said primer sites flanking one or more of the other components of the editing cassette. Primer sites can be approximately 10, 15, 16, 17, 18, 19, 20, 21, 22, 23, 24, 25, 26 or more nucleotides in length.

An editing cassette can comprise an editing template as disclosed herein. An editing cassette can comprise an editing sequence. An editing sequence can be homologous to a target sequence. An editing sequence can comprise at least one mutation relative to a target sequence. An editing sequence often comprises homology region (or homology arms) flanking at least one mutation relative to a target sequence, such that the flanking homology regions facilitate homologous recombination of the editing sequence into a target sequence. An editing sequence can comprise an editing template as disclosed herein. For example, the editing sequence can comprise at least one mutation relative to a target sequence including one or more PAM mutations that mutate or delete a PAM site. An editing sequence can comprise one or more mutations in a codon or non-coding sequence relative to a non-editing target site.

A PAM mutation can be a silent mutation. A silent mutation can be a change to at least one nucleotide of a codon relative to the original codon that does not change the amino acid encoded by the original codon. A silent mutation can be a change to a nucleotide within a non-coding region, such as an intron, 5' untranslated region, 3' untranslated region, or other non-coding region.

A PAM mutation can be a non-silent mutation. Non-silent mutations can include a missense mutation. A missense mutation can be when a change to at least one nucleotide of a codon relative to the original codon that changes the amino acid encoded by the original codon. Missense mutations can occur within an exon, open reading frame, or other coding region.

An editing sequence can comprise at least one mutation relative to a target sequence. A mutation can be a silent mutation or non-silent mutation, such as a missense mutation. A mutation can include an insertion of one or more nucleotides or base pairs. A mutation can include a deletion of one or more nucleotides or base pairs. A mutation can include a substitution of one or more nucleotides or base pairs for a different one or more nucleotides or base pairs. Inserted or substituted sequences can include exogenous or heterologous sequences.

An editing cassette can comprise a polynucleotide encoding a guide nucleic acid sequence. In some cases, the guide nucleic acid sequence is optionally operably linked to a promoter. A guide nucleic acid sequence can comprise a scaffold sequence and a guide sequence as described herein.

An editing cassette can comprise a barcode. A barcode can be a unique DNA sequence that corresponds to the editing sequence such that the barcode can identify the one or more mutations of the corresponding editing sequence. In some examples, the barcode is 15 nucleotides. The barcode can comprise less than 10, 10, 11, 12, 13, 14, 15, 16, 17, 18, 19, 20, 25, 30, 35, 40, 45, 50, 55, 60, 65, 70, 75, 80, 88, 90, 100, 110, 120, 130, 140, 150, 160, 170, 180, 190, 200, or more than 200 nucleotides. A barcode can be a non-naturally occurring sequence. An editing cassette comprising a barcode can be a non-naturally occurring sequence.

An editing cassette can comprise one or more of an editing sequence and a polynucleotide encoding a guide nucleic acid optionally operably linked to a promoter, wherein the editing cassette and guide nucleic acid sequence are flanked by primer sites. An editing cassette can further comprise a barcode.

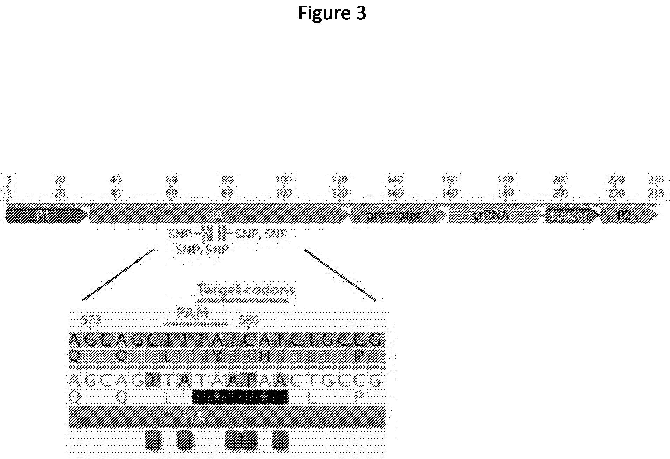

An example of an editing cassette is depicted in Figure 3. Each editing cassette can be designed to edit a site in a target sequence Sites to be targeted can be coding regions, non-coding regions, functionally neutral sites, or they can be a screenable or selectable marker gene. Homology regions within the editing sequence flank the one or more mutations of the editing cassette and can be inserted into the target sequence by recombination. Recombination can comprise DNA cleavage, such as by a nucleic acid-guided nuclease, and repair via homologous recombination.

Editing cassettes can be generated by chemical synthesis, Gibson assembly, SLIC, CPEC, PCA, ligation-free cloning, overlapping oligo extension, *in vitro* assembly, *in vitro* oligo assembly, PCR, traditional ligation-based cloning, other known methods in the art, or any combination thereof.

Trackable sequences, such as barcodes or recorder sequences, can be designed in silico via standard code with a degenerate mutation at the target codon. The degenerate mutation can comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or more than 30 nucleic acid residues. In some examples, the degenerate mutations can comprise 15 nucleic acid residues (N15).

Homology arms can be added to an editing sequence to allow incorporation of the editing sequence into the desired location via homologous recombination or homology-driven repair. Homology arms can be added by synthesis, *in vitro* assembly, PCR, or other known methods in the art. For example, chemical synthesis, Gibson assembly, SLIC, CPEC, PCA, ligation-free cloning, overlapping oligo extension, *in vitro* assembly, *in vitro* oligo assembly, PCR, traditional ligation-based cloning, other known methods in the art, or any combination thereof. A homology arm can be added to both ends of a barcode, recorder sequence, and/or editing sequence, thereby flanking the sequence with two distinct homology arms, for example, a 5' homology arm and a 3' homology arm.

A homology arm can comprise sequence homologous to a target sequence. A homology arm can comprise sequence homologous to sequence adjacent to a target sequence. A homology arm can comprise sequence homologous to sequence upstream or downstream of a target sequence. A homology arm can comprise sequence homologous to sequence within the same gene or open reading frame as a target sequence. A homology arm can comprise sequence homologous to sequence upstream or downstream of a gene or open reading frame the target sequence is within. A homology arm can comprise sequence homologous to a 5' UTR or 3' UTR of a gene or open reading frame within which is a target sequence. A homology arm can comprise sequence homologous to a different gene, open reading frame, promoter, terminator, or nucleic acid sequence than that which the target sequence is within.

The same 5' and 3' homology arms can be added to a plurality of distinct editing sequences, thereby generating a library of unique editing sequences that each have the same targeted insertion site. The same 5' and 3' homology arms can be added to a plurality of distinct editing templates, thereby generating a library of unique editing templates that each have the same targeted insertion site. In alternative examples, different or a variety of 5' or 3' homology arms can be added to a plurality of editing sequences or editing templates.

A barcode library or recorder sequence library comprising flanking homology arms can be cloned into a vector backbone. In some examples, the barcode comprising flanking homology arms are cloned into an editing cassette. Cloning can occur by chemical synthesis, Gibson assembly, SLIC, CPEC, PCA, ligation-free cloning, overlapping oligo extension, *in vitro* assembly, *in vitro* oligo assembly, PCR, traditional ligation-based cloning, other known methods in the art, or any combination thereof.

An editing sequence library comprising flanking homology arms can be cloned into a vector backbone. In some examples, the editing sequence and homology arms are cloned into an editing cassette. Editing cassettes can, in some cases, further comprise a nucleic acid sequence encoding a guide nucleic acid or gRNA engineered to target the desired site of editing sequence insertion, e.g. the target sequence. Editing cassettes can, in some cases, further comprise a barcode or recorder sequence. Cloning can occur by chemical synthesis, Gibson assembly, SLIC, CPEC, PCA, ligation-free cloning, overlapping oligo extension, *in vitro* assembly, *in vitro* oligo assembly, PCR, traditional ligation-based cloning, other known methods in the art, or any combination thereof.

Gene-wide or genome-wide editing libraries can be cloned into a vector backbone. A barcode or recorder sequence library can be inserted or assembled into a second site to generate competent trackable plasmids that can embed the recording barcode at a fixed locus while integrating the editing libraries at a wide variety of user defined sites. Cloning can occur by chemical synthesis, Gibson assembly, SLIC, CPEC, PCA, ligation-free cloning, overlapping oligo extension, *in vitro* assembly, *in vitro* oligo assembly, PCR, traditional ligation-based cloning, other known methods in the art, or any combination thereof.

A guide nucleic acid or sequence encoding the same can be assembled or inserted into a vector backbone first, followed by insertion of an editing sequence and/or cassette. In other cases, an editing sequence and/or cassette can be inserted or assembled into a vector backbone first, followed by insertion of a guide nucleic acid or sequence encoding the same. In other cases, guide nucleic acid or sequence encoding the same and an editing sequence and/or cassette are simultaneous inserted or assembled into a vector A recorder sequence or barcode can be inserted before or after any of these steps. In other words, it should be understood that there are many possible permutations to the order in which elements of the disclosure are assembled. The vector can be linear or circular and can be generated by chemical synthesis, Gibson assembly, SLIC, CPEC, PCA, ligation-free cloning, overlapping oligo extension, *in vitro* assembly, *in vitro* oligo assembly, PCR, traditional ligation-based cloning, other known methods in the art, or any combination thereof.

A nucleic acid molecule can be synthesized which comprises one or more elements disclosed herein. A nucleic acid molecule can be synthesized that comprises an editing cassette. A nucleic acid molecule can be synthesized that comprises a guide nucleic acid. A nucleic acid molecule can be synthesized that comprises a recorder cassette. A nucleic acid molecule can be synthesized that comprises a barcode. A nucleic acid molecule can be synthesized that comprises a homology arm. A nucleic acid molecule can be synthesized that comprises an editing cassette and a guide nucleic acid. A nucleic acid molecule can be synthesized that comprises an editing cassette and a barcode. A nucleic acid molecule can be synthesized that comprises an editing cassette, a guide nucleic acid, and a recorder cassette. A nucleic acid molecule can be synthesized that comprises an editing cassette, a recorder cassette, and two guide nucleic acids. A nucleic acid molecule can be synthesized that comprises a recorder cassette and a guide nucleic acid. In any of these cases, the guide nucleic acid can optionally be operably linked to a promoter. In any of these cases, the nucleic acid molecule can further include one or more barcodes.

Synthesis can occur by any nucleic acid synthesis method known in the art. Synthesis can occur by enzymatic nucleic acid synthesis. Synthesis can occur by chemical synthesis. Synthesis can occur by array-based synthesis. Synthesis can occur by solid-phase synthesis or phosphoramidite methods. Synthesis can occur by column or multi-well methods. Synthesized nucleic acid molecules can be non-naturally occurring nucleic acid molecules.

Software and automation methods can be used for multiplex synthesis and generation. For example, software and automation can be used to create $10$, $10^2$, $10^3$, $10^4$, $10^5$, $10^6$, or more synthesized polynucleotides, cassettes, or plasmids. An automation method can generate desired sequences and libraries in rapid fashion that can be processed through a workflow with minimal steps to produce precisely defined libraries, such as gene-wide or genome-wide editing libraries.

Polynucleotides or libraries can be generated which comprise two or more nucleic acid molecules or plasmids comprising any combination disclosed herein of recorder sequence, editing sequence, guide nucleic acid, and optional barcode, including combinations of one or more of any of the previously mentioned elements. For example, such a library can comprise at least 2, 3, 4, 5, 10, 20, 30, 40, 50, 60, 70, 80, 90, 100, 200, 300, 400, 500, 600, 700, 800, 1000, 1500, 2000, 2500, 3000, 3500, 4000, 4500, 5000, 5500, 6000, 6500, 7000, 7500, 8000, 8500, 9000, 9500, $10^4$, $10^5$, $10^6$, $10^7$, $10^8$, $10^9$, $10^{10}$, or more nucleic acid molecules or plasmids of the present disclosure. It should be understood that such a library can include any number of nucleic acid molecules or plasmids, even if the specific number is not explicit listed above.

Trackable plasmid libraries or nucleic acid molecule libraries can be sequenced in order to determine the recorder sequence and editing sequence pair that is comprised on each trackable plasmid. In other cases, a known recorder sequence is paired with a known editing sequence during the library generation process. Other methods of determining the association between a recorder sequence and editing sequence comprised on a common nucleic acid molecule or plasmid are envisioned such that the editing sequence can be identified by identification or sequencing of the recorder sequence.

Methods and compositions for tracking edited episomal libraries that are shuttled between *E. coli* and other organisms/cell lines are provided herein. The libraries can be comprised on plasmids, Bacterial artificial chromosomes (BACs), Yeast artificial chromosomes (YACs), synthetic chromosomes, or viral or phage genomes. These methods and compositions can be used to generate portable barcoded libraries in host organisms, such as *E. coli.* Library generation in such organisms can offer the advantage of established techniques for performing homologous recombination. Barcoded plasmid libraries can be deep-sequenced at one site to track mutational diversity targeted across the remaining portions of the plasmid allowing dramatic improvements in the depth of library coverage.

Any nucleic acid molecule disclosed herein can be an isolated nucleic acid. Isolated nucleic acids may be made by any method known in the art, for example using standard recombinant methods, assembly methods, synthesis techniques, or combinations thereof. In some embodiments, the nucleic acids may be cloned, amplified, assembled, or otherwise constructed.

Isolated nucleic acids may be obtained from cellular, bacterial, or other sources using any number of cloning methodologies known in the art. In some embodiments, oligonucleotide probes which selectively hybridize, under stringent conditions, to other oligonucleotides or to the nucleic acids of an organism or cell can be used to isolate or identify an isolated nucleic acid.

Cellular genomic DNA, RNA, or cDNA may be screened for the presence of an identified genetic element of interest using a probe based upon one or more sequences. Various degrees of stringency of hybridization may be employed in the assay.

High stringency conditions for nucleic acid hybridization are well known in the art. For example, conditions may comprise low salt and/or high temperature conditions, such as provided by about 0.02 M to about 0.15 M NaCl at temperatures of about 50° C. to about 70° C. It is understood that the temperature and ionic strength of a desired stringency are determined in part by the length of the particular nucleic acid(s), the length and nucleotide content of the target sequence(s), the charge composition of the nucleic acid(s), and by the presence or concentration of formamide, tetramethylammonium chloride or other solvent(s) in a hybridization mixture. Nucleic acids may be completely complementary to a target sequence or may exhibit one or more mismatches.

Nucleic acids of interest may also be amplified using a variety of known amplification techniques. For instance, polymerase chain reaction (PCR) technology may be used to amplify target sequences directly from DNA, RNA, or cDNA. PCR and other *in vitro* amplification methods may also be useful, for example, to clone nucleic acid sequences, to make nucleic acids to use as probes for detecting the presence of a target nucleic acid in samples, for nucleic acid sequencing, or for other purposes.

Isolated nucleic acids may be prepared by direct chemical synthesis by methods such as the phosphotriester method, or using an automated synthesizer. Chemical synthesis generally produces a single stranded oligonucleotide. This may be converted into double stranded DNA by hybridization with a complementary sequence or by polymerization with a DNA polymerase using the single strand as a template.

**Recorder**

In some example, two editing cassettes can be used together to track a genetic engineering step. For example, one editing cassette can comprise an editing template and an encoded guide nucleic acid, and a second editing cassette, referred to as a recorder cassette, can comprise an editing template comprising a recorder sequence and an encoded nucleic acid which has a distinct guide sequence compared to that of the first editing cassette. In such cases, the editing sequence and the recorder sequence can be inserted into separate target sequences and determined by their corresponding guide nucleic acids. A recorder sequence can comprise a barcode, trackable or traceable sequence, and/or a regulatory element operable with a screenable or selectable marker.

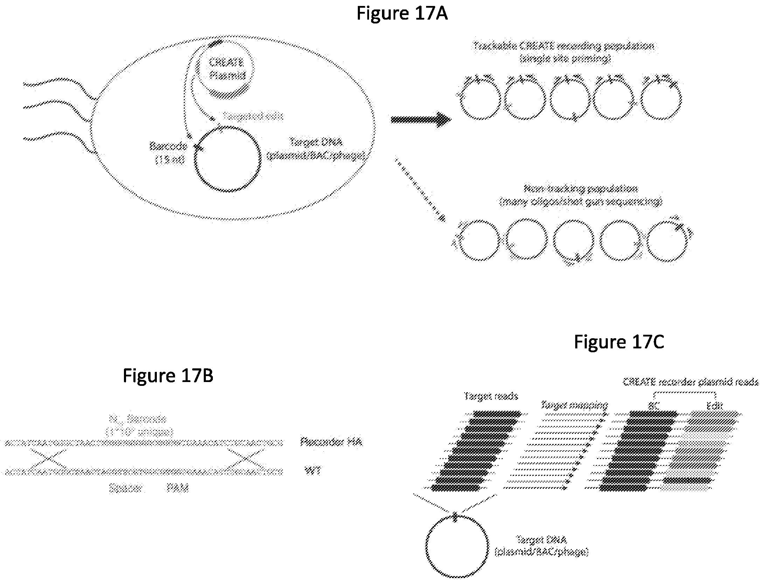

Through a multiplexed cloning approach, the recorder cassette can be covalently coupled to at least one editing cassette in a plasmid (e.g., Figure 17A) to generate plasmid libraries that have a unique recorder and editing cassette combination. This library can be sequenced to generate the recorder/edit mapping and used to track editing libraries across large segments of the target DNA (e.g., Figure 17C). Recorder and editing sequences can be comprised on the same cassette, in which case they are both incorporated into the target nucleic acid sequence, such as a genome or plasmid, by the same recombination event. In other examples, the recorder and editing sequences can be comprised on separate cassettes within the same plasmid, in which case the recorder and editing sequences are incorporated into the target nucleic acid sequence by separate recombination events, either simultaneously or sequentially.

Methods are provided herein for combining multiplex oligonucleotide synthesis with recombineering, to create libraries of specifically designed and trackable mutations. Screens and/or selections followed by high-throughput sequencing and/or barcode microarray methods can allow for rapid mapping of mutations leading to a phenotype of interest.

Methods and compositions disclosed herein can be used to simultaneously engineer and track engineering events in a target nucleic acid sequence.

Such plasmids can be generated using *in vitro* assembly or cloning techniques. For example, the plasmids can be generated using chemical synthesis, Gibson assembly, SLIC, CPEC, PCA, ligation-free cloning, other *in vitro* oligo assembly techniques, traditional ligation-based cloning, or any combination thereof.

Such plasmids can comprise at least one recording sequence, such as a barcode, and at least one editing sequence. In most cases, the recording sequence is used to record and track engineering events. Each editing sequence can be used to incorporate a desired edit into a target nucleic acid sequence. The desired edit can include insertion, deletion, substitution, or alteration of the target nucleic acid sequence. In some examples, the one or more recording sequence and editing sequences are comprised on a single cassette comprised within the plasmid such that they are incorporated into the target nucleic acid sequence by the same engineering event. In other examples, the recording and editing sequences are comprised on separate cassettes within the plasmid such that they are each incorporated into the target nucleic acid by distinct engineering events. In some examples, the plasmid comprises two or more editing sequences. For example, one editing sequence can be used to alter or silence a PAM sequence while a second editing sequence can be used to incorporate a mutation into a distinct sequence.

Recorder sequences can be inserted into a site separated from the editing sequence insertion site. The inserted recorder sequence can be separated from the editing sequence by 1bp to 1Mbp. For example, the separation distance can be about 1bp, 10bp, 50bp, 100bp, 500bp, 1kp, 2kb, 5kb, 10kb, or greater. The separation distance can be any discrete integer between 1bp and 10Mbp. In some examples, the maximum distance of separation depends on the size of the target nucleic acid or genome.

Recorder sequences can be inserted adjacent to editing sequences, or within proximity to the editing sequence. For example, the recorder sequence can be inserted outside of the open reading frame within which the editing sequence is inserted. Recorder sequence can be inserted into an untranslated region adjacent to an open reading frame within which an editing sequence has been inserted. The recorder sequence can be inserted into a functionally neutral or non-functional site. The recorder sequence can be inserted into a screenable or selectable marker gene.

In some examples, the target nucleic acid sequence is comprised within a genome, artificial chromosome, synthetic chromosome, or episomal plasmid. In various examples, the target nucleic acid sequence can be *in vitro* or *in vivo*. When the target nucleic acid sequence is *in vivo,* the plasmid can be introduced into the host organisms by transformation, transfection, conjugation, biolistics, nanoparticles, cell-permeable technologies, or other known methods for DNA delivery, or any combination thereof. In such examples, the host organism can be a eukaryote, prokaryote, bacterium, archaea, yeast, or other fungi.

The engineering event can comprise recombineering, non-homologous end joining, homologous recombination, or homology-driven repair. In some examples, the engineering event is performed *in vitro* or *in vivo.*

The methods described herein can be carried out in any type of cell in which a targetable nuclease system can function (e.g., target and cleave DNA), including prokaryotic and eukaryotic cells. In some embodiments the cell is a bacterial cell, such as Escherichia spp. (e.g., *E. coli*). In other embodiments, the cell is a fungal cell, such as a yeast cell, e.g., Saccharomyces spp. In other embodiments, the cell is an algal cell, a plant cell, an insect cell, or a mammalian cell, including a human cell.

In some examples, the cell is a recombinant organism. For example, the cell can comprise a non-native targetable nuclease system. Additionally or alternatively, the cell can comprise recombination system machinery. Such recombination systems can include lambda red recombination system, Cre/Lox, attB/attP, or other integrase systems. Where appropriate, the plasmid can have the complementary components or machinery required for the selected recombination system to work correctly and efficiently.

Method for genome editing can comprise: (a) introducing a vector that encodes at least one editing cassette and at least one guide nucleic acid into a first population of cells, thereby producing a second population of cells comprising the vector; (b) maintaining the second population of cells under conditions in which a nucleic acid-guided nuclease is expressed or maintained, wherein the nucleic acid-guided nuclease is encoded on the vector, a second vector, on the genome of cells of the second population of cells, or otherwise introduced into the cell, resulting in DNA cleavage and incorporation of the editing cassette; (c) obtaining viable cells; and (d) sequencing the target DNA molecule in at least one cell of the second population of cells to identify the mutation of at least one codon.

A method for genome editing can comprise: (a) introducing a vector that encodes at least one editing cassette comprising a PAM mutation as disclosed herein and at least one guide nucleic acid into a first population of cells, thereby producing a second population of cells comprising the vector; (b) maintaining the second population of cells under conditions in which a nucleic acid-guided nuclease is expressed or maintained, wherein the nucleic acid-guided nuclease is encoded on the vector, a second vector, on the genome of cells of the second population of cells, or otherwise introduced into the cell, resulting in DNA cleavage, incorporation of the editing cassette, and death of cells of the second population of cells that do not comprise the PAM mutation, whereas cells of the second population of cells that comprise the PAM mutation are viable; (c) obtaining viable cells; and (d) sequencing the target DNA in at least one cell of the second population of cells to identify the mutation of at least one codon.

Method for trackable genome editing can comprise: (a) introducing a vector that encodes at least one editing cassette, at least one recorder cassette, and at least two guide nucleic acids into a first population of cells, thereby producing a second population of cells comprising the vector; (b) maintaining the second population of cells under conditions in which a nucleic acid-guided nuclease is expressed or maintained, wherein the nucleic acid-guided nuclease is encoded on the vector, a second vector, on the genome of cells of the second population of cells, or otherwise introduced into the cell, resulting in DNA cleavage and incorporation of the editing and recorder cassettes; (c) obtaining viable cells; and (d) sequencing the recorder sequence of the target DNA molecule in at least one cell of the second population of cells to identify the mutation of at least one codon.

In some examples where the plasmid comprises a second editing sequence designed to silence a PAM, a method for trackable genome editing can comprise: (a) introducing a vector that encodes at least one editing cassette, a recorder cassette, and at least two guide nucleic acids into a first population of cells, thereby producing a second population of cells comprising the vector; (b) maintaining the second population of cells under conditions in which a nucleic acid-guided nuclease is expressed or maintained, wherein the nucleic acid-guided nuclease is encoded on the vector, a second vector, on the genome of cells of the second population of cells, or otherwise introduced into the cell, resulting in DNA cleavage, incorporation of the editing and recorder cassettes, and death of cells of the second population of cells that do not comprise the PAM mutation, whereas cells of the second population of cells that comprise the PAM mutation are viable; (c) obtaining viable cells; and (d) sequencing the recorder sequence of the target DNA in at least one cell of the second population of cells to identify the mutation of at least one codon.

In some examples transformation efficiency is determined by using a non-targeting control guide nucleic acid, which allows for validation of the recombineering procedure and CFU/ng calculations. In some cases, absolute efficient is obtained by counting the total number of colonies on each transformation plate, for example, by counting both red and white colonies from a galK control. In some examples, relative efficiency is calculated by the total number of successful transformants (for example, white colonies) out of all colonies from a control (for example, galK control).

The methods of the disclosure can provide, for example, greater than 1000x improvements in the efficiency, scale, cost of generating a combinatorial library, and/or precision of such library generation.

The methods of the disclosure can provide, for example, greater than: 10x, 50x, 100x, 200x, 300x, 400x, 500x, 600x, 700x, 800x, 900x, 1000x, 1100x, 1200x, 1300x, 1400x, 1500x, 1600x, 1700x, 1800x, 1900x, 2000x, or greater improvements in the efficiency of generating genomic or combinatorial libraries.

The methods of the disclosure can provide, for example, greater than: 10×, 50x, 100x, 200x, 300x, 400x, 500x, 600x, 700x, 800x, 900x, 1000x, 1100x, 1200x, 1300x, 1400x, 1500x, 1600x, 1700x, 1800x, 1900x, 2000x, or greater improvements in the scale of generating genomic or combinatorial libraries.

The methods of the disclosure can provide, for example, greater than: 10×, 50x, 100x, 200x, 300x, 400x, 500x, 600x, 700x, 800x, 900x, 1000x, 1100x, 1200x, 1300x, 1400x, 1500x, 1600x, 1700x, 1800x, 1900x, 2000x, or greater decrease in the cost of generating genomic or combinatorial libraries.

The methods of the disclosure can provide, for example, greater than: 10×, 50x, 100x, 200x, 300x, 400x, 500x, 600x, 700x, 800x, 900x, 1000x, 1100x, 1200x, 1300x, 1400x, 1500x, 1600x, 1700x, 1800x, 1900x, 2000x, or greater improvements in the precision of genomic or combinatorial library generation.

## Recursive tracking for combinatorial engineering

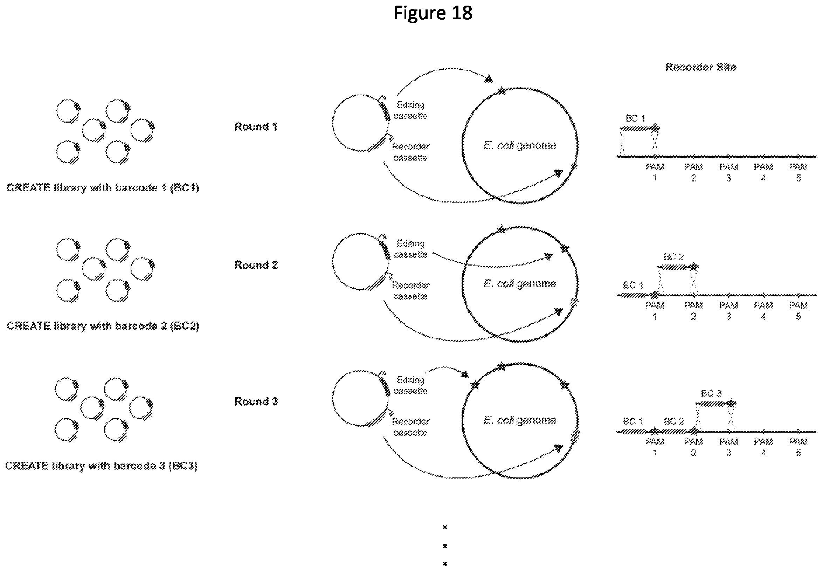

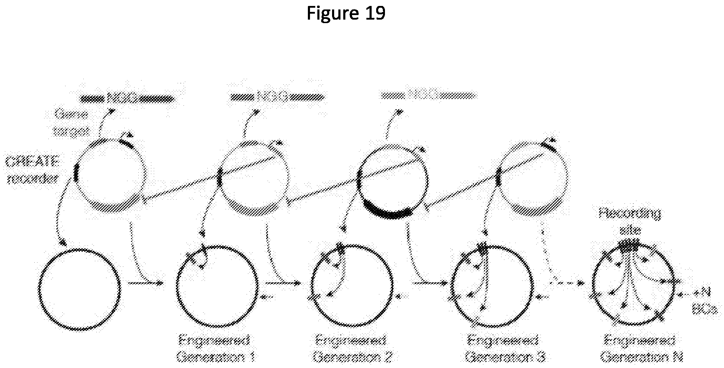

Disclosed herein are methods and compositions for iterative rounds of engineering. Disclosed herein are recursive engineering strategies that allow implementation of CREATE recording at the single cell level through several serial engineering cycles (e.g., Figure 18 and Figure 19). These disclosed methods and compositions can enable search-based technologies that can effectively construct and explore complex genotypic space. The terms recursive and iterative can be used interchangeably.

Combinatorial engineering methods can comprise multiple rounds of engineering. Methods disclosed herein can comprise 2 or more rounds of engineering For example, a method can comprise 2, 3, 4, 5, 6, 7, 8, 9, 10, 11, 12, 13, 14, 15, 20, 25, 30, or more than 30 rounds of engineering.

In some examples, during each round of engineering a new recorder sequence, such as a barcode, is incorporated at the same locus in nearby sites (e.g., Figure 18, grey bars or Figure 19, black bars) such that following multiple engineering cycles to construct combinatorial diversity throughout the genome (e.g., Figure 18, grey bars or Figure 19, grey bars) a simple PCR of the recording locus can be used to reconstruct each combinatorial genotype or to confirm that the engineered edit from each round has been incorporated into the target site.

Disclosed herein are methods for selecting for successive rounds of engineering. Selection can occur by a PAM mutation incorporated by an editing cassette. Selection can occur by a PAM mutation incorporated by a recorder cassette. Selection can occur using a screenable, selectable, or counter-selectable marker. Selection can occur by targeting a site for editing or recording that was incorporated by a prior round of engineering, thereby selecting for variants that successfully incorporated edits and recorder sequences from both rounds or all prior rounds of engineering.

Quantitation of these genotypes can be used for understanding combinatorial mutational effects on large populations and investigation of important biological phenomena such as epistasis.

Serial editing and combinatorial tracking can be implemented using recursive vector systems as disclosed herein. These recursive vector systems can be used to move rapidly through the transformation procedure. In some examples, these systems consist of two or more plasmids containing orthogonal replication origins, antibiotic markers, and encoded guide nucleic acids. The encoded guide nucleic acid in each vector can be designed to target one of the other resistance markers for destruction by nucleic acid-guided nuclease-mediated cleavage. These systems can be used, in some examples, to perform transformations in which the antibiotic selection pressure is switched to remove the previous plasmid and drive enrichment of the next round of engineered genomes. Two or more passages through the transformation loop can be performed, or in other words, multiple rounds of engineering can be performed. Introducing the requisite recording cassettes and editing cassettes into recursive vectors as disclosed herein can be used for simultaneous genome editing and plasmid curing in each transformation step with high efficiencies.

In some examples, the recursive vector system disclosed herein comprises 2, 3, 4, 5, 6, 7, 8, 9, 10, or more than 10 unique plasmids. In some examples, the recursive vector system can use a particular plasmid more than once as long as a distinct plasmid is used in the previous round and in the subsequent round.

Recursive methods and compositions disclosed herein can be used to restore function to a selectable or screenable element in a targeted genome or plasmid. The selectable or screenable element can include an antibiotic resistance gene, a fluorescent gene, a unique DNA sequence or watermark, or other known reporter, screenable, or selectable gene. In some examples, each successive round of engineering can incorporate a fragment of the selectable or screenable element, such that at the end of the engineering rounds, the entire selectable or screenable element has been incorporated into the target genome or plasmid. In such examples, only those genome or plasmids which have successfully incorporated all of the fragments, and therefore all of the desired corresponding mutations, can be selected or screened for. In this way, the selected or screened cells will be enriched for those that have incorporated the edits from each and every iterative round of engineering.

Recursive methods can be used to switch a selectable or screenable marker between an on and an off position, or between an off and an on position, with each successive round of engineering. Using such a method allows conservation of available selectable or screenable markers by requiring, for example, the use of only one screenable or selectable marker. Furthermore, short regulatory sequence or start codon or non-start codons can be used to turn the screenable or selectable marker on and off. Such short sequences can easily fit within a synthesized cassette or polynucleotide.

One or more rounds of engineering can be performed using the methods and compositions disclosed herein. In some examples, each round of engineering is used to incorporate an edit unique from that of previous rounds. Each round of engineering can incorporate a unique recording sequence. Each round of engineering can result in removal or curing of the plasmid used in the previous round of engineering. In some examples, successful incorporation of the recording sequence of each round of engineering results in a complete and functional screenable or selectable marker or unique sequence combination.

Unique recorder cassettes comprising recording sequences such as barcodes or screenable or selectable markers can be inserted with each round of engineering, thereby generating a recorder sequence that is indicative of the combination of edits or engineering steps performed. Successive recording sequences can be inserted adjacent to one another. Successive recording sequences can be inserted within proximity to one another. Successive sequences can be inserted at a distance from one another.

Successive sequences can be inserted at a distance from one another. For example, successive recorder sequences can be inserted and separated by 0, 1, 2, 3, 4, 5, 6, 7, 8, 9, 10, 11, 12, 13, 14, 15, 16, 17, 18, 19, 20 ,21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 78, 79, 80, 81, 82, 83, 84, 85, 86, 87, 88, 89, 90, 91, 92, 93, 94, 95, 96, 97, 98, 99, 100, or greater than 100 bp. In some examples, successive recorder sequences are separated by about 10, 50, 100, 150, 200, 250, 300, 350, 400, 450, 500, 550, 600, 650, 700, 750, 800, 850, 900, 950, 1000, 1100, 1200, 1300, 1400, 1500, or greater than 1500bp.

Successive recorder sequences can be separated by any desired number of base pairs and can be dependent and limited on the number of successive recorder sequences to be inserted, the size of the target nucleic acid or target genomes, and/or the design of the desired final recorder sequence. For example, if the compiled recorder sequence is a functional screenable or selectable marker, than the successive recording sequences can be inserted within proximity and within the same reading frame from one another. If the compiled recorder sequence is a unique set of barcodes to be identified by sequencing and have no coding sequence element, then the successive recorder sequences can be inserted with any desired number of base pairs separating them. In these cases, the separation distance can be dependent on the sequencing technology to be used and the read length limit.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

Some definitions

As used herein the term "wild type" is a term of the art understood by skilled persons and means the typical form of an organism, strain, gene or characteristic as it occurs in nature as distinguished from mutant or variant forms.

As used herein the term "variant" should be taken to mean the exhibition of qualities that have a pattern that deviates from what occurs in nature.

The terms "orthologue" (also referred to as "ortholog" herein) and "homologue" (also referred to as "homolog" herein) are well known in the art. By means of further guidance, a "homologue" of a protein as used herein is a protein of the same species which performs the same or a similar function as the protein it is a homologue of. Homologous proteins may but need not be structurally related, or are only partially structurally related. An "orthologue" of a protein as used herein is a protein of a different species which performs the same or a similar function as the protein it is an orthologue of Orthologous proteins may but need not be structurally related, or are only partially structurally related. Homologs and orthologs may be identified by homology modelling (see, e.g., Greer, Science vol. 228 (1985) 1055, and Blundell et al. Eur J Biochem vol 172 (1988), 513) or "structural BLAST" (Dey F, Cliff Zhang Q, Petrey D, Honig B. Toward a "structural BLAST": using structural relationships to infer function. Protein Sci. 2013 April; 22(4):359-66. doi: 10.1002/pro.2225.).

The terms "polynucleotide", "nucleotide", "nucleotide sequence", "nucleic acid" and "oligonucleotide" are used interchangeably. They refer to a polymeric form of nucleotides of any length, either deoxyribonucleotides or ribonucleotides, or analogs thereof. Polynucleotides may have any three dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA, ribosomal RNA, short interfering RNA (siRNA), short-hairpin RNA (shRNA), micro-RNA (miRNA), ribozymes, cDNA, recombinant polynucleotides, branched polynucleotides, plasmids, vectors, isolated DNA of any sequence, isolated RNA of any sequence, nucleic acid probes, and primers. The term also encompasses nucleic-acid-like structures with synthetic backbones, see, e.g., Eckstein, 1991; Baserga et al., 1992; Milligan, 1993; WO 97/03211; WO 96/39154; Mata, 1997; Strauss-Soukup, 1997; and Samstag, 1996. A polynucleotide may comprise one or more modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component.

"Complementarity" refers to the ability of a nucleic acid to form hydrogen bond(s) with another nucleic acid sequence by either traditional Watson-Crick base pairing or other non-traditional types. A percent complementarity indicates the percentage of residues in a nucleic acid molecule which can form hydrogen bonds (e.g., Watson-Crick base pairing) with a second nucleic acid sequence (e.g., 5, 6, 7, 8, 9, 10 out of 10 being 50%, 60%, 70%, 80%, 90%, and 100% complementary). "Perfectly complementary" means that all the contiguous residues of a nucleic acid sequence will hydrogen bond with the same number of contiguous residues in a second nucleic acid sequence. "Substantially complementary" as used herein refers to a degree of complementarity that is at least 60%, 65%, 70%, 75%, 80%, 85%, 90%, 95%, 97%, 98%, 99%, or 100% over a region of 8, 9, 10, 11, 12, 13, 14, 15, 16, 17, 18, 19, 20, 21, 22, 23, 24, 25, 30, 35, 40, 45, 50, or more nucleotides, or refers to two nucleic acids that hybridize under stringent conditions.

As used herein, "stringent conditions" for hybridization refer to conditions under which a nucleic acid having complementarity to a target sequence predominantly hybridizes with the target sequence, and substantially does not hybridize to non-target sequences. Stringent conditions are generally sequence-dependent, and vary depending on a number of factors. In general, the longer the sequence, the higher the temperature at which the sequence specifically hybridizes to its target sequence. Non-limiting examples of stringent conditions are described in detail in Tijssen (1993). Laboratory Techniques In Biochemistry And Molecular Biology-Hybridization With Nucleic Acid Probes Part I, Second Chapter "Overview of principles of hybridization and the strategy of nucleic acid probe assay", Elsevier, N.Y. Where reference is made to a polynucleotide sequence, then complementary or partially complementary sequences are also envisaged. These are preferably capable of hybridising to the reference sequence under highly stringent conditions. Generally, in order to maximize the hybridization rate, relatively low-stringency hybridization conditions are selected: about 20 to 25 degrees Celsius. lower than the thermal melting point (Tm). The Tm is the temperature at which 50% of specific target sequence hybridizes to a perfectly complementary probe in solution at a defined ionic strength and pH. Generally, in order to require at least about 85% nucleotide complementarity of hybridized sequences, highly stringent washing conditions are selected to be about 5 to 15 degrees Celsius lower than the Tm. In order to require at least about 70% nucleotide complementarity of hybridized sequences, moderately-stringent washing conditions are selected to be about 15 to 30 degrees Celsius lower than the Tm. Highly permissive (very low stringency) washing conditions may be as low as 50 degrees Celsius below the Tm, allowing a high level of mis-matching between hybridized sequences. Those skilled in the art will recognize that other physical and chemical parameters in the hybridization and wash stages can also be altered to affect the outcome of a detectable hybridization signal from a specific level of homology between target and probe sequences

"Hybridization" refers to a reaction in which one or more polynucleotides react to form a complex that is stabilized via hydrogen bonding between the bases of the nucleotide residues. The hydrogen bonding may occur by Watson Crick base pairing, Hoogstein binding, or in any other sequence specific manner. The complex may comprise two strands forming a duplex structure, three or more strands forming a multi stranded complex, a single self-hybridizing strand, or any combination of these. A hybridization reaction may constitute a step in a more extensive process, such as the initiation of PCR, or the cleavage of a polynucleotide by an enzyme. A sequence capable of hybridizing with a given sequence is referred to as the "complement" of the given sequence.

As used herein, the term "genomic locus" or "locus" (plural loci) is the specific location of a gene or DNA sequence on a chromosome. A "gene" refers to stretches of DNA or RNA that encode a polypeptide or an RNA chain that has functional role to play in an organism and hence is the molecular unit of heredity in living organisms. For the purpose of this invention, it may be considered that genes include regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites and locus control regions.

As used herein, "expression of a genomic locus" or "gene expression" is the process by which information from a gene is used in the synthesis of a functional gene product. The products of gene expression are often proteins, but in non-protein coding genes such as rRNA genes or tRNA genes, the product is functional RNA. The process of gene expression is used by all known life--eukaryotes (including multicellular organisms), prokaryotes (bacteria and archaea) and viruses to generate functional products to survive. As used herein "expression" of a gene or nucleic acid encompasses not only cellular gene expression, but also the transcription and translation of nucleic acid(s) in cloning systems and in any other context. As used herein, "expression" also refers to the process by which a polynucleotide is transcribed from a DNA template (such as into and mRNA or other RNA transcript) and/or the process by which a transcribed mRNA is subsequently translated into peptides, polypeptides, or proteins. Transcripts and encoded polypeptides may be collectively referred to as "gene product." If the polynucleotide is derived from genomic DNA, expression may include splicing of the mRNA in a eukaryotic cell.

The terms "polypeptide", "peptide" and "protein" are used interchangeably herein to refer to polymers of amino acids of any length. The polymer may be linear or branched, it may comprise modified amino acids, and it may be interrupted by non-amino acids. The terms also encompass an amino acid polymer that has been modified; for example, disulfide bond formation, glycosylation, lipidation, acetylation, phosphorylation, or any other manipulation, such as conjugation with a labeling component. As used herein the term "amino acid" includes natural and/or unnatural or synthetic amino acids, including glycine and both the D or L optical isomers, and amino acid analogs and peptidomimetics.

As used herein, the term "domain" or "protein domain" refers to a part of a protein sequence that may exist and function independently of the rest of the protein chain.

As described in aspects of the invention, sequence identity is related to sequence homology. Homology comparisons may be conducted by eye, or more usually, with the aid of readily available sequence comparison programs. These commercially available computer programs may calculate percent (%) homology between two or more sequences and may also calculate the sequence identity shared by two or more amino acid or nucleic acid sequences. Sequence homologies may be generated by any of a number of computer programs known in the art, for example BLAST or FASTA, etc. A suitable computer program for carrying out such an alignment is the GCG Wisconsin Bestfit package (University of Wisconsin. U.S.A; Devereux et al., 1984, Nucleic Acids Research 12:387). Examples of other software than may perform sequence comparisons include, but are not limited to, the BLAST package (see Ausubel et al., 1999 ibid--Chapter 18), FASTA (Atschul et al., 1990, J. Mol. Biol., 403-410) and the GENEWORKS suite of comparison tools. Both BLAST and FASTA are available for offline and online searching (see Ausubel et al, 1999 ibid, pages 7-58 to 7-60). However it is preferred to use the GCG Bestfit program.

Percent homology may be calculated over contiguous sequences, i.e., one sequence is aligned with the other sequence and each amino acid or nucleotide in one sequence is directly compared with the corresponding amino acid or nucleotide in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion may cause the following amino acid residues to be put out of alignment, thus potentially resulting in a large reduction in % homology when a global alignment is performed.

Consequently, most sequence comparison methods are designed to produce optimal alignments that take into consideration possible insertions and deletions without unduly penalizing the overall homology or identity score. This is achieved by inserting "gaps" in the sequence alignment to try to maximize local homology or identity.

However, these more complex methods assign "gap penalties" to each gap that occurs in the alignment so that, for the same number of identical amino acids, a sequence alignment with as few gaps as possible--reflecting higher relatedness between the two compared sequences--may achieve a higher score than one with many gaps. "Affinity gap costs" are typically used that charge a relatively high cost for the existence of a gap and a smaller penalty for each subsequent residue in the gap. This is the most commonly used gap scoring system. High gap penalties may, of course, produce optimized alignments with fewer gaps. Most alignment programs allow the gap penalties to be modified. However, it is preferred to use the default values when using such software for sequence comparisons. For example, when using the GCG Wisconsin Bestfit package the default gap penalty for amino acid sequences is -12 for a gap and -4 for each extension.

Calculation of maximum % homology therefore first requires the production of an optimal alignment, taking into consideration gap penalties. A suitable computer program for carrying out such an alignment is the GCG Wisconsin Bestfit package (Devereux et al., 1984 Nuc. Acids Research 12 p387). Examples of other software that may perform sequence comparisons include, but are not limited to, the BLAST package (see Ausubel et al., 1999 Short Protocols in Molecular Biology, 4th Ed.--Chapter 18), FASTA (Altschul et al., 1990 J. Mol. Biol. 403-410) and the GENEWORKS suite of comparison tools. Both BLAST and FASTA are available for offline and online searching (see Ausubel et al., 1999, Short Protocols in Molecular Biology, pages 7-58 to 7-60). However, for some applications, it is preferred to use the GCG Bestfit program. A new tool, called BLAST 2 Sequences is also available for comparing protein and nucleotide sequences (see FEMS Microbiol Lett. 1999 174(2): 247-50; FEMS Microbiol Lett. 1999 177(1): 187-8 and the website of the National Center for Biotechnology information at the website of the National Institutes for Health).

Although the final % homology may be measured in terms of identity, the alignment process itself is typically not based on an all-or-nothing pair comparison. Instead, a scaled similarity score matrix is generally used that assigns scores to each pair-wise comparison based on chemical similarity or evolutionary distance. An example of such a matrix commonly used is the BLOSUM62 matrix--the default matrix for the BLAST suite of programs. GCG Wisconsin programs generally use either the public default values or a custom symbol comparison table, if supplied (see user manual for further details). For some applications, it is preferred to use the public default values for the GCG package, or in the case of other software, the default matrix, such as BLOSUM62.

Alternatively, percentage homologies may be calculated using the multiple alignment feature in DNASIS™ (Hitachi Software), based on an algorithm, analogous to CLUSTAL (Higgins D G & Sharp P M (1988), Gene 73(1), 237-244). Once the software has produced an optimal alignment, it is possible to calculate % homology, preferably % sequence identity. The software typically does this as part of the sequence comparison and generates a numerical result.

Sequences may also have deletions, insertions or substitutions of amino acid residues which produce a silent change and result in a functionally equivalent substance. Deliberate amino acid substitutions may be made on the basis of similarity in amino acid properties (such as polarity, charge, solubility, hydrophobicity, hydrophilicity, and/or the amphipathic nature of the residues) and it is therefore useful to group amino acids together in functional groups. Amino acids may be grouped together based on the properties of their side chains alone. However, it is more useful to include mutation data as well. The sets of amino acids thus derived are likely to be conserved for structural reasons. These sets may be described in the form of a Venn diagram (Livingstone C. D. and Barton G. J. (1993) "Protein sequence alignments: a strategy for the hierarchical analysis of residue conservation" Comput. Appl. Biosci. 9: 745-756) (Taylor W. R. (1986) "The classification of amino acid conservation" J. Theor. Biol. 119; 205-218). Conservative substitutions may be made, for example according to the table below which describes a generally accepted Venn diagram grouping of amino acids.

Embodiments of the invention include sequences (both polynucleotide or polypeptide) which may comprise homologous substitution (substitution and replacement are both used herein to mean the interchange of an existing amino acid residue or nucleotide, with an alternative residue or nucleotide) that may occur i.e., like-for-like substitution in the case of amino acids such as basic for basic, acidic for acidic, polar for polar, etc. Non-homologous substitution may also occur i.e., from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyridylalanine, thienylalanine, naphthylalanine and phenylglycine.

Variant amino acid sequences may include suitable spacer groups that may be inserted between any two amino acid residues of the sequence including alkyl groups such as methyl, ethyl or propyl groups in addition to amino acid spacers such as glycine or beta.-alanine residues. A further form of variation, which involves the presence of one or more amino acid residues in peptoid form, may be well understood by those skilled in the art. For the avoidance of doubt, "the peptoid form" is used to refer to variant amino acid residues wherein the alpha.-carbon substituent group is on the residue's nitrogen atom rather than the .alpha.-carbon. Processes for preparing peptides in the peptoid form are known in the art, for example Simon R J et al., PNAS (1992) 89(20), 9367-9371 and Horwell D C, Trends Biotechnol. (1995) 13(4), 132-134.

The practice of the present invention employs, unless otherwise indicated, conventional techniques of immunology, biochemistry, chemistry, molecular biology, microbiology, cell biology, genomics and recombinant DNA, which are within the skill of the art. See Green and Sambrook, (Molecular Cloning: A Laboratory Manual. 4th, ed., Cold Spring Harbor Laboratory Press, Cold Spring Harbor, N.Y., 2014); CURRENT PROTOCOLS IN MOLECULAR BIOLOGY (F. M. Ausubel, et al. eds., (2017)); Short Protocols in Molecular Biology, (Ausubel et al., 1999)); the series METHODS IN ENZYMOLOGY (Academic Press, Inc.): PCR 2: A PRACTICAL APPROACH (M. J. MacPherson, B. D. Hames and G. R. Taylor eds. (1995)), ANTIBODIES, A LABORATORY MANUAL, SECOND EDITION (Harlow and Lane, eds. (2014) and CULTURE or ANIMAL CELLS : A MANUAL OF BASIC TECHNIQUE, 7TH EDITION (R. I. Freshney, ed. (2016)).

EXAMPLES

Example 1. Nucleic acid-guided nucleases

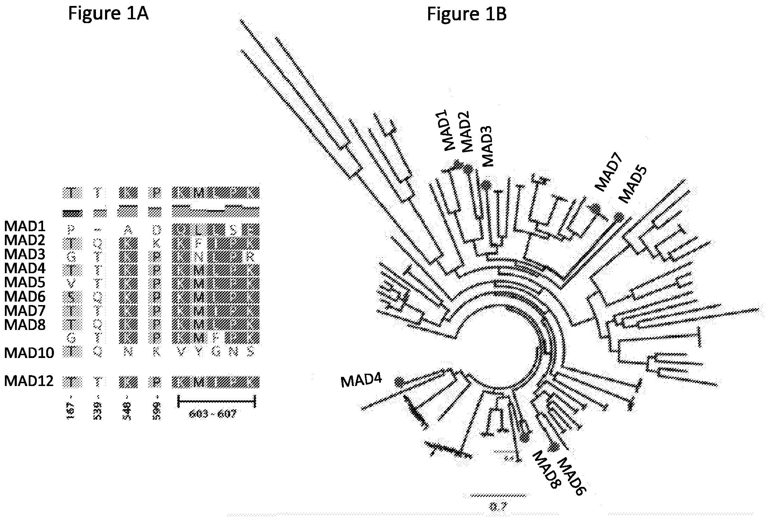

Sequences for twenty nucleic acid guided nucleases, termed MAD1-MAD20 (SEQ ID NOs 1-20), were aligned and compared to other nucleic acid guided nucleases. A partial alignment of specific amino acids of the nucleases and a phylogenetic tree are depicted in Figure 1A and Figure 1B respectively. Key residues in that may be involved in the recognition of a PAM site are shown in Figure 1A. These include the amino acids at positions 167, 539, 548, 599, 603, 604, 605, 606, and 607.

Sequence alignments were built using PSI-BLAST to search for MAD nuclease homologs in the NCBI non-redundant databases. Multiple sequence alignments were further refined using the MUSCLE alignment algorithm with default settings as implemented in Geneious 10. The percent identity of each homolog to SpCas9 and AsCpf1 reference sequences were computed based on the pairwise alignment matching from these global alignments.

Genomic source sequences were identified using Uniprot linkage information or TBLASTN searches of NCBI using the default parameters and searching all possible frames for translational matches.

Percent identities of MAD1-8 and 10-12 to other various nucleases are summarized in Table 1. These percent identities represent the shared amino acid sequence identity between the indicated proteins.

Table 1

| Protein identifier or accession number | MAD 1 | MAD2 | MAD3 | MAD4 | MAD5 | MAD6 | MAD7 | MAD8 | MAD10 | MAD11 | MAD12 |
|---|---|---|---|---|---|---|---|---|---|---|---|
| gi\|1025734861\|pdb\|5B 43\|A | 6.4 | 32.8 | 33.2 | 29.7 | 29.4 | 31.1 | 30.3 | 31.7 | 26.7 | 27.9 | 98.8 |
| gi\|1052245173\|pdb\|5K K5\|A | 6.4 | 32.7 | 33.1 | 29.7 | 29.3 | 31 | 30.3 | 31.7 | 26.7 | 27.8 | 98.7 |
| gi\|1086216683\|emb\|S DC16215.1\| | 6.1 | 33 | 34.4 | 29.6 | 30.1 | 33.5 | 32.3 | 32.1 | 26.2 | 27.2 | 46.8 |
| gi\|1120175333\|ref\|WP_073043853.1 | 5.9 | 30.9 | 37.2 | 32.8 | 33.6 | 34.4 | 35.7 | 35.1 | 26.3 | 28.3 | 34.9 |
| Cpf1.Sj \|WP_081839471 | 6.6 | 33.6 | 41.7 | 37.2 | 33.4 | 37.6 | 40.1 | 37.7 | 29.1 | 30.3 | 34.1 |
| Cpf1.Ss\|KFO67989 | 6.9 | 32.3 | 35.7 | 43 | 33.7 | 45.9 | 34.8 | 48 | 33.2 | 33.4 | 33.8 |
| MAD3 | 5.8 | 31 | 100 | 32.9 | 35.9 | 35 | 35.6 | 34.3 | 28 | 27.6 | 33.1 |
| gi\|1082474576\|gb\|OF Y19591.1\| | 7 | 31.4 | 35.9 | 43.2 | 31.4 | 45 | 33.6 | 48.6 | 30.8 | 33.5 | 33 |
| MAD2 | 6.1 | 100 | 31 | 30.7 | 30.2 | 31 | 31.2 | 31.2 | 25.8 | 27.7 | 32.6 |
| Cpf1.Lb5\|WP_016301 126 | 7.8 | 32.8 | 36.5 | 38.2 | 34.2 | 45.5 | 35.8 | 43.6 | 30.7 | 35.7 | 32.5 |
| gi\|1082286736\|gb\|OH B41002.1\| | 6.7 | 30.6 | 35.3 | 42.4 | 33.2 | 44.7 | 32.1 | 46.8 | 30.7 | 32.6 | 32.4 |
| gi\|1094423310\|emb\|S ER03894.1\| | 6.8 | 30.8 | 36.1 | 40.4 | 31.8 | 50.4 | 35.2 | 46.6 | 30.4 | 36.8 | 32.3 |
| gi\|493326531\|ref\|WP_006283774.1\| | 6.8 | 30.8 | 36.1 | 40.3 | 31.8 | 50.3 | 35.1 | 46.6 | 30.4 | 36.8 | 32.3 |
| MAD8 | 7.6 | 31.2 | 34.3 | 40.4 | 32 | 41.6 | 32.8 | 100 | 30.1 | 32.1 | 31.7 |
| Cpf1.Bot\|WP_009217 842 | 6.9 | 30.1 | 36.6 | 41.5 | 32.5 | 50.2 | 35.4 | 45.5 | 29.8 | 34.1 | 31.6 |
| Cpf1.Li\|WP_0209887 26 | 7.3 | 30.2 | 34.6 | 39.3 | 30.3 | 40.7 | 31.8 | 39.4 | 32.1 | 31.3 | 31.5 |
| Cpf1.Pb\|WP_0441101 23 | 6.3 | 31.4 | 31.8 | 36.1 | 30.8 | 45.7 | 30.4 | 39.4 | 27.7 | 33.5 | 31.5 |
| gi\|817911372\|gb\|AKG 08867.1\| | 7.3 | 29.8 | 35 | 40.7 | 32.1 | 40.3 | 32.6 | 41.7 | 29.1 | 31 | 31.4 |
| gi\|1052838533\|emb\|S CH45297.1\| | 6.6 | 30.8 | 35.5 | 32 | 31.5 | 34.4 | 51.9 | 33.4 | 26.1 | 29 | 31.3 |
| gi\|1053713332\|ref\|WP_066040075.1\| | 7.2 | 29.6 | 33.2 | 39.6 | 29.8 | 49.1 | 32.2 | 41.4 | 30.1 | 32.4 | 31.3 |
| gi\|817909002\|gb\|AKG 06878.1\| | 7.3 | 29.8 | 35 | 40.7 | 32 | 40.3 | 32.5 | 41.6 | 29.1 | 30.9 | 31.3 |
| gi\|1042201477\|ref\|WP_065256572.1\| | 7.2 | 29.5 | 35.2 | 40.6 | 31.9 | 40.1 | 32.7 | 41.6 | 29 | 30.8 | 31.2 |
| MAD6 | 7.5 | 31 | 35 | 38.9 | 33.1 | 100 | 34.3 | 41.6 | 30.5 | 33.6 | 31 |
| gi\|490468773\|ref\|WP_004339290.1\| | 6.8 | 31.8 | 31.7 | 36.2 | 28.6 | 36.5 | 31.4 | 38.4 | 28.5 | 31.4 | 31 |
| gi\|565853704\|ref\|WP_023936172.1\| | 7.5 | 30.8 | 34.9 | 38.9 | 33.1 | 99.7 | 34.1 | 41.6 | 30.4 | 33.6 | 31 |

| Protein identifier or accession number | MAD 1 | MAD2 | MAD3 | MAD4 | MAD5 | MAD6 | MAD7 | MAD8 | MAD10 | MAD11 | MAD12 |
|---|---|---|---|---|---|---|---|---|---|---|---|
| gi\|739005707\|ref\|WP_036887416.1\| | 7.5 | 30.9 | 35 | 38.9 | 33 | 99.9 | 34.2 | 41.5 | 30.4 | 33.5 | 31 |
| gi\|739008549\|ref\|WP_036890108.1\| | 7.5 | 31 | 35 | 38.8 | 33 | 99.8 | 34.2 | 41.5 | 30.4 | 33.5 | 31 |
| Cpf1.Ft\|WP_0145500 95 | 7.1 | 31.9 | 33.8 | 40.3 | 29.7 | 39.4 | 34.1 | 41 | 29.8 | 32.5 | 30.8 |
| gi\|504362993\|ref\|WP_014550095.1\| | 7.2 | 32.4 | 33.8 | 40.3 | 29.6 | 39.4 | 33.8 | 40.9 | 30.1 | 32.5 | 30.8 |
| gi\|640557447\|ref\|WP_024988992.1\| | 6.6 | 31.4 | 34.8 | 40.7 | 31.2 | 48 | 34.1 | 45.1 | 28.8 | 35.2 | 30.8 |
| gi\|1098944113\|ref\|WP_071304624.1\| | 7.1 | 32.3 | 33.5 | 40.3 | 29.6 | 39.2 | 33.8 | 40.9 | 30.1 | 32.5 | 30.6 |
| gi\|489124848\|ref\|WP_003034647.1\| | 7.1 | 32.3 | 33.9 | 40.9 | 29.9 | 39.2 | 33.9 | 40.9 | 29.9 | 32.2 | 30.6 |
| gi\|738967776\|ref\|WP_036851563.1\| | 6.8 | 29.4 | 33.1 | 35.5 | 28.9 | 40.3 | 30.7 | 35.9 | 28.7 | 31.3 | 30.5 |
| MAD7 | 5.9 | 31.2 | 35.6 | 30.8 | 33.9 | 34.3 | 100 | 32.8 | 24.2 | 28.9 | 30.5 |
| Cpf1.Lb6\|WP_044910 713 | 6.7 | 29.8 | 33.7 | 36.6 | 30.9 | 43 | 34 | 39.8 | 29.1 | 32.1 | 30.4 |
| gi\|1052961977\|emb\|S CH47915.1\| | 5.5 | 30.5 | 35.8 | 32.3 | 34 | 35 | 53.8 | 33.4 | 26.2 | 27.4 | 30.4 |
| gi\|817918353\|gb\|AKG 14689.1\| | 7 | 29.1 | 34.4 | 39.8 | 31.7 | 40 | 32.4 | 41.1 | 28.4 | 30.1 | 30.3 |
| gi\|917059416\|ref\|WP_051666128.1\| | 6.9 | 29.9 | 31.5 | 35.7 | 31.6 | 41.8 | 32.9 | 39.1 | 30.1 | 34 | 30.2 |
| gi\|1011649201\|ref\|WP_062499108.1\| | 6.8 | 29 | 34.7 | 40.3 | 31.4 | 40.1 | 33.1 | 41.6 | 28.5 | 30.4 | 30.1 |
| Cpf1.Pm\|WP_018359 861 | 6.3 | 29.2 | 32.3 | 34.2 | 27.4 | 38.7 | 29.4 | 35 | 27.2 | 30.1 | 30 |
| gi\|817922537\|gb\|AKG 18099.1\| | 6.8 | 29.1 | 34.5 | 39.6 | 31.5 | 39.9 | 32.7 | 40.7 | 28.3 | 29.8 | 30 |
| gi\|769142322\|ref\|WP_044919442.1\| | 6.7 | 31 | 34.6 | 37.8 | 31.5 | 41.4 | 33.3 | 39.2 | 28 | 31.9 | 29.9 |
| gi\|1023176441\|pdb\|5I | 6.7 | 29.7 | 31.3 | 35.5 | 31.3 | 41 | 32.6 | 38.5 | 29.7 | 33.3 | 29.8 |
| D6\|A | | | | | | | | | | | |
| gi\|491540987\|ref\|WP_005398606.1\| | 5.9 | 28.3 | 30.4 | 29.7 | 28.5 | 29 | 30.7 | 29.8 | 25.8 | 27.8 | 29.8 |
| gi\|652820612\|ref\|WP_027109509.1\| | 6.4 | 31.1 | 34 | 35.3 | 31.7 | 40.3 | 33.4 | 37.5 | 28.5 | 33.3 | 29.8 |
| gi\|502240446\|ref\|WP_012739647.1\| | 5.9 | 31.6 | 36.1 | 31.2 | 33 | 35.4 | 49.4 | 34 | 26.6 | 29.4 | 29.7 |
| gi\|524278046\|emb\|CD A41776.1\| | 5.8 | 31.6 | 36 | 31 | 33 | 35.4 | 50 | 34 | 26.6 | 29.5 | 29.7 |
| gi\|737831580\|ref\|WP_035798880.1\| | 6.2 | 31.3 | 34.8 | 38.1 | 31.5 | 42.1 | 33 | 39.6 | 28.4 | 32.4 | 29.7 |
| gi\|909652572\|ref\|WP_049895985.1\| | 6.9 | 30.7 | 34.2 | 37.2 | 30.8 | 41.5 | 34.2 | 38.7 | 28 | 32 | 29.7 |

| Protein identifier or accession number | MAD 1 | MAD2 | MAD3 | MAD4 | MAD5 | MAD6 | MAD7 | MAD8 | MAD10 | MAD11 | MAD12 |
|---|---|---|---|---|---|---|---|---|---|---|---|
| MAD4 | 6.7 | 30.7 | 32.9 | 100 | 30.7 | 38.9 | 30.8 | 40.4 | 28.8 | 29.4 | 29.7 |
| gi\|942073049\|ref\|WP_ 055286279.1\| | 5.9 | 31.6 | 36.1 | 31.1 | 32.7 | 35 | 49.7 | 33.9 | 27.1 | 29.5 | 29.6 |
| gi\|654794505\|ref\|WP_ 028248456.1\| | 7.4 | 30.5 | 35.9 | 37.4 | 31.3 | 42.8 | 34.2 | 40.2 | 27.9 | 33.5 | 29.5 |
| gi\|933014786\|emb\|CU O47728.1\| | 5.6 | 31.3 | 34.9 | 31.2 | 31.5 | 32.4 | 46.7 | 30.6 | 25.4 | 27.7 | 29.4 |
| gi\|941887450\|ref\|WP_ 055224182.1\| | 5.6 | 31.4 | 35 | 31.3 | 31.6 | 32.5 | 46.6 | 30.7 | 25.3 | 27.8 | 29.4 |
| gi\|920071674\|ref\|WP_ 052943011.1\| | 6.3 | 31 | 31.8 | 38.8 | 31.8 | 41.3 | 33.8 | 42.6 | 29.8 | 34.7 | 29 |
| MAD5 | 5.1 | 30.2 | 35.9 | 30.7 | 100 | 33.1 | 33.9 | 32 | 24.3 | 28.7 | 29 |
| gi\|1081462674\|emb\|S CZ76797.1\| | 6.9 | 30.4 | 33.5 | 34.7 | 29.7 | 40.1 | 30.5 | 37.4 | 27.3 | 32.5 | 28.9 |
| gi\|918722523\|ref\|WP_ 052585281.1\| | 7.4 | 27.5 | 30.5 | 35.7 | 28.3 | 35.2 | 28.5 | 36 | 26 | 27.1 | 28.8 |
| gi\|524816323\|emb\|CD F09621.1\| | 6.2 | 30 | 34.1 | 29.3 | 31.2 | 32.7 | 47.6 | 32.2 | 25.5 | 25.9 | 28.4 |
| gi\|941782328\|ref\|WP_ 055176369.1\| | 6.2 | 30.2 | 33.1 | 28.9 | 30.9 | 32 | 46.9 | 32.1 | 26 | 27.1 | 28.4 |
| gi\|942113296\|ref\|WP_ 055306762.1\| | 6.4 | 29.8 | 33.8 | 29.7 | 31.3 | 33.1 | 48 | 32.5 | 25.8 | 26.2 | 28.4 |
| MAD11 | 6.4 | 27.7 | 27.6 | 29.4 | 28.7 | 33.6 | 28.9 | 32.1 | 26.2 | 100 | 27.8 |
| gi\|653158548\|ref\|WP_ 027407524.1\| | 5.9 | 26.4 | 28.1 | 33.5 | 27.4 | 32.5 | 27.8 | 32 | 27 | 26.8 | 27.6 |
| gi\|652963004\|ref\|WP_ 027216152.1\| | 6.6 | 30.3 | 32.5 | 33.2 | 30.4 | 38.2 | 29.6 | 34.6 | 25.9 | 30.5 | 27.2 |
| gi\|1083069650\|gb\|OG D68774.1\| | 6.2 | 25 | 24.3 | 26.6 | 23.1 | 28.1 | 23.2 | 26.4 | 45 | 24.9 | 27.1 |
| gi\|302483275\|gb\|EFL4 6285.1\| | 5.6 | 24.7 | 26.8 | 30.3 | 24.9 | 34.8 | 26 | 30.4 | 24.4 | 27.5 | 27.1 |
| gi\|915400855\|ref\|WP_ 050786240.1\| | 5.6 | 24.7 | 26.8 | 30.3 | 24.9 | 34.8 | 26 | 30.4 | 24.4 | 27.5 | 27.1 |
| MAD10 | 5.6 | 25.8 | 28 | 28.8 | 24.3 | 30.5 | 24.2 | 30.1 | 100 | 26.2 | 26.6 |
| gi\|1101117967\|gb\|OIO 75780.1\| | 6.1 | 26.8 | 26 | 27.3 | 24.3 | 28.1 | 24.4 | 28.2 | 44.1 | 25.4 | 26.1 |
| gi\|1088204458\|gb\|OH A63117.1\| | 6.5 | 25.2 | 23.5 | 25.8 | 22.9 | 27 | 22 | 26.1 | 36.5 | 24.2 | 24.7 |
| gi\|809198071\|ref\|WP_ 046328599.1\| | 4.9 | 25.6 | 26.5 | 22.2 | 23.9 | 23.8 | 25.8 | 23.9 | 20.3 | 25.1 | 24 |
| gi\|1088079929\|gb\|OG Z45678.1\| | 5.6 | 21.9 | 23.8 | 26.9 | 23.4 | 27.8 | 23.3 | 26.7 | 28.8 | 24.7 | 23.5 |
| gi\|1101053499\|gb\|OIO 15737.1\| | 5.9 | 23.1 | 26.2 | 25.2 | 23 | 26.4 | 25.1 | 26.5 | 29.2 | 23.2 | 23.4 |

(continued)

| Protein identifier or accession number | MAD 1 | MAD2 | MAD3 | MAD4 | MAD5 | MAD6 | MAD7 | MAD8 | MAD10 | MAD11 | MAD12 |
|---|---|---|---|---|---|---|---|---|---|---|---|
| gi\|1101058058\|gb\|OIO 19978.1\| | 5.4 | 21.2 | 22.8 | 23.6 | 20.6 | 25 | 20.7 | 25 | 25.9 | 22.2 | 23 |
| gi\|1088000848\|gb\|OG Y73485.1\| | 5.7 | 23.5 | 25.2 | 25.5 | 23.9 | 27 | 25.1 | 25.6 | 31.6 | 23.6 | 22.9 |
| gi\|407014433\|gb\|EKE 28449.1\| | 5.2 | 23.5 | 25.9 | 26.7 | 24.3 | 25.8 | 23 | 27.8 | 29.9 | 25.3 | 22.9 |
| gi\|818249855\|gb\|KKP 36646.1\| | 6 | 21 | 20.7 | 23.5 | 20 | 24.2 | 21 | 24 | 24.6 | 21.8 | 22.6 |
| gi\|818703647\|gb\|KKT 48220.1\| | 5.8 | 23.3 | 25 | 25.1 | 23.5 | 26.5 | 24.7 | 25.3 | 31.2 | 23.3 | 22.6 |
| gi\|818705786\|gb\|KKT 50231.1\| | 5.8 | 23.1 | 24.6 | 24.7 | 22.9 | 26.2 | 24.2 | 24.8 | 30.8 | 22.9 | 22.2 |
| gi\|1083950632\|gb\|OGJ 66851.1\| | 4.5 | 20 | 22.1 | 23.5 | 20.6 | 24.6 | 20 | 24 | 23.5 | 20.7 | 22.1 |
| gi\|1083932199\|gb\|OGJ 49885.1\| | 6 | 20.4 | 20.2 | 22.6 | 19.3 | 23.3 | 20.6 | 23.2 | 23.9 | 21 | 21.8 |
| gi\|1083410735\|gb\|OG F20863.1\| | 5 | 21.7 | 23.3 | 25.5 | 23 | 25 | 22.7 | 25.9 | 27.2 | 22.4 | 21.5 |
| gi\|1011480927\|ref\|WP _062376669.1\| | 4.7 | 20.1 | 20.1 | 21.4 | 19.3 | 23.3 | 21.4 | 22 | 20.2 | 19.7 | 20.9 |
| gi\|818539593\|gb\|KKR 91555.1\| | 5.1 | 19.8 | 21.6 | 22.1 | 20.5 | 22.9 | 21.2 | 22.8 | 24 | 20.5 | 19.9 |
| gi\|503048015\|ref\|WP_ 013282991.1\| | 5.1 | 18.8 | 20.7 | 15.3 | 19.7 | 18.9 | 19.3 | 17.7 | 15.9 | 19 | 19.2 |
| gi\|1096232746\|ref\|WP _071177645.1\| | 5 | 19.1 | 20.5 | 17.4 | 20.1 | 19.7 | 20.4 | 20.4 | 17.5 | 18.5 | 18.9 |
| gi\|769130404\|ref\|WP_ 044910712.1\| | 4.6 | 19.4 | 18.2 | 16.1 | 18.1 | 17.1 | 18.7 | 17.9 | 14.5 | 16.8 | 17.5 |
| gi\|1085569500\|gb\|OG X23684.1\| | 2.6 | 11.6 | 12.1 | 12.7 | 10.2 | 12.1 | 12.7 | 11.6 | 10.9 | 11.1 | 10.5 |
| gi\|818357062\|gb\|KKQ 38176.1\| | 3.3 | 10 | 11.1 | 10.6 | 11.1 | 11.8 | 12.1 | 11.5 | 12.2 | 10.8 | 9.8 |
| gi\|745626763\|gb\|KIE1 8642.1\| | 3.7 | 9.4 | 11.7 | 11.1 | 11.1 | 12.5 | 11.9 | 11.9 | 10.2 | 10.6 | 8.8 |
| MAD1 | 100 | 6.1 | 5.8 | 6.7 | 5.1 | 7.5 | 5.9 | 7.6 | 5.6 | 6.4 | 6.4 |
| SpCas9 | 4 | 6.3 | 6.5 | 8.3 | 5.6 | 8.1 | 6.9 | 7.7 | 6.9 | 6.3 | 6.3 |
| MAD12 | 6.4 | 32.6 | 33.1 | 29.7 | 29 | 31 | 30.5 | 31.7 | 26.6 | 27.8 | 100 |

Example 2: Expression of MAD nucleases

Wild-type nucleic acid sequences for MAD1-MAD20 include SEQ ID NOs 21-40, respectively. These MAD nucleases were codon optimized for expression in *E. coli* and the codon optimized sequences are listed as SEQ ID NO: 41-60 (summarized in Table 2).

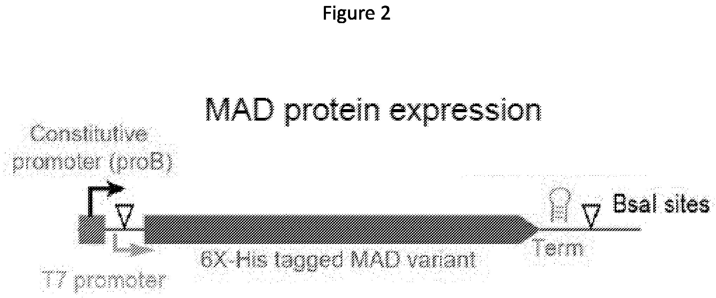

These codon optimized MAD1-MAD20 were cloned into an expression construct comprising a constitutive or inducible promoter (e.g., proB promoter SEQ ID NO: 83, or pBAD promoter SEQ ID NO: 81 or SEQ ID NO: 82) and an optional 6X-His tag (SEQ ID NO: 376) (e.g., Figure 2). The generated MAD1-MAD20 expression constructs are provided as SEQ ID NOs: 61-80, respectively. The expression constructs as depicted in Figure 2 were generated either by restriction/ligation-based cloning or homology-based cloning.

Example 3. Testing guide nucleic acid sequences compatible with MAD nucleases

A nucleic acid-guided nuclease and a compatible guide nucleic acid is needed to have a functioning targetable nuclease complex. Multiple approaches were taken to determine the compatible guide nucleic acid sequence, and specifically the scaffold sequence portion of the guide nucleic acid. First, the endogenous loci of each MAD nuclease were scanned for potential scaffold sequences. In some cases, such as with MAD2, no endogenous scaffold sequence was found. Therefore, the compatibility of MAD2 with scaffold sequences found near the endogenous loci of the other MAD nucleases was tested. The MAD nucleases and corresponding endogenous scaffold sequences that were tested are listed in Table 2.

Table 2.

| MAD nuclease | WT nucleic acid sequence | Codon optimized *E. Coli* nucleic acid sequence | Amino acid sequence | Endogenous scaffold sequence for guide nucleic acid |
|---|---|---|---|---|
| MAD1 | SEQ ID NO: 21 | SEQ ID NO: 41 | SEQ ID NO: 1 | SEQ ID NO: 84 |
| MAD2 | SEQ ID NO: 22 | SEQ ID NO: 42 | SEQ ID NO: 2 | None identified |
| MAD3 | SEQ ID NO: 23 | SEQ ID NO: 43 | SEQ ID NO: 3 | SEQ ID NO: 86 |
| MAD4 | SEQ ID NO: 24 | SEQ ID NO: 44 | SEQ ID NO: 4 | SEQ ID NO: 87 |
| MAD5 | SEQ ID NO: 25 | SEQ ID NO: 45 | SEQ ID NO: 5 | SEQ ID NO: 88 |
| MAD6 | SEQ ID NO: 26 | SEQ ID NO: 46 | SEQ ID NO: 6 | SEQ ID NO: 89 |
| MAD7 | SEQ ID NO: 27 | SEQ ID NO: 47 | SEQ ID NO: 7 | SEQ ID NO: 90 |
| MAD8 | SEQ ID NO: 28 | SEQ ID NO: 48 | SEQ ID NO: 8 | SEQ ID NO: 91 |
| MAD9 | SEQ ID NO: 29 | SEQ ID NO: 49 | SEQ ID NO: 9 | SEQ ID NO: 92; SEQ ID NO: 103; SEQ ID NO: 106 |
| MAD10 | SEQ ID NO: 30 | SEQ ID NO: 50 | SEQ ID NO: 10 | SEQ ID NO: 93 |
| MAD11 | SEQ ID NO: 31 | SEQ ID NO: 51 | SEQ ID NO: 11 | SEQ ID NO: 94 |
| MAD12 | SEQ ID NO: 32 | SEQ ID NO: 52 | SEQ ID NO: 12 | SEQ ID NO: 95 |
| MAD13 | SEQ ID NO: 33 | SEQ ID NO: 53 | SEQ ID NO: 13 | SEQ ID NO: 96; SEQ ID NO: 105; SEQ ID NO: 107 |
| MAD14 | SEQ ID NO: 34 | SEQ ID NO: 54 | SEQ ID NO: 14 | SEQ ID NO: 97 |
| MAD15 | SEQ ID NO: 35 | SEQ ID NO: 55 | SEQ ID NO: 15 | SEQ ID NO: 98 |
| MAD16 | SEQ ID NO: 36 | SEQ ID NO: 56 | SEQ ID NO: 16 | SEQ ID NO: 99 |
| MAD17 | SEQ ID NO: 37 | SEQ ID NO: 57 | SEQ ID NO: 17 | SEQ ID NO: 100 |
| MAD18 | SEQ ID NO: 38 | SEQ ID NO: 58 | SEQ ID NO: 18 | SEQ ID NO: 101 |
| MAD19 | SEQ ID NO: 39 | SEQ ID NO: 59 | SEQ ID NO: 19 | SEQ ID NO: 102 |
| MAD20 | SEQ ID NO: 40 | SEQ ID NO: 60 | SEQ ID NO: 20 | SEQ ID NO: 103 |

Editing cassettes as depicted in Figure 3 were generated to assess the functionality of the MAD nucleases and corresponding guide nucleic acids. Each editing cassette comprises an editing sequence and a promoter operably linked to an encoded guide nucleic acid. The editing cassettes further comprises primer sites (P1 and P2) on flanking ends. The guide nucleic acids comprised various scaffold sequences to be tested, as well as a guide sequence to guide the MAD nuclease to the target sequence for editing. The editing sequences comprised a PAM mutation and/or codon mutation relative to the target sequence. The mutations were flanked by regions of homology (homology arms or HA) which would allow recombination into the cleaved target sequence.

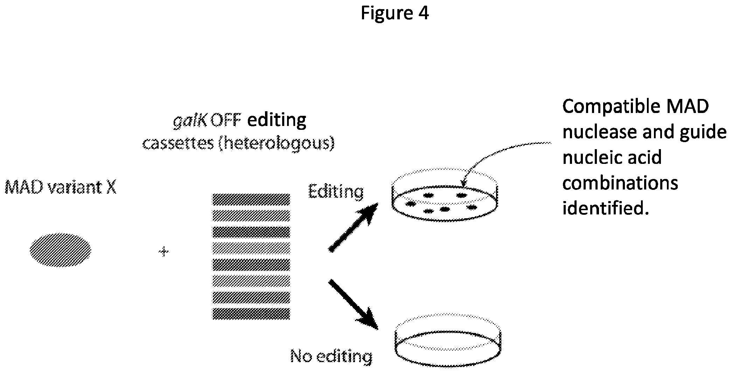

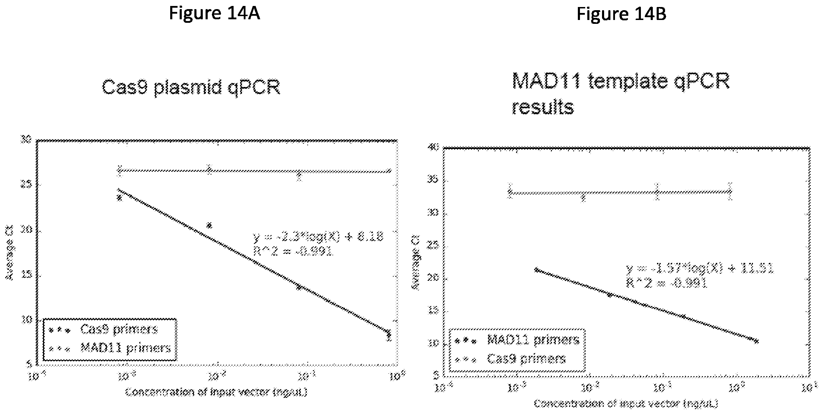

Figure 4 depicts an experiment designed to test different MAD nuclease and guide nucleic acid combinations. An expression cassette encoding the MAD nuclease were added to host cells along with various editing cassettes as described above. In this example, the guide nucleic acids were engineered to target the galK gene in the host cell, and the editing sequence was designed to mutate the targeted galK gene in order to turn the gene off, thereby allowing for screening of successfully edited cells. This design was used for identification of functional or compatible MAD nuclease and guide nucleic acid combinations. Editing efficiency was determined by qPCR to measure the editing plasmid in the recovered cells in a high-throughput manner. Validation of the specificity of MAD11 and Cas9 primers is shown in Figures 14A and 14B. These results show that the selected primer pairs are orthogonal and allow quantitative measurement of input plasmid DNA.

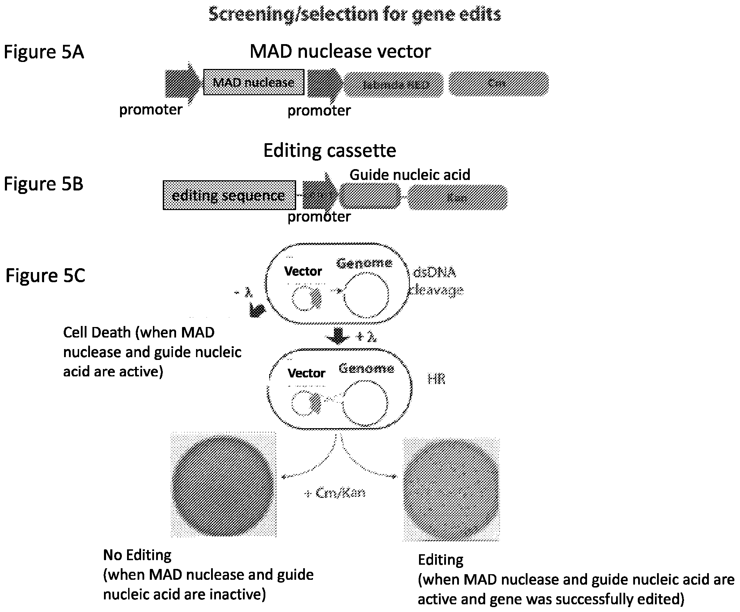

Figures 5A-5B is a depiction of an experiment with a similar experimental design. In this case, the editing cassette (Figure 5B) further comprises a selectable marker, in this case kanamycin resistance (kan) and the MAD nuclease expression vector (Figure 5A) further comprises a selectable marker, in this case chloramphenicol resistance (Cm), and the lambda RED recombination system to aid homologous recombination (HR) of the editing sequence into the target sequence. A compatible MAD nuclease and guide nucleic acid combination will cause a double strand break in the target sequence if a PAM sequence is present. Since the editing sequence (e.g. Figure 3) contains a PAM mutation that is not recognized by the MAD nuclease, edited cells that contain the PAM mutation survive cleavage by the MAD nuclease, while wild-type non-edited cells die (Figure 5C). The editing sequence further comprises a mutation in the galK gene that allows for screening of edited cells, while the MAD nuclease expression vector and editing cassette contain drug selection markers, allowing for selection of edited cells.

Using these methods, compatible guide nucleic acids for MAD1-MAD20 were tested. Twenty scaffold sequences were tested. The guide nucleic acids used in the experiments contained one of the twenty scaffold sequences, referred to as scaffold-1, scaffold-2, etc., and a guide sequence that targets the galK gene. Sequences for Scaffold-1through Scaffold-20 are listed as SEQ ID NO: 84-103, respectively. It should be understood that the guide sequence of the guide nucleic acid is variable and can be engineered or designed to target any desired target sequence, as will be apparent to one skilled in the art upon reading this disclosure. Since MAD2 does not have an endogenous scaffold sequence to test, a scaffold sequence from a nuclease with close homology (scaffold-2, SEQ ID NO: 85) was tested and found to be non-functional, meaning MAD2 and scaffold-2 were not compatible. Therefore, MAD2 was tested with nineteen other scaffold sequences, despite the low sequence homology between MAD2 and the other MAD nucleases.

This workflow was also used to identify or test PAM sequences compatible with a given MAD nuclease. Another method for identifying a PAM site is described in the next example.

In general, for the assays described, transformations were carried out as follows. E. *coli* strains expressing the codon optimized MAD nucleases were grown overnight. Saturated cultures were diluted 1/100 and grown to an OD600 of 0.6 and induced by adding arabinose at a filing concentration of 0.4% and (if a temperature sensitive plasmid is used) shifting the culture to 42 degrees Celsius in a shaking water bath. Following induction, cells were chilled on ice for 15 min prior to washing thrice with ¼ the initial culture volume with 10% glycerol (for example, 50 mL washed for a 200 mL culture). Cells were resuspended in 1/100 the initial volume (for example, 2 mL for a 200 mL culture) and stores at -90 degrees Celsius until ready to use. To perform the compatibility and editing efficiency screens described here, 50 ng of editing cassette was transformed into cell aliquots by electroporation. Following electroporation, the cells were recovered in LB for 3 hours and 100 μL of cells were plated on Macconkey plates containing 1% galactose.

Editing efficiencies were determined by dividing the number of white colonies (edited cells) by the total number of white and red colonies (edited and non-edited cells).

Example 4. PAM selection assay

In order to generate a double strand break in a target sequence, a guide nucleic acid must hybridize to a target sequence, and the MAD nuclease must recognize a PAM sequence adjacent to the target sequence. If the guide nucleic acid hybridizes to the target sequence, but the MAD nuclease does not recognize a PAM site, then cleavage does not occur.

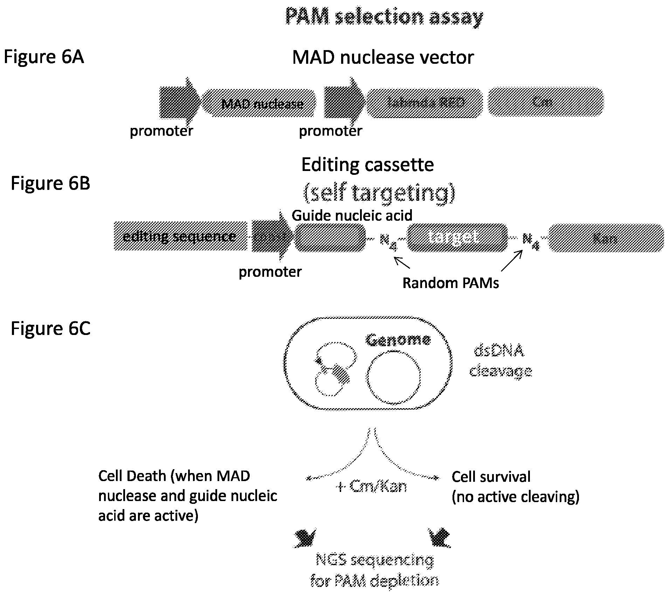

A PAM is MAD nuclease-specific and not all MAD nucleases necessarily recognize the same PAM. In order to assess the PAM site requirements for the MAD nucleases, an assay as depicted in Figures 6A-6C was performed.

Figure 6A depicts a MAD nuclease expression vector as described elsewhere, which also contains a chloramphenicol resistance gene and the lambda RED recombination system.

Figure 6B depicts a self-targeting editing cassette. The guided nucleic acid is designed to target the target sequence which is contained on the same nucleic acid molecule. The target sequence is flanked by random nucleotides, depicted by N4, meaning four random nucleotides on either end of the target sequence. It should be understood that any number of random nucleotides could also be used (for example, 3, 5, 6, 7, 8, etc). The random nucleotides serve as a library of potential PAMs.

Figure 6C depicts the experimental design. Basically, the MAD nuclease expression vector and editing cassette comprising the random PAM sites were transformed into a host cell. If a functional targetable nuclease complex was formed and the MAD nuclease recognized a PAM site, then the editing cassette vector was cleaved and which leads to cell death. If a functional targetable complex was not formed or if the MAD nuclease did not recognize the PAM, then the target sequence was not cleaved and the cell survived. Detection mechanisms (*e.g.* next generation sequence (NGS)) were used to sequence the starting and final cell populations to determine what PAM sites were recognized by a given MAD nuclease. These recognized PAM sites were then used to determine a consensus or non-consensus PAM for a given MAD nuclease.

The consensus PAM for MAD1-MAD8, and MAD10-MAD12 was determined to be TTTN. The consensus PAM for MAD9 was determined to be NNG. The consensus PAM for MAD13-MAD15 was determined to be TTN. The consensus PAM for MAD16-MAD18 was determined to be TA. The consensus PAM for MAD19-MAD20 was determined to be TTCN.

Example 5: Testing heterologous guide nucleic acids

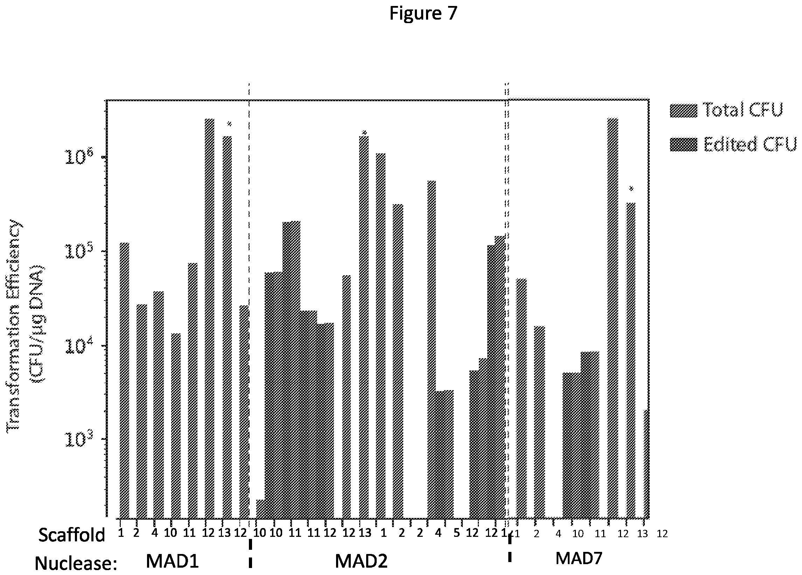

Editing efficiencies were tested for MAD1, MAD2, and MAD7 and are depicted in Figure 7. Experiment details and editing efficiencies are summarized in Table 3. Editing efficiency was determined by dividing the number of edited cells by the total number of recovered cells. Various editing cassettes targeting the galK gene were used to allow screening of editing cells. The guide nucleic acids encoded on the editing cassette contained a guide sequence targeting the galK gene and one of various scaffold sequences in order to test the compatibility of the indicated MAD nuclease with the indicated scaffold sequence, as summarized in Table 3.

Editing efficiencies for compatible MAD nuclease and guide nucleic acids (comprising the indicated scaffold sequences) were observed to have between 75-100% editing efficiency. MAD2 had between a 75-100% editing efficiency and MAD7 had between a 97-100% editing efficiency.

MAD2 combined with scaffold-1, scaffold-2, scaffold-4, or scaffold-13 in these experiments results in 0% editing efficiency. These data imply that MAD2 did not form a functional complex with these tested guide nucleic acids and that MAD2 is not compatible with these scaffold sequences. MAD7 combined with scaffold-1, scaffold-2, scaffold-4, or scaffold-13 in these experiments results in 0% editing efficiency. These data imply that MAD7 did not form a functional complex with these tested guide nucleic acids and that MAD7 is not compatible with these scaffold sequences. Thus, the guide nucleic acids useful in the systems and methods of the disclosure can be identified using empirical data, and will require reasonable experimentation by one of skill in the art when using the methods taught in the present disclosure

For MAD1, all tested guide nucleic acid combinations resulted in 0% editing efficiency, implying that MAD1 did not form a functional complex with any of the tested guide nucleic acids. These data also imply that MAD1 is not compatible with the tested scaffold sequences.

Combined, these data highlight the unpredictability of finding a compatible MAD nuclease and scaffold sequence pair in order to form a functional targetable nuclease complex. Some tested MAD nucleases did not function with any tested scaffold sequence. Some tested MAD nucleases only functioned with some tested scaffold sequences and not with others.

Table 3

| # | Nucleic acid-guided nuclease | Guide nucleic acid scaffold sequence | Editing sequence mutation | Target gene | Editing efficiency |
|---|---|---|---|---|---|
| 1 | MAD1 | Scaffold-1; SEQ ID NO: 84 | L80** | galK | 0% |
| 2 | MAD1 | Scaffold-2; SEQ ID NO: 85 | Y145** | galK | 0% |
| 3 | MAD1 | Scaffold-4; SEQ ID NO: 87 | Y145** | galK | 0% |
| 4 | MAD1 | Scaffold-10; SEQ ID NO: 93 | Y145** | galK | 0% |
| 5 | MAD1 | Scaffold-11; SEQ ID NO: 94 | L80** | galK | 0% |
| 6 | MAD1 | Scaffold-12; SEQ ID NO: 95 | L10KpnI | galK | 0% |
| 7 | MAD1 | Scaffold-13; SEQ ID NO: 96 | Y145** | galK | 0% |

(continued)

| # | Nucleic acid-guided nuclease | Guide nucleic acid scaffold sequence | Editing sequence mutation | Target gene | Editing efficiency |
|---|---|---|---|---|---|
| 8 | MAD1 | Scaffold-12; SEQ ID NO: 95 | L10KpnI | galK | 0% |
| 9 | MAD2 | Scaffold-10; SEQ ID NO: 93 | L80** | galK | 0% |
| 10 | MAD2 | Scaffold-10; SEQ ID NO: 93 | Y145** | galK | 100% |
| 11 | MAD2 | Scaffold-11; SEQ ID NO: 94 | L80** | galK | 98% |
| 12 | MAD2 | Scaffold-11; SEQ ID NO: 94 | Y145** | galK | 99% |
| 13 | MAD2 | Scaffold-12; SEQ ID NO: 95 | Y145** | galK | 98% |
| 14 | MAD2 | Scaffold-12; SEQ ID NO: 95 | Y145** | galK | 0% |
| 15 | MAD2 | Scaffold-13; SEQ ID NO: 96 | Y145** | galK | 0% |
| 16 | MAD2 | Scaffold-1; SEQ ID NO: 84 | L80** | galK | 0% |
| 17 | MAD2 | Scaffold-2; SEQ ID NO: 85 | Y145** | galK | 0% |
| 18 | MAD2 | Scaffold-2; SEQ ID NO: 85 | Y145** | galK | 0% |
| 19 | MAD2 | Scaffold-4; SEQ ID NO: 87 | Y145** | galK | 0% |
| 20 | MAD2 | Scaffold-5; SEQ ID NO: 88 | L80** | galK | 99% |
| 21 | MAD2 | Scaffold-12; SEQ ID NO: 95 | 89** | galK | 0% |
| 22 | MAD2 | Scaffold-12; SEQ ID NO: 95 | 70** | galK | 75% |
| 23 | MAD2 | Scaffold-12; SEQ ID NO: 95 | L10KpnI | galK | 79% |
| 32 | MAD7 | Scaffold-1; SEQ ID NO: 84 | L80** | galK | 0% |
| 33 | MAD7 | Scaffold-2; SEQ ID NO: 85 | Y145** | galK | 0% |
| 34 | MAD7 | Scaffold-4; SEQ ID NO: 87 | Y145** | galK | 0% |
| 35 | MAD7 | Scaffold-10; SEQ ID NO: 93 | Y145** | galK | 100% |
| 36 | MAD7 | Scaffold-11; SEQ ID NO: 94 | L80** | galK | 97% |
| 37 | MAD7 | Scaffold-12; SEQ ID NO: 95 | L10KpnI | galK | 0% |
| 38 | MAD7 | Scaffold-13; SEQ ID NO: 96 | Y145** | galK | 0% |
| 39 | MAD7 | Scaffold-12; SEQ ID NO: 95 | L10KpnI | galK | 0% |

Example 6. Assessment of MAD2 and MAD7

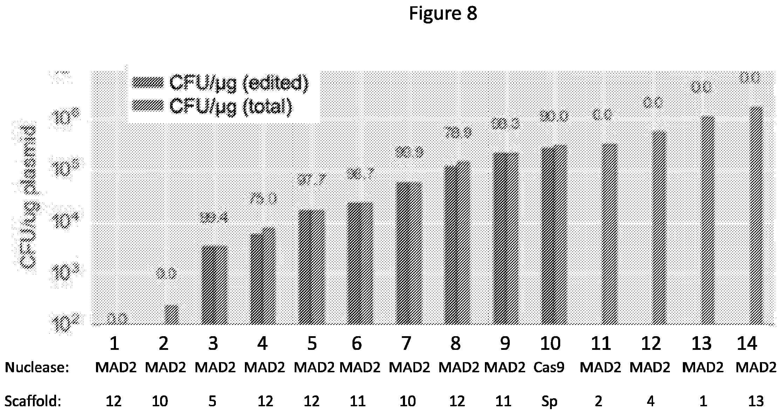

The ability of MAD2 and MAD7 to function with heterologous guide nucleic acids were tested using a similar experimental design as described above. The compatibility of MAD2 with other scaffold sequences was tested and the results of an experiment are depicted in Figure 8. The MAD nucleases, guide nucleic acid scaffold sequences, and editing sequences used in this experiment are summarized in Table 4.

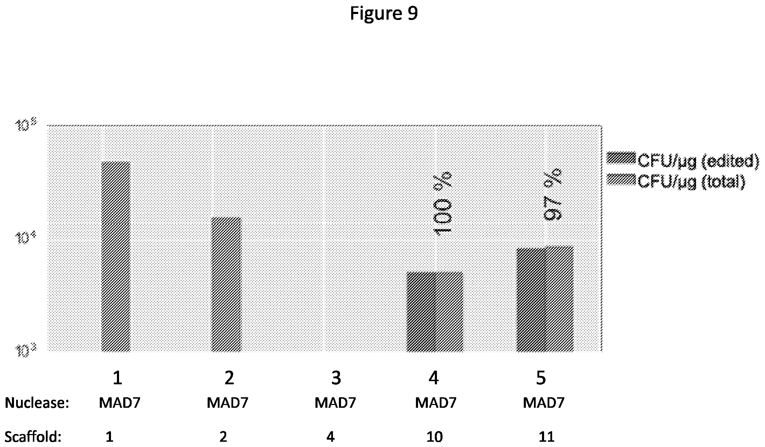

The compatibility of MAD7 with other scaffold sequences was tested and the results of an experiment are depicted in Figure 9. The MAD nucleases, guide nucleic acid scaffold sequences, and editing sequences used in this experiment are summarized in Table 5.

Table 4

| # | Nucleic acid-guided nuclease | Guide nucleic acid scaffold sequence | Editing sequence mutation | Target gene |
|---|---|---|---|---|
| 1 | MAD2 | Scaffold-12; SEQ ID NO: 95 | N89KpnI | galK |

(continued)

| # | Nucleic acid-guided nuclease | Guide nucleic acid scaffold sequence | Editing sequence mutation | Target gene |
|---|---|---|---|---|
| 2 | MAD2 | Scaffold-10; SEQ ID NO: 93 | L80** | galK |
| 3 | MAD2 | Scaffold-5; SEQ ID NO: 88 | L80** | galK |
| 4 | MAD2 | Scaffold-12; SEQ ID NO: 95 | D70KpnI | galK |
| 5 | MAD2 | Scaffold-12; SEQ ID NO: 95 | Y145** | galK |
| 6 | MAD2 | Scaffold-11; SEQ ID NO: 94 | Y145** | galK |
| 7 | MAD2 | Scaffold-10; SEQ ID NO: 93 | Y145** | galK |
| 8 | MAD2 | Scaffold-12; SEQ ID NO: 95 | L10KpnI | galK |
| 9 | MAD2 | Scaffold-11; SEQ ID NO: 94 | L80** | galK |
| 10 | SpCas9 | S. pyogenese gRNA | Y145** | galK |
| 11 | MAD2 | Scaffold-2; SEQ ID NO: 85 | Y145** | galK |
| 12 | MAD2 | Scaffold-4; SEQ ID NO: 87 | Y145** | galK |
| 13 | MAD2 | Scaffold-1; SEQ ID NO: 84 | L80** | galK |
| 14 | MAD2 | Scaffold-13; SEQ ID NO: 96 | Y145** | galK |

Table 5

| # | Nucleic acid-guided nuclease | Guide nucleic acid scaffold sequence | Editing sequence mutation | Target gene |
|---|---|---|---|---|
| 1 | MAD7 | Scaffold-1; SEQ ID NO: 84 | L80** | galK |
| 2 | MAD7 | Scaffold-2; SEQ ID NO: 85 | Y145** | galK |
| 3 | MAD7 | Scaffold-4; SEQ ID NO: 87 | Y145** | galK |
| 4 | MAD7 | Scaffold-10; SEQ ID NO: 93 | Y145** | galK |
| 5 | MAD7 | Scaffold-11; SEQ ID NO: 95 | L80** | galK |

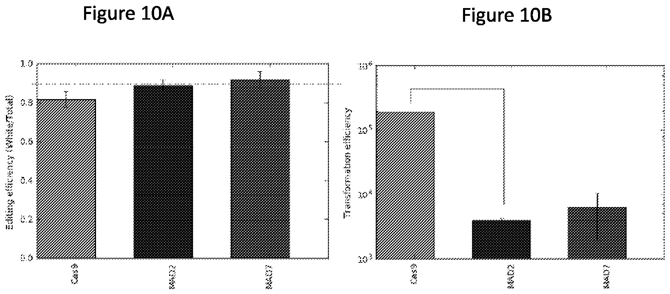

In another experiment, editing efficiencies (Figure 10A) were determined by calculating the ratio of editing colonies (white colonies, edited galK gene) versus total colonies. Transformation efficiencies (Figure 10B) were determined by calculating the total number of recovered cells compared to the start number of cells.

In this example (Figure 10A-10B), cells expressing galK were transformed with expression constructs expressing either MAD2 or MAD7 and a corresponding editing cassette comprising a guide nucleic acid targeting the galK gene. The guide nucleic acid was comprised of a guide sequence targeting the galK gene and the scaffold-12 sequence (SEQ ID NO: 95).

In the depicted example, MAD2 and MAD7 has a lower transformation efficiency compared to S. pyogenes Cas9, though the editing efficiency of MAD2 and MAD7 was slightly higher than S. pyogenes Cas9.

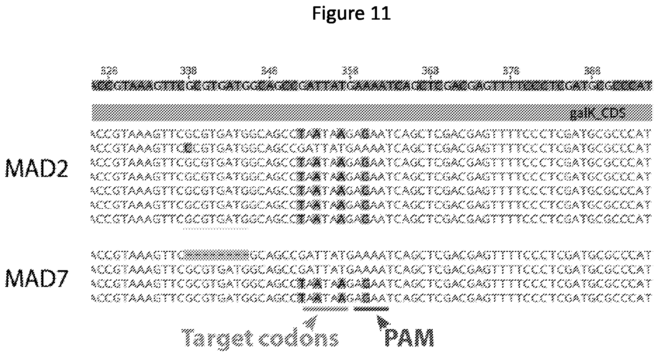

Colonies from the editing experiments were recovered, and a representative number were subjected to NGS to determine the presence of the edits. Figure 11 depicts the sequencing results from these select colonies recovered from the assay described above. The target sequence was in the galK coding sequence (CDS). The TTTN PAM is shown as the reverse complement (wild-type NAAA, mutated NGAA). The mutations targeted by the editing sequence are labeled as target codons. Changes compared to the wild-type sequence are highlighted. In these experiments, the scaffold-12 sequence (SEQ ID NO: 95) was used. The guide sequence of the guide nucleic acid targeted the galK gene.

Six of the seven depicted sequences from the MAD2 experiment contained the designed PAM mutation and designed mutations in the target codons of galK, which one sequences colony maintained the wild-type PAM and wild-type target codons while also containing an unintended mutation upstream of the target site.

Two of the four depicted sequences from the MAD7 experiment contained the designed PAM mutation and mutated target codons. One colony comprises a wildtype sequence, while another contained a deletion of eight nucleotides upstream of the target sequence.

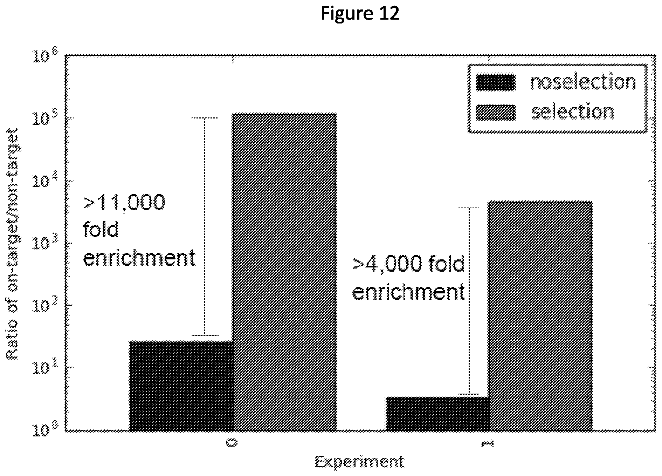

Figure 12 depicts results from two experiments testing the ability of selection to aid in the recovery of edited cells. In this experiment, the MAD2 nuclease was used with a guide nucleic acid comprising scaffold-11 sequence and a guide sequence targeting galK. The editing cassette comprised an editing sequence designed to incorporate an L80** mutation into galK, thereby allowing screening of the edited cells. In experiment 1, the MAD2 nuclease was used with a guide nucleic acid comprising scaffold-12 sequence and a guide sequence targeting galK. The editing cassette comprised an editing sequence designed to incorporate an L10KpnI mutation into galK. In both experiments, a negative control plasmid using a guide nucleic acid that is not compatible with MAD2 was included in the transformations. Following transformation, the ratio of the compatible editing cassette (those containing scaffold-11 or scaffold-12 guide nucleic acids) to the non-compatible editing cassette (negative control) was measure. The experiments were done in the presence or absence of selection. The results show that more compatible editing cassette containing cells were recovered compared to the non-compatible editing cassette, and this result is magnified when selection is used.

Example 7. Guide nucleic acid characterization

The sequences of scaffolds 1-8, and 10-12 (SEQ ID NO: 84-91, and 93-95) were aligned and are depicted in Figure 13. Nucleotides that match the consensus sequence are faded, while those diverging from the consensus sequence are visible. The predicted pseudoknot region is indicated. Without being bound by theory, the region 5' of the pseudoknot may be influence binding and/or kinetics of the nucleic acid-guided nuclease. As is shown in Figure 13, in general, there appears to be less variability between scaffold sequences in the pseudoknot region (e.g., SEQ ID NO: 172-181) as compared to the sequence outside of the pseudoknot region.

Example 8. Editing efficiency of the MAD Nucleases

A plate-based editing efficiency assay and a molecular editing efficiency assay were used to test editing efficiency of various MAD nuclease and guide nucleic acid combinations.

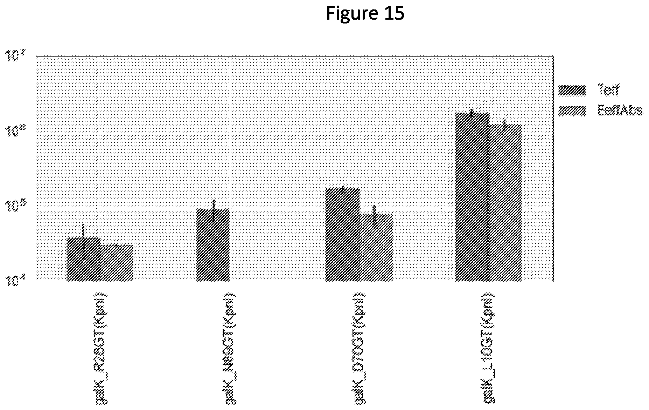

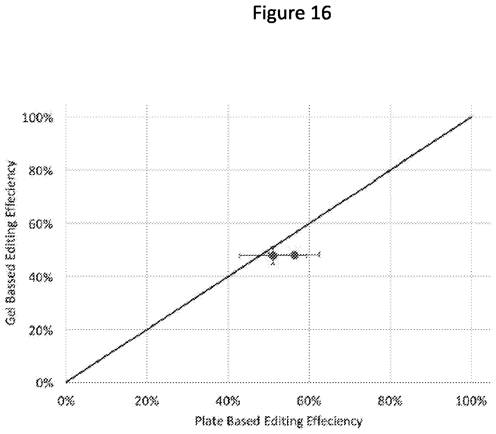

Figure 15 depicts quantification of the data obtained using the molecular editing efficiency assay using MAD2 nuclease with a guide nucleic acid comprising scaffold-12 and a guide sequencing targeting galK. The indicated mutations were incorporated into the galK using corresponding editing cassettes containing the mutation. Figure 16 shows the comparison of the editing efficiencies determined by the plate-based assay using white and red colonies as described previously, and the molecular editing efficiency assay. As shown in Figure 16, the editing efficiencies as determined by the two separate assays are consistent.

Example 9. Trackable editing

Genetic edits can be tracked by the use of a barcode. A barcode can be incorporated into or near the edit site as described in the present specification. When multiple rounds of engineering are being performed, with a different edit being made in each round, it may be beneficial to insert a barcode in a common region during each round of engineering, this way one could sequence a single site and get the sequences of all of the barcodes from each round without the need to sequence each edited site individually. Figures 17A and 17C, 18, and 19 depict examples of such trackable engineering workflows.
As depicted in Figure 17A, a cell expressing a MAD nuclease is transformed with a plasmid containing an editing cassette and a recording cassette. The editing cassette contains a PAM mutation and a gene edit. The recorder cassette comprises a barcode, in this case a 15nt barcode unique for the sequences tested. Both the editing cassette and recording cassette each comprise a guide nucleic acid to a distinct target sequence. Within a library of such plasmids, the recorder cassette for each round can contain the same guide nucleic acid, such that the first round barcode is inserted into the same location across all variants, regardless of what editing cassette and corresponding gene edit is used. The correlation between the barcode and editing cassette is determined beforehand though such that the edit can be identified by sequencing the barcode. Figure 17B shows an example of a recording cassette designed to delete a PAM site while incorporating a 15nt barcode. The deleted PAM is used to enrich for edited cells since mutated PAM cells escape cell death while cells containing a wild-type PAM sequence are killed.
A similar approach is depicted in Figure 18. In this case, the recorder cassette from each round is designed to target a sequence adjacent to the previous round, and each time, a new PAM site is deleted by the recorder cassette. The result is a barcode array with the barcodes from each round that can be sequenced to confirm each round of engineering took place and to determine which combination of mutations are contained in the cell, and in which order the mutations were made. Each successive recorder cassette can be designed to be homologous on one end to the region comprising the mutated PAM from the previous round, which could increase the efficiency of getting fully edited cells at the end of the experiment. In other examples, the recorder cassette is designed to target a unique landing site that was incorporated by the previous recorder cassette. This increases the efficiency of recovering cells containing all of the desired mutations since the subsequent recorder cassette and barcode can only target a cell that has successfully completed the previous round of engineering.

Figure 19 depicts another approach that allows the recycling of selectable markers or to otherwise cure the cell of the plasmid form the previous round of engineering. In this case, the transformed plasmid containing a guide nucleic acid designed to target a selectable marker or other unique sequence in the plasmid form the previous round of engineering.

Example 10. Plasmid based PAM (pPAM) library design

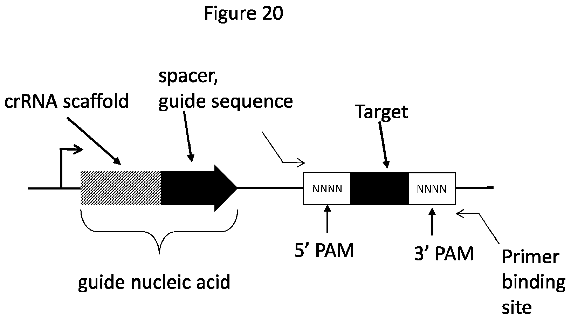

The pPAM target libraries were designed by taking an individual spacer sequence flanked by degenerate nucleotides on the 5' termini and 3' termini of the target sequence. Arrangement formats of N4-SPACERN=20-N4 and N5-SPACERN=20-N3 were used. These sequences were ordered as a single oligonucleotide with degeneracy at the designated positions (e.g., N3, N4, and N5). The oligonucleotides were amplified and cloned into a gRNA vector containing a spacer matched to the target library to create a self-targeting gRNA vector that will deplete under competitive growth situations (Figure 20). In one experiment, a total of eight different spacer sequences were cloned into vectors that contained a gRNA designed to match the respective target.

Example 11. Methods

Cloning of target libraries

All target libraries used to analyze specificity of the PAM and spacer motifs were cloned into the desired cloning site by amplification of a single stranded overlapping oligo pool. Linearized backbones for cloning were generated by PCR amplification with overlaps compatible to the insert amplicons and dpnl digested to eliminate parent vector contamination. The linearized backbone and insert pools were cloned via a Gibson assembly (using either Gibson Assembly Master Mix or NEBuilder HiFi DNA assembly kit according to manufacturer's protocol). Half of each Gibson assembly was desalted for 30 min using a 0.025 $\mu$m dialysis membrane floated on deionized water in a petri dish. This was used to transform E. cloni 10G supreme competent cells and recovered for 1 hr in LB. 1% of the recovered transformation (10 $\mu$L) was used for dilution-based plating to estimate CFU per cloning reaction and library coverage. Cloning was repeated, if CFU counts were <10X the library size to ensure complete coverage of the desired sequence space. The remaining recovery volume was transferred to 25 mL overnight cultures containing 100 $\mu$g/mL carbenecillin to maintain selective pressure for replication of the cloned library.

Following overnight recovery, 2 $\times$ 1 mL aliquots were taken from each library cloning reaction and stored as glycerol stocks. The remaining 23 mL of culture was pelleted and used to extract plasmid DNA using the Qiagen Plasmid Plus Midi kit. This DNA was used in subsequent transformations to generate the data presented.

Competent cell prep for gRNA depletion studies

To prepare competent cells for gRNA depletion, an editing plasmid into *E. coli* MG1655. The editing plasmid contained a temperature-inducible RNA guided nuclease of interest (RGEN), arabinose-inducible $\lambda$ RED operon, and a chloramphenicol resistance marker. Following overnight growth, saturated RGEN containing cell lines were then introduced at 1/100 dilution into 250 mL LB + 25 $\mu$g/mL chloramphenicol in 500 mL baffled shake flasks. Inoculated cultures were grown to 0.5-0.8 OD and transferred to a 42°C shaking water bath to induce RGEN expression. Following the 42°C induction cells, were placed on ice for 10 min. Cells were then washed 3X with 100 mL ddH2O or 10% glycerol. Following the final wash step cells were resuspended in 2.5 mL (or 1/100th total culture volume) of 10% glycerol and aliquoted into 200 $\mu$L portions to store @-80°C.

gRNA depletion method

Each gRNA depletion experiment was done using a single 200 $\mu$L aliquot of competent RGEN containing cells. This aliquot of cells was dispensed into a chilled 2 cm gap cuvette and electroporated using the Nepagene system. The electroporation was done using a 2400 V transfer pulse and 20 150V pulses to mix. Each transformation was performed with 50-500 ng of the desired library as described in the experimental folders. Following 2 hrs of recovery, 1% of the transformation was spot plated to determine transformation efficiencies (i.e. total CFU) and the remaining transformation volume was used to inoculate 100 mL outgrowth cultures. Cultures were sampled at various time points by removing a 1 mL aliquot and performing DNA extraction using QiaPrep Miniprep kit from Qiagen for downstream sequencing.

General NGS prep and analysis

Plasmid mini-preps were amplified with experiment indexed primers. Amplicons were pooled to normalize expected read-counts and gel purified prior to loading on the MiSeq/NextSeq instruments. The indexed fastq read files were then mapped against the expected variants with 100% identity from the experimental designs and counts tallied to perform the comparative analyses seen in the data. All counts were normalized to frequency by the eqn. Vf=(V counts)/(total counts) where Vf is the frequency of the variant in a given index, V counts is the counts seen for that variant and total counts are the total counts observed across the entire experimental index.

Data analysis and depletion calculations.

Depletion scores (or absolute fitness scores) were calculated as the log2 depletion score using the following equation: $W = log2(F_{x,f}/F_{x,i})$; where $F_{x,f}$ is the frequency of cassette X at the final time point and $F_{x,i}$ is the initial frequency of cassette X, and W is the absolute fitness of each variant. Frequencies were determined by dividing the read counts for each variant by the total experimental counts including those that were lost to filtering. Each selection was performed in duplicate and the count weighted average of the two measurements was used to infer the average fitness score of each mutation as follows: $Wavg = (\Sigma^N_{i=1} counts_i * W_i) / (\Sigma^N_{i=1} counts_i)$. Note that the calculated scores are referred to as a depletion score when the calculated value is negative, though it can also be referred to as an enrichment score if the calculated value is positive.

These scores were used to rank and assess the fitness contributions of each mutation under the various selection pressures investigated. For all selections, the average absolute fitness scores for the synonymous mutants were provided as a composite measure of the average growth rate. Absolute enrichment scores were considered significant if the mutant enrichment was at least $\mu \pm 2*\sigma$ (i.e., $P = 0.05$, assuming a normal distribution) of the wild-type value. The mean and significance thresholds were reported for each selection. At least two replicates of each selection were performed and a cutoff threshold of 10 across the replicate experiments applied for inclusion in each analysis.

In some cases, data was normalized against NRRN PAM control library data or other non-target control library data.

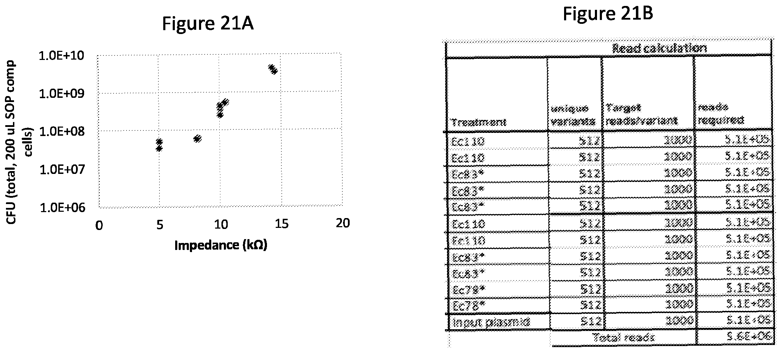

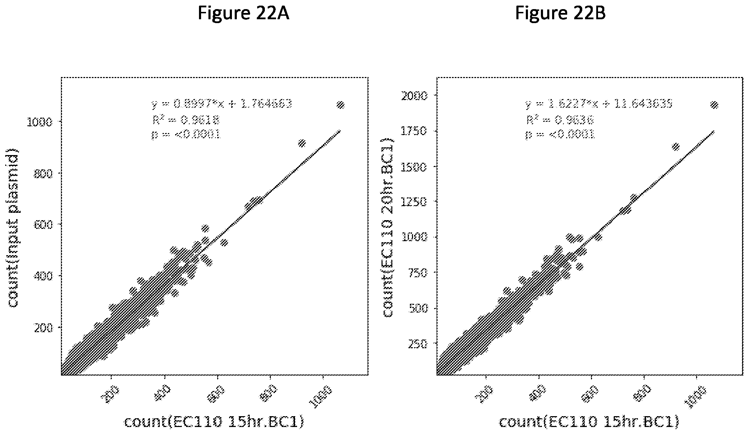

Figure 21A showed that impedance reflected the number of transformants per well with SOP competent cells and fixed amount of DNA. Figure 21B depicts a comparison of count weighted average depletion score with Ec110 (4 replicates), Ec83* (5 replicates), and Ec78* (duplicates). These amplicons were sequences and the 3'-PAM was used as a sample barcode. Figure 22A shows that the counts for the input EC110 control plasmid and EC110 control plasmid after 15 hours of out-growth are nearly identical. Figure 22B shows that the counts for the EC110 control plasmid following 15 hours and 20 hours of liquid out-growth are nearly identical, which demonstrates stable maintenance of the input plasmid.

Example 12. pPAM assay for identification of MAD7 PAM and MAD2 PAM

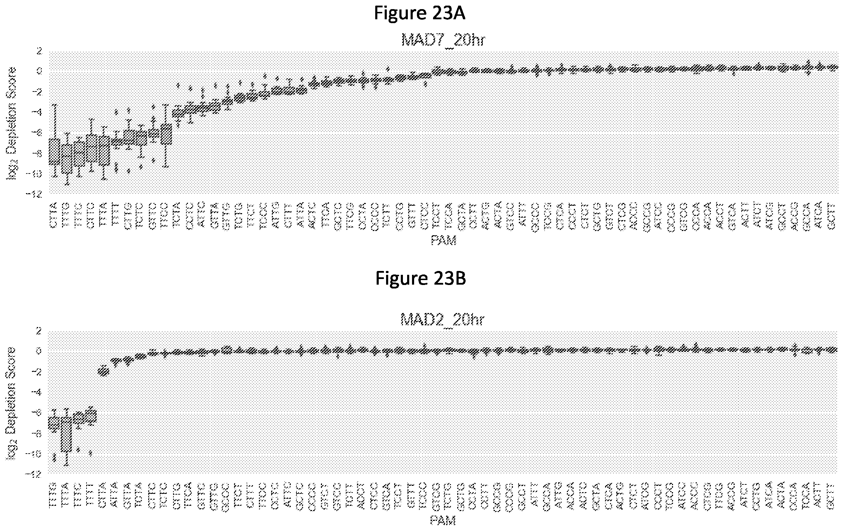

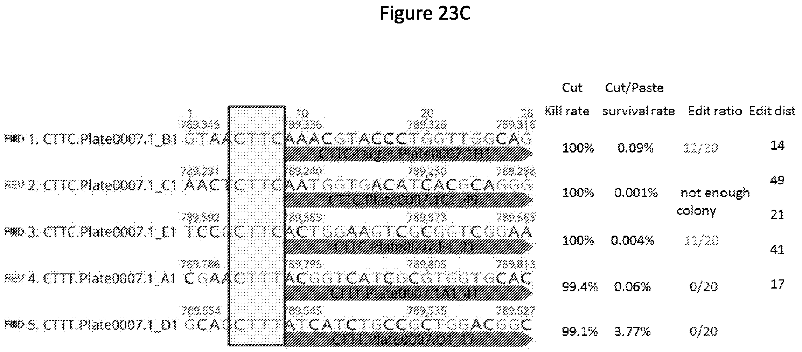

The PAM preference for MAD2 and MAD7 was determined using the pPAM plasmid depletion assay and data analysis methods described above. Depletion scores for the various PAMs were calculated as described above at 20 hours for both MAD7 (Figure 23A) and MAD2 (Figure 23B). PAMs that were depleted or decreased are those that were able to be recognized and thus cleaved by the nuclease and gRNA complex. The preferential PAM sites for both enzymes are shown from left to right, respectively. MAD2 and MAD7 both have a preference for NYYN PAMs, though some NYYN PAMs worked better than others (Figures 23A and 23B). The editing versus cutting efficiencies were further characterized for select MAD7 PAM plasmids (Figures 23C).

Example 13. Synthetic Plasmid based Off-Target (SPOT) assay design

The Synthetic Plasmid based Off-Target (SPOT) target libraries were designed by taking individual spacer sequences 20 nucleotides in length and appending a subset of PAM sequences to either side. In some experiments, an YTTN PAM was appended on the 5' side of each target and an NGG PAM was appended on the 3' side, thereby generating the format YTTN-SPACERN=20-NGG (SEQ ID NO: 377). As based on official IUPAC nomenclature, Y = C or T; and N = A, C, T, or G. Eight combinations of 5' PAM-3' PAM pairs were tested in an initial experiment for each target, one for each 5' PAM with each 3' PAM sampled twice. Specifically, the following 5' PAM - 3' PAM combinations were generated and tested: TTTA-AGG (SEQ ID NO: 378); TTTC-CGG (SEQ ID NO: 379); TTTG-GGG (SEQ ID NO: 380); TTTT-TGG (SEQ ID NO: 381); CTTA-AGG (SEQ ID NO: 382); CTTC-CGG (SEQ ID NO: 383); CTTG-GGG (SEQ ID NO: 384); and CTTT-TGG (SEQ ID NO: 385), where a 20 nucleotide spacer was in between each PAM combination. Although only a representative number of combinations were tested in this experiment, it should be appreciated that every possible 5'-YTTN-N=20-NGG-3' (SEQ ID NO: 377) PAM combination can be tested as well. The set of target spacers with different PAM combinations were then used as a template to design point mutations throughout the spacer sequence. The design of the mutation libraries in the target spacer consisted of four different mutation sets for each PAM-spacer sequence and internal controls.

The first mutation library was a scanning mismatch library consisting of 1, 2, 3, or 4 bp contiguous mismatches, wherein each mismatch was the complement nucleotide of the wildtype sequence. Each possible 1, 2, 3, and 4 contiguous bp mutation along the entire length of the spacer sequence was generated.

The second mutation library was a scanning deletion library consisting of 1, 2, 3, or 4 bp contiguous deletions. Each possible 1, 2, 3, and 4 contiguous bp deletion along the entire length of the spacer sequence was generated.

The third mutation library was a single insertion library wherein a single base insertion was made at each position in the spacer. In some experiments, each 1 bp insertion was made by duplicating the nucleotide directly 5' of the insertion site. Other insertion library designs include duplicating the nucleotide directly 3' of the insertion site, or by generating each possible nucleotide insertion variant, e.g., an individual variant with the insertion one of A, T, C, and G at each position in the spacer region.

The fourth mutation library was a random mutagenesis library wherein 2-5 bp positions were randomly mutated to create a diverse set of mutant sequences that approximate the most biologically relevant kind of diversity. These 2-5 bp mutations were not required to be contiguous and therefore often had intervening non-mutated or wildtype nucleotide in between the mutated nucleotides. Such noncontiguous random mutations have been found to be common in biological systems.

As with the pPAM libraries, the SPOT sequences were cloned into gRNA vectors containing a spacer matched to the target library to create a self-targeting gRNA vector. The oligo pools were amplified and cloned into the gRNA vector containing a spacer matched to the target library to create a self-targeting gRNA vector that would deplete under competitive growth conditions due to cutting and loss of the plasmid under selective pressure. A the eight selected 5'-3' PAM combinations flanking each of the 8 different spacer sequences were cloned into vectors that contained a gRNA designed to match the target.

Using the SPOT assay, *in vivo* depletion assays are possible, independent of ribonucleoprotein complex production. This assay also allows unique off-target analysis with systematic off-target designs through the various mutation library design options. Additional advantages of the SPOT assay include more controlled variation of off-target candidates, the assay can be used to compare different PAM architectures, and the assay can be combined or mixed with the pPAM library assay described above.

Other off-target assays, such as Site-Seq, BLISS, Digenome-Seq, or Circle-Seq, mine the cut-sites from mammalian experiments and report off-target sequences with NHEJ repair efficiencies from deep sequencing. Using these other assays, most studies show off-target effects of greater than 3 bp random mismatches.

Example 14. Off-target characterization

The off-target effects of MAD7 and MAD2 were analyzed using the Synthetic Plasmid based Off-Target (SPOT) assay described above. First, eight targets with various flanking PAM sites were tested for cleavage activity using either a reference nuclease, MAD7, or MAD2. Select targets from above were then used as starting points for generating libraries of various random mutations within the target. In this example assay, a 171 bp amplicon was generated when the target and flanking PAM regions were amplified, allowing for either 87 bp reads with inline index, or 46 bp reads with 12 bp index reads.

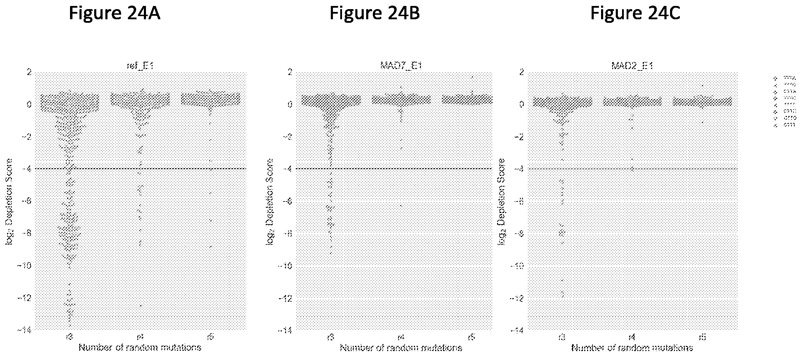

Data from example experiments are shown in Figures 24A-C. The targets contained the indicated number of random mutations (r3 = 3 bp; r4 = 4 bp; r5 = 5 bp). Cas9 was used to generate the depletion plot in Figure 24A, MAD7 was used to generate the depletion plot in Figure 24B and MAD2 was used to generate the depletion plot in Figure 24C.

As seen when comparing the reference protein depletion plots to the MAD7 and MAD2 depletion plots, the reference protein has far more off-target cutting events across each of the r3, r4, and r5 libraries. Both MAD7 and MAD2 showed less off-target cutting events for the random mutations, and there were virtually no off-target cutting events with 5 bp random mismatches. These off-target cutting events, or lack thereof, are independent of PAM or target sequence.

Example 15. Combined PAM determination and off-target analysis

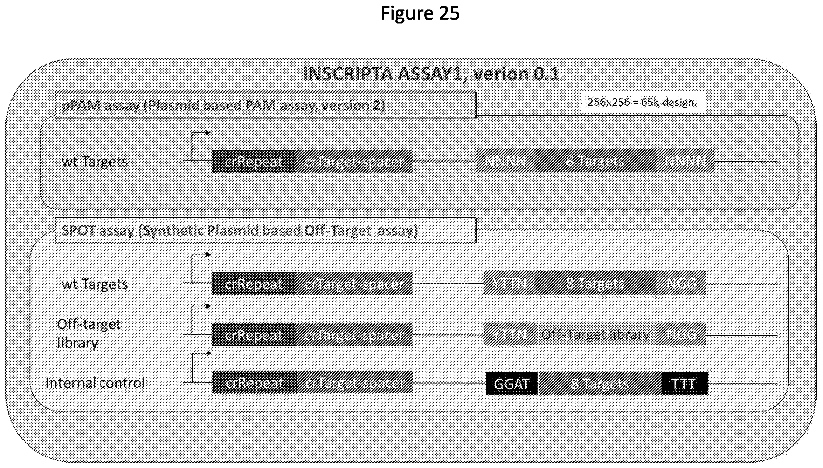

The ability to characterize PAM specificity and off-target effects in a combined experiment was tested by combining the pPAM assay and the SPOT assay that were described above, which combined are referred to herein as the Inscripta assay (e.g., Figure 25). In an example Inscripta assay a 171 bp amplicon is generated when the target and flanking PAM regions are amplified, allowing for 46 bp reads with 12 bp index reads. Targeting sequences were chosen to uniformly sample a wide range of melting temperature values for the spacer sequence.

The Inscripta assay was used to rapidly characterize the PAM specificity and off-target rates of 10 enzymes. MAD7 depletion was essentially identical between 20 hours (MAD7) and 24 hours (MAD7.24) of depletion. The plasmid input was similar to Ec110, which is an E. *coli* strain containing a control plasmid similar to the engine vector backbone described above but lacking a nuclease encoding gene. MAD2 depletion remained the same with constitutive promoter Ec78* (MAD2) versus Ec113, which is an *E. coli* strain containing an engine vector backbone as described above containing a gene encoding the MAD7 nuclease controlled by a constitutive proA promoter instead of the pL inducible promoter system. Strong depletion was observed with using MAD7 or MAD2 and a multi-target library, and strong depletion within the multi-target library was also observed with MAD4. Less depletion observed with MAD5 as compared to MAD2, MAD7, and MAD4.

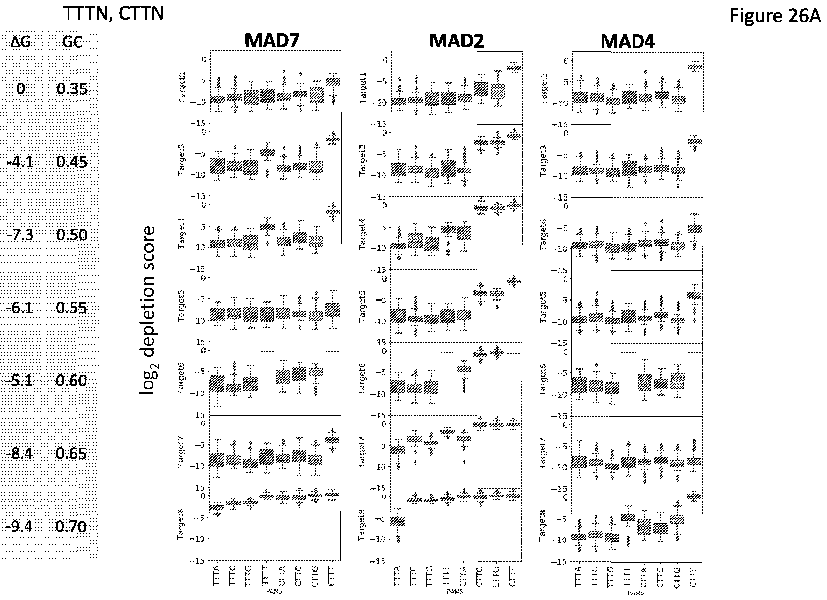

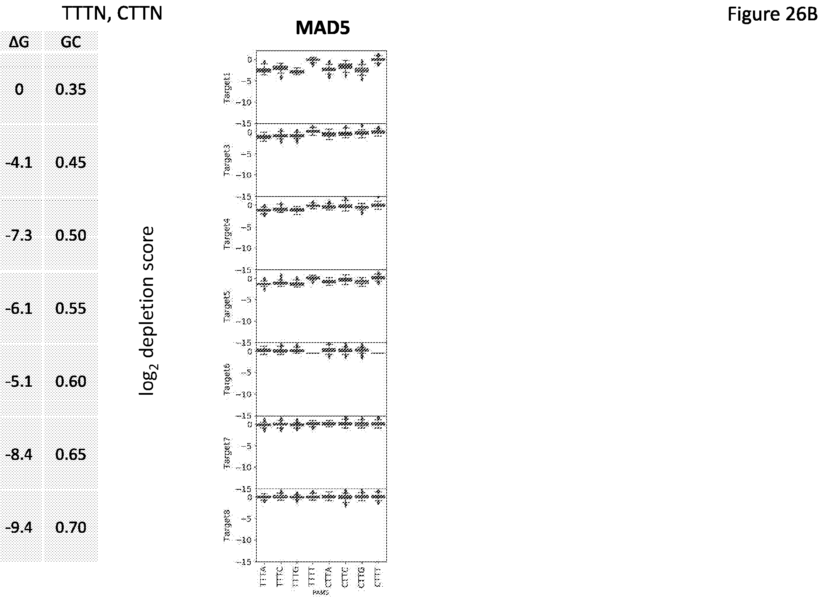

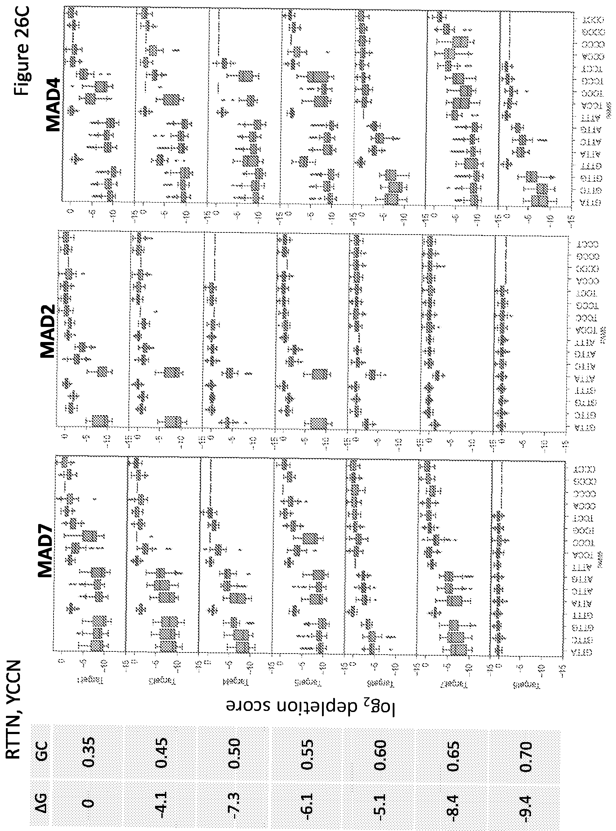

Based on these data, MAD7, MAD2, MAD4, and MAD5 were selected for further characterization. PAM specificities were analyzed for each using the pPAM assay data. Depletion plots for tested PAMs TTN and CTTN are depicted in Figure 26A and Figure 26B, and PAMs RTTN and YCCN are depicted in Figure 26C.

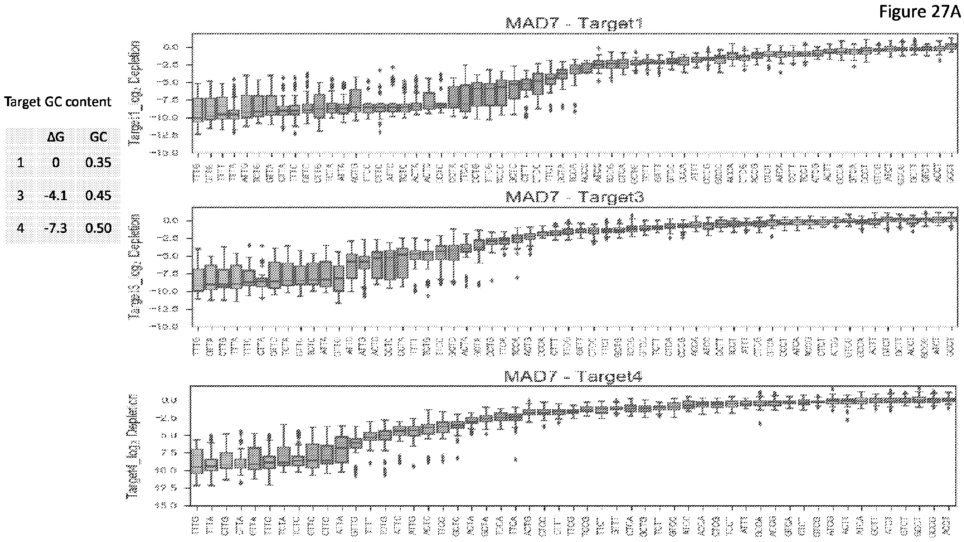

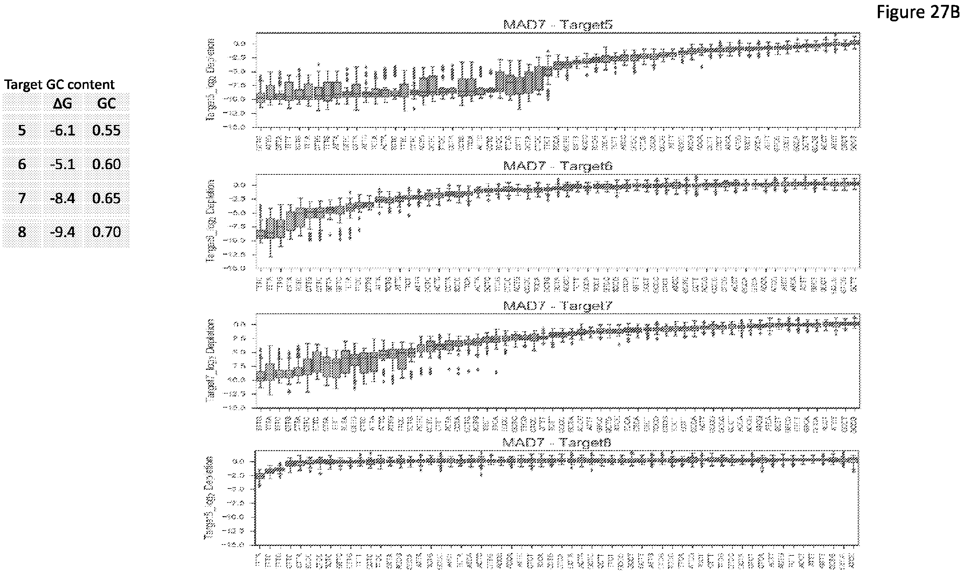

MAD7 depletions scores for various PAMs are also depicted in Figure 27A-27B and these data indicate that the GC content of the target sequence can affect the scale of the depletion activity, suggesting that cleavage and targeting can be tuned based on the PAM and target sequence choice.

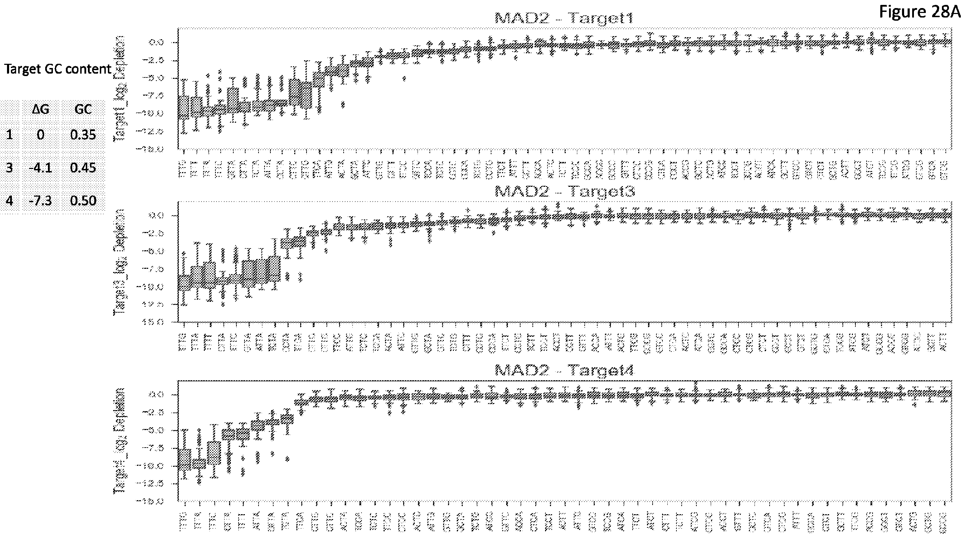

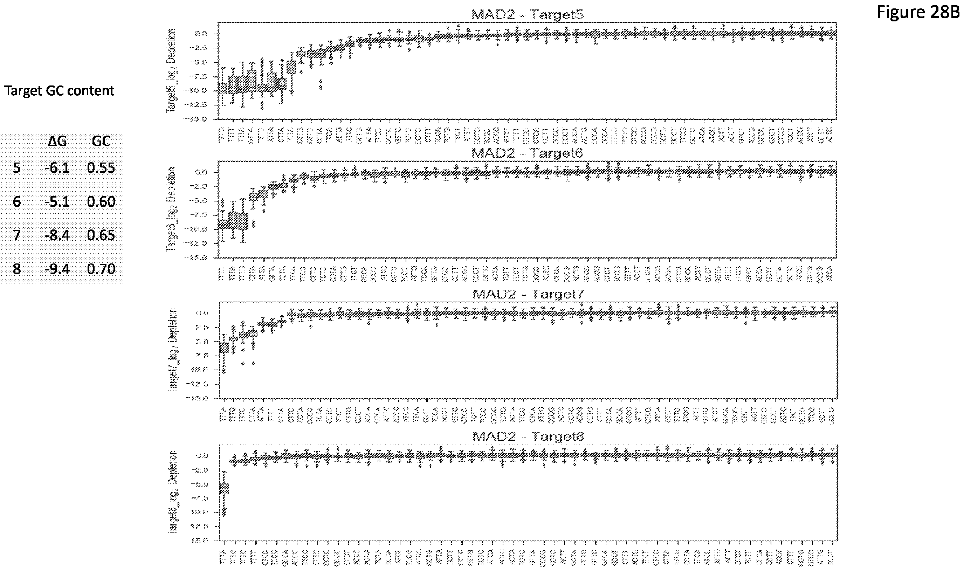

MAD2 depletions scores for various PAMs are also depicted in Figure 28A-29B and these data indicate that the GC content of the target sequence can affect the scale of the depletion activity, suggesting that cleavage and targeting can be tuned based on the PAM and target sequence choice. For example, a PAM of TTTA with a GC content equivalent to Target 8 shows very strong depletion.

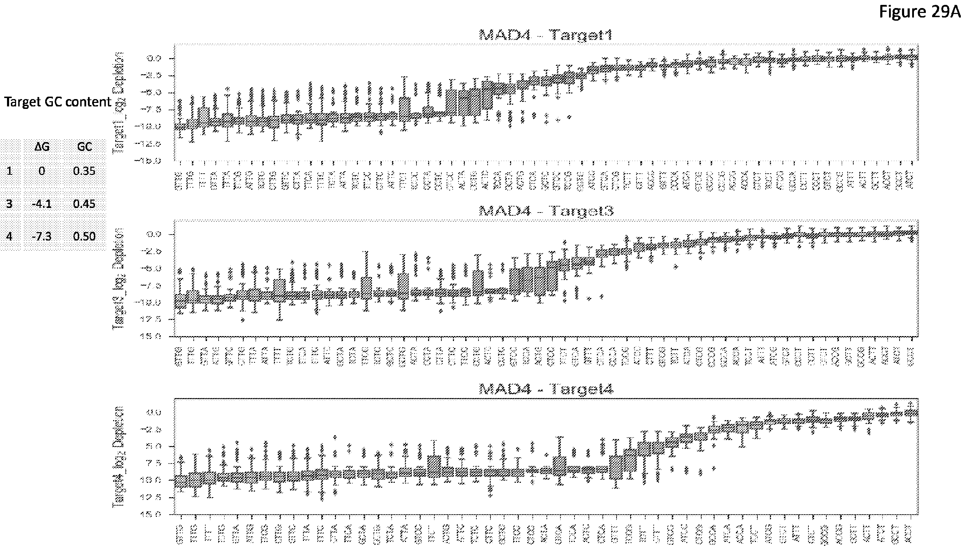

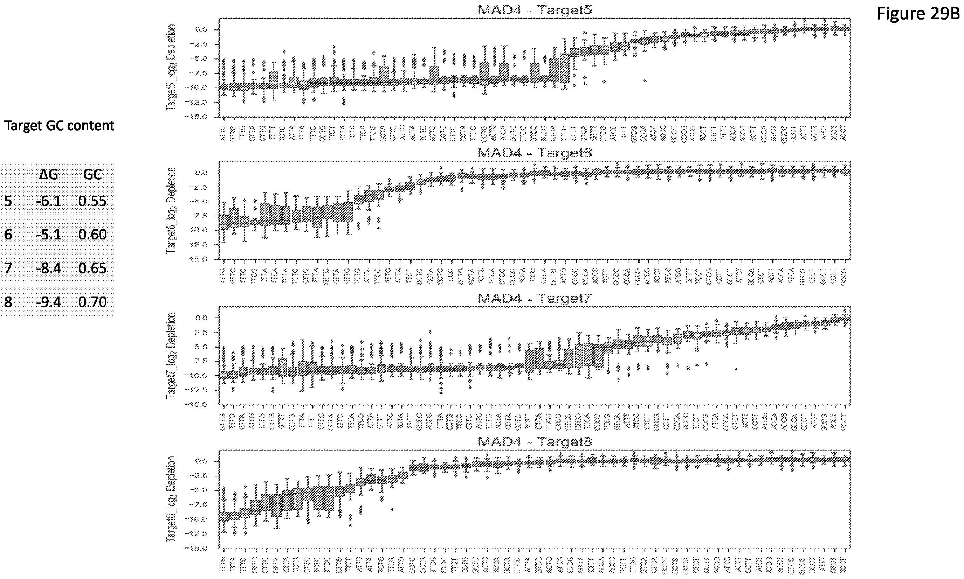

MAD4 depletions scores for various PAMs are also depicted in Figure 29A-29B and these data indicate that the GC content of the target sequence can affect the scale of the depletion activity, suggesting that cleavage and targeting can be tuned based on the PAM and target sequence choice. MAD4 also showed high depletion scores with Target 8, which has the highest GC content out of all of the targets tested.

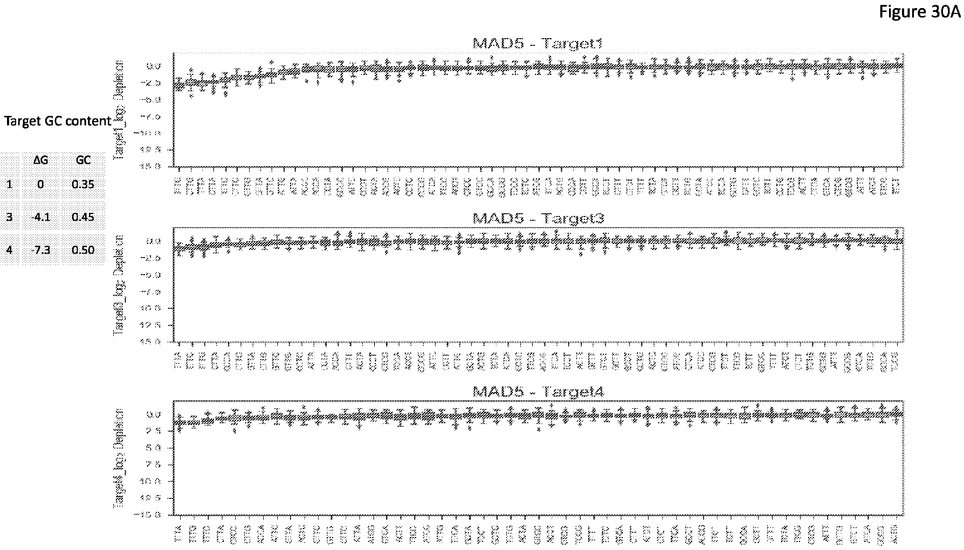

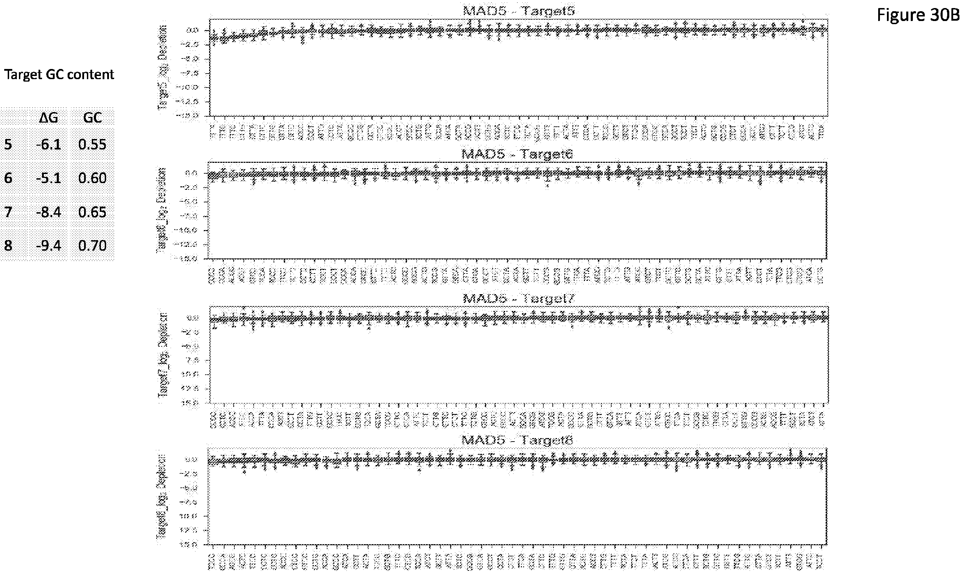

MAD5 depletion scores for various PAMs are also depicted in Figures 30A-30B. These data indicate that MAD5 does not produce strong depletion of the target sequences and PAM sequence combinations tested.

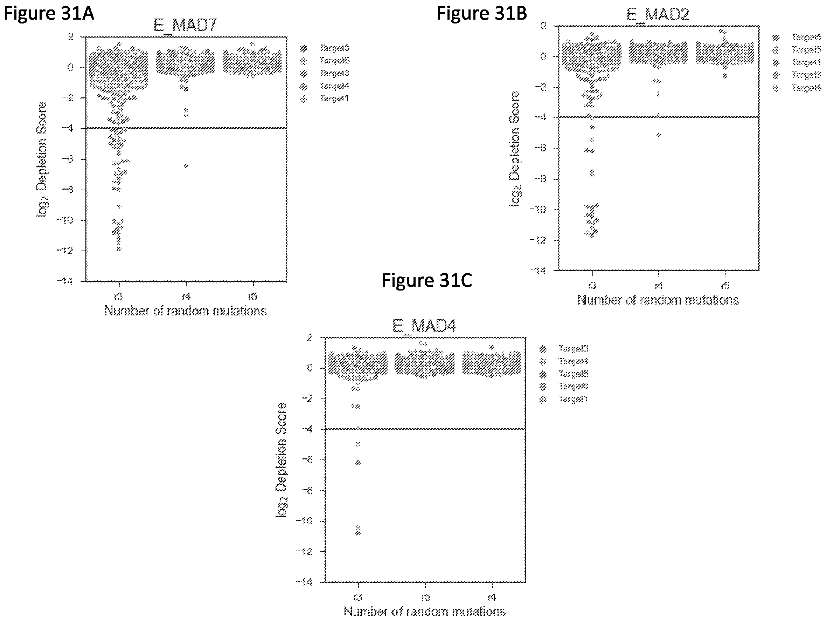

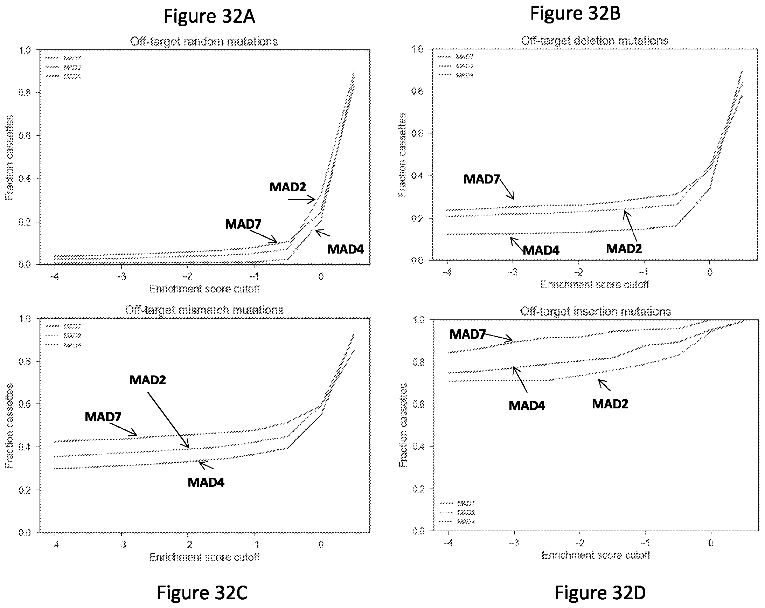

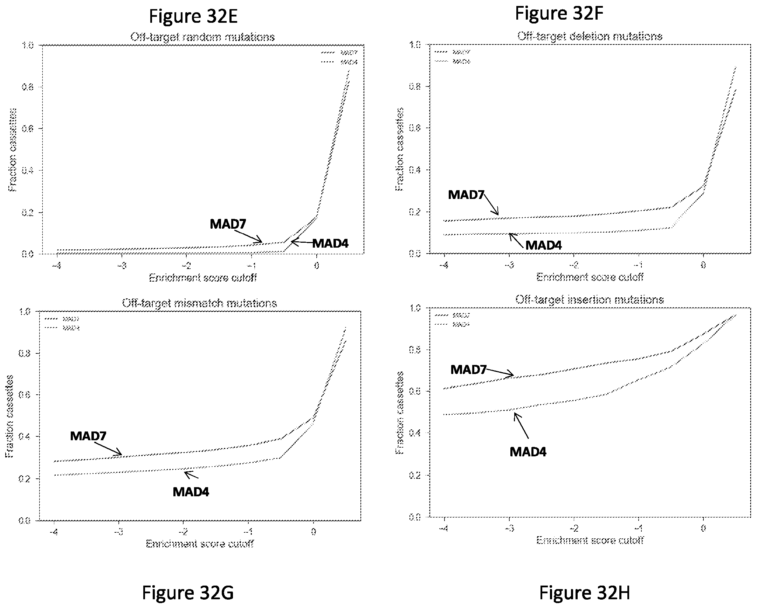

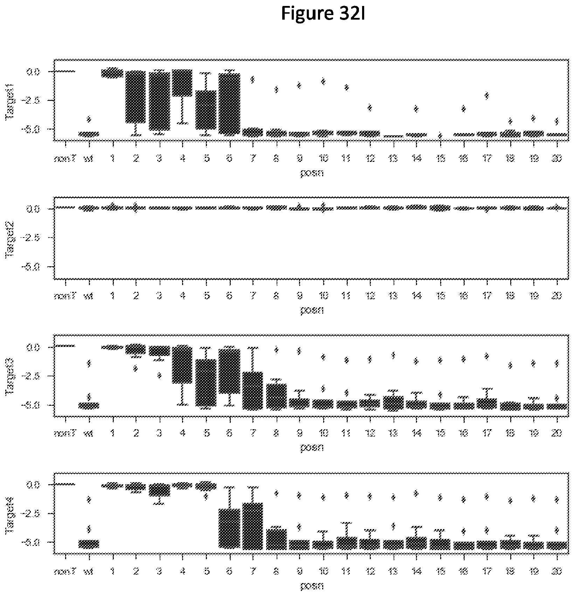

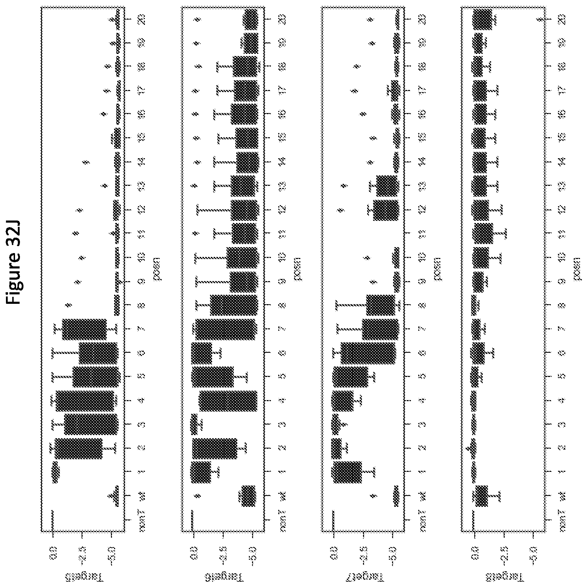

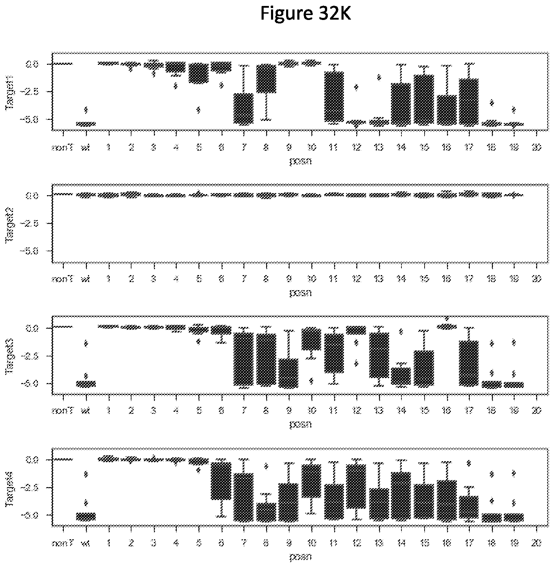

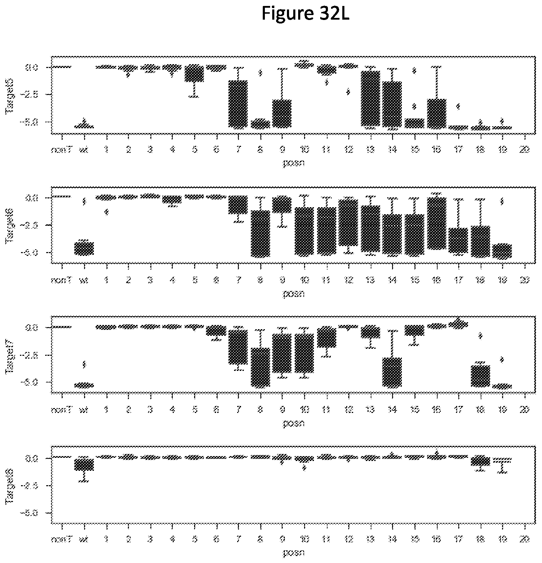

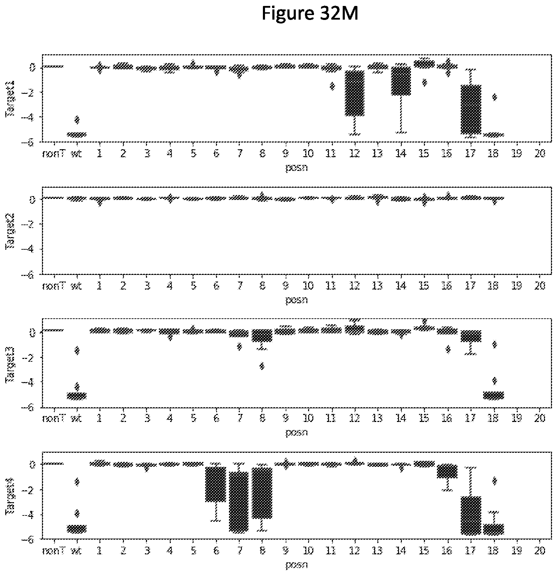

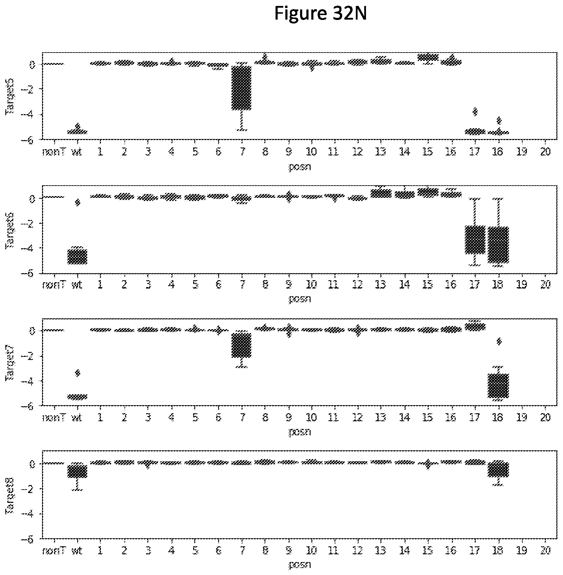

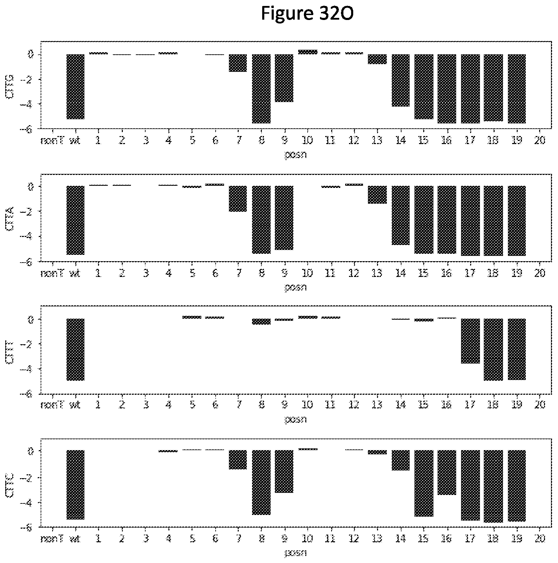

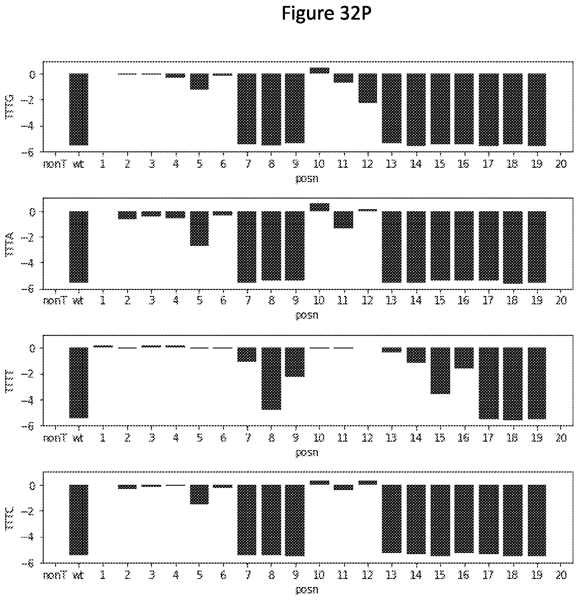

Off-target cutting effects of MAD7, MAD2, and MAD4 were further characterized. Data from an example SPOT assay using mutant libraries of various tested targets are depicted in Figures 31A-31C. MAD4 showed even less off-target activity than MAD2 and MAD7 in many of the tested target classes, as is indicated in the example data depicted in Figures 32A-32H. The mutant libraries depicted in each graph are indicated and include either random mutations (Figures 32A and 32E), deletion mutations (Figures 32B and 32F), mismatch mutations (Figures 32C and 32G), or insertion mutations (Figures 32D and 32H). Figures 32I-32P depict the depletion scores from experiments using MAD7 and the indicated mutation libraries which comprise mutations at the indicated position within each target sequence that were tested. These data are a combination of all of the YTTN PAMs used in the experiment using MAD7. For Figures 32I-32P, nonT was calculated from a 5'-NGGN PAM and wt (wildtype) is a control with no mutations in the target, and "posn" is the position of the mutation within the target sequence. Figures 32I-32J depict data from a 1 bp scanning mutation library (m1). Figures 32K-32L depict data from a 2 bp scanning mutation library (m2), which have 2 consecutive mutations starting at the indicated position. Figures 32M-32N depict data from a 3 bp scanning mutation library (m3), which have three consecutive mutations starting at the indicated position. Combined, these data indicate that the seed sequence of positions 1-7 are important as mutations within this region tend to disrupt MAD7 cleavage activity, as indicated by the reduced depletion scores for mutations within these positions. Figures 32O-32P depict data the 2 bp scanning mutation library (m2) and depict data from one target organized by the PAM sequence. Combined, these data indicate that some PAM sequences are less specific than others, which suggests a relationship between PAM strength and target specificity. For example, the TTTA PAM seems to support cutting of templates with 2 bp tandem mismatches at a higher rate than the rest of the tested combinations, for example when compared to the CTTT PAM. These observations suggest that the PAM strength should be factored into target choice.

Example 16. Compatible guide RNA sequence characterization

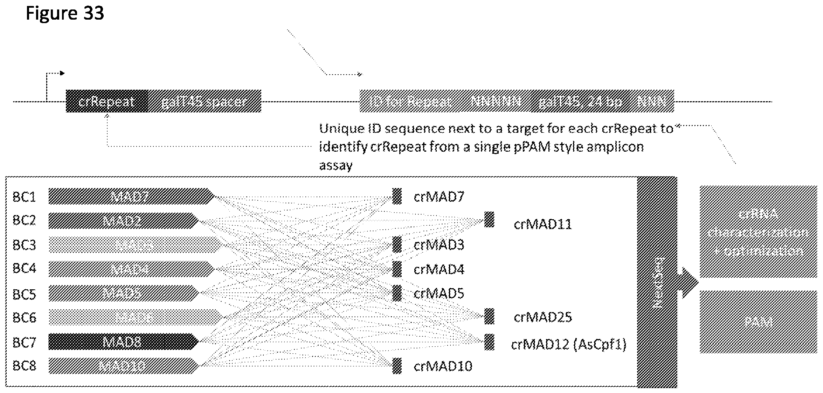

Various guide RNAs comprising different scaffold sequences were tested in order to characterize which scaffold sequences are compatible with the tested MAD nucleases. Scaffolds from the subject MAD nuclease's endogenous CRISPR array were tested, as were heterologous scaffold sequences derived from heterologous or orthogonal MAD nuclease systems. Figure 33 depicts an example schematic of the various MAD nuclease and guide RNA scaffold combinations that were tested and the construct that was used to test the compatibility of the MAD nuclease (e.g., MAD#) and guide RNA scaffold sequence (e.g., crMAD#) being tested. The construct contained a promoter driving expression of the guide RNA, which comprises the scaffold sequence (crRepeat) and the targeting sequence (e.g., galT45 spacer). The construct also contained a target sequence (e.g., galT45, 24 bp) which was targeted by the targeting sequence of the guide RNA. Flanking the target sequence were 3-4 bp PAM regions. Each construct also contained a unique sequence ID or barcode that were used to identify the scaffold sequence. The identification could occur, for example, after amplification of the barcode/unique ID and the target sequence region using primers flanking that region, as indicated in Figure 33. In some examples, the amplification results in a 176 bp amplicon, which allows for 50 bp reads with 12 bp index reads using a V3 HIGH Throughput 1x75 bp kit.

In an exemplary experiment, 12 difference MAD enzymes were tested with 10 different scaffold sequences, the resulting amplicons from which were subsequently sequences on a single NextSeq Run.

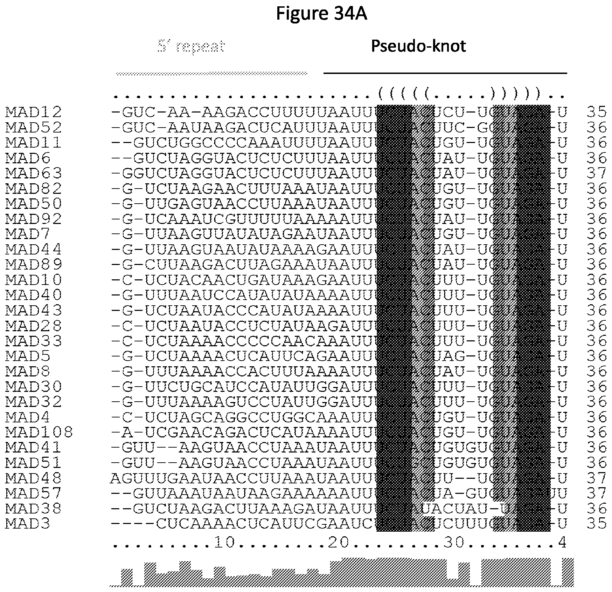

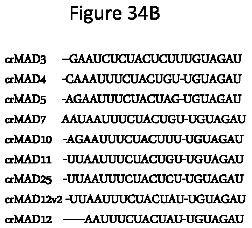

This assay was used to test the compatibility of specific MAD enzymes with various scaffold sequences. Primary and secondary structures of some of the tested scaffold sequences (crMAD#) are depicted in Figures 34A and 34B. Figure 34A depicts an alignment of the crRNA scaffold sequences from the indicated MAD system. Figure 34B depicts a portion of the pseudoknot region from the indicated MAD crRNAs. These alignments in Figures 34A-34B indicate a strong sequence and structural conservation of the pseudoknot region of these aligned MAD crRNAs. The alignments further indicate that the presence, though not necessarily the sequence, of the 5' end of the repeat region (as marked in Figure 34A) may be less relevant for cleavage activity. Sequence variations from the consensus sequence from select MAD crRNAs are indicated; for example, the 5' most pictured nucleotide is a C in the MAD3 crRNA as opposed to the U in the consensus sequence. As further example, the second nucleotide in the loop structure is an A in the consensus sequence and is instead a 1) U in the MAD10 crRNA, 2) G in the MAD4, MAD7, and MAD11 crRNAs, 3) C in the MAD25 crRNA, and 4) a CU in the MAD3 crRNA. As a further example, the third nucleotide in the loop structure of the consensus sequence is a U and is instead a G in the MAD5 crRNA. As is shown, for some MAD crRNAs, the loop sequence is 4 nucleotides in length, while in others (e.g., MAD3 crRNA) the loop sequence is 4 nucleotides in length.

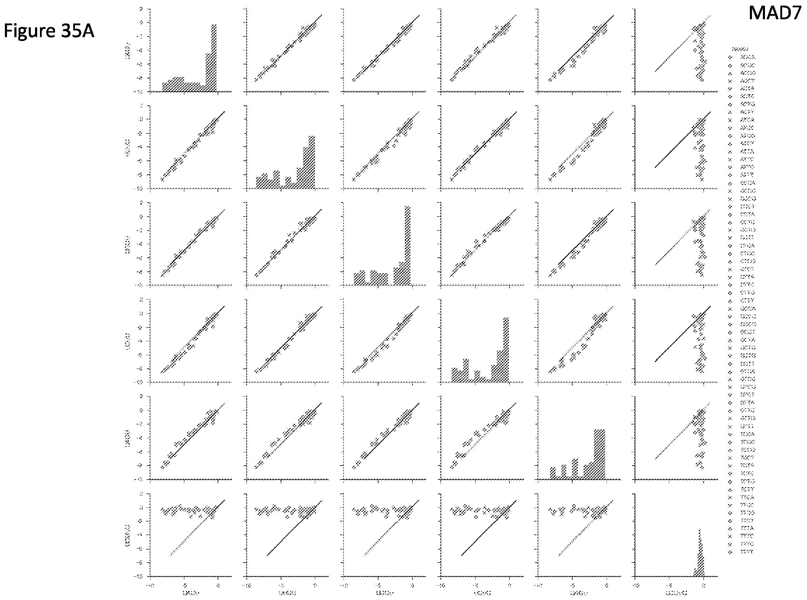

Example results from this assay are depicted in Figures 35A-35C for MAD7, MAD2, and MAD4 respectively. Some observations from each data set are listed below.

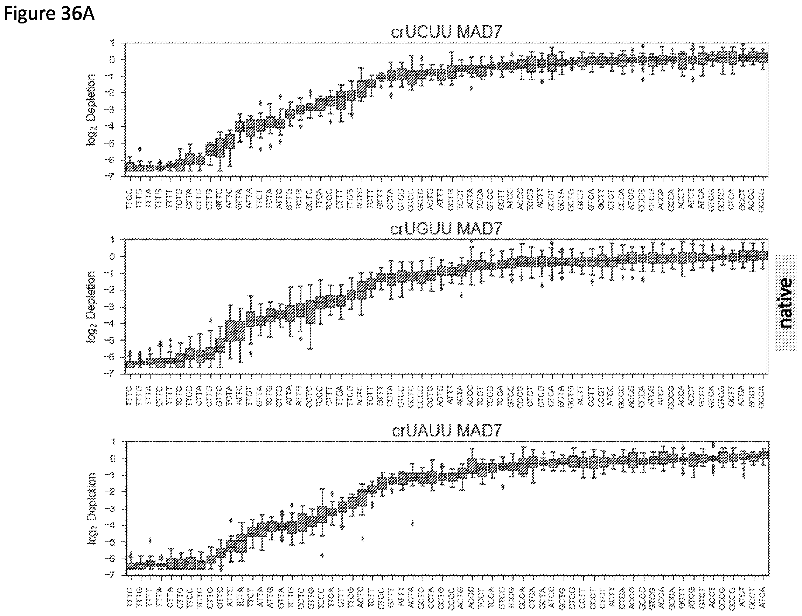

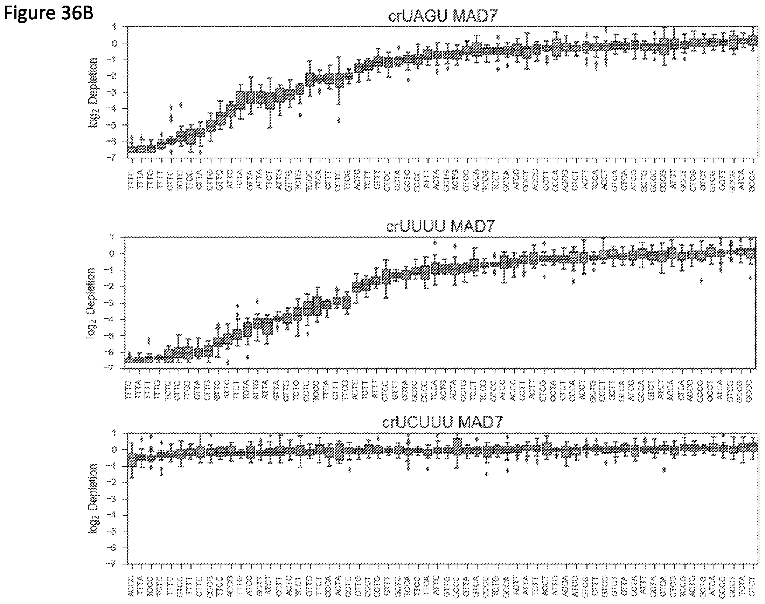

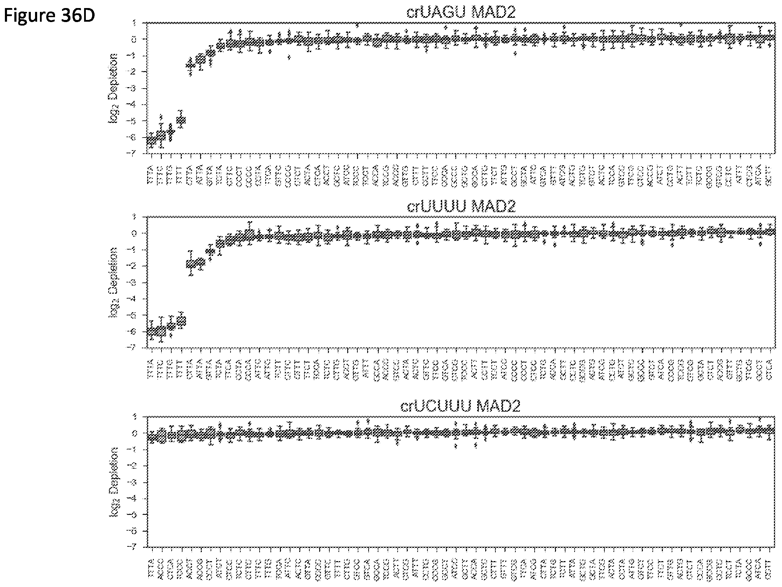

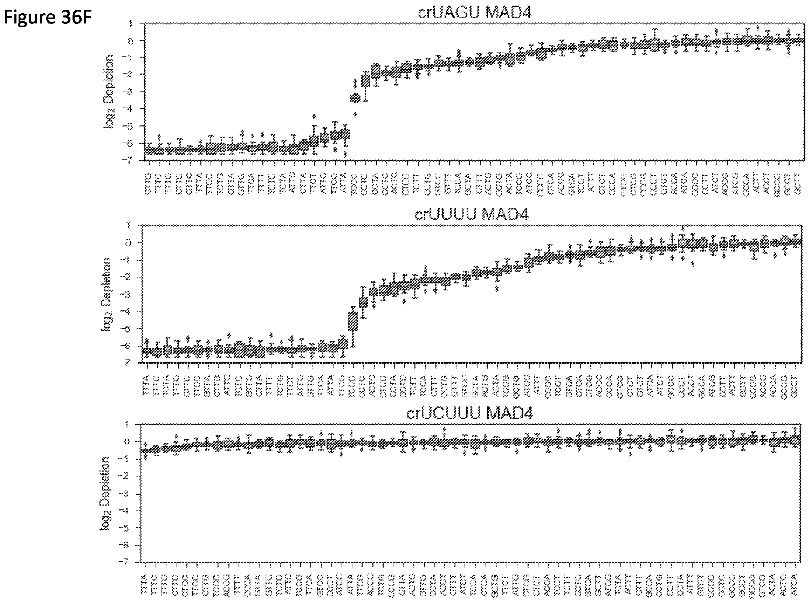

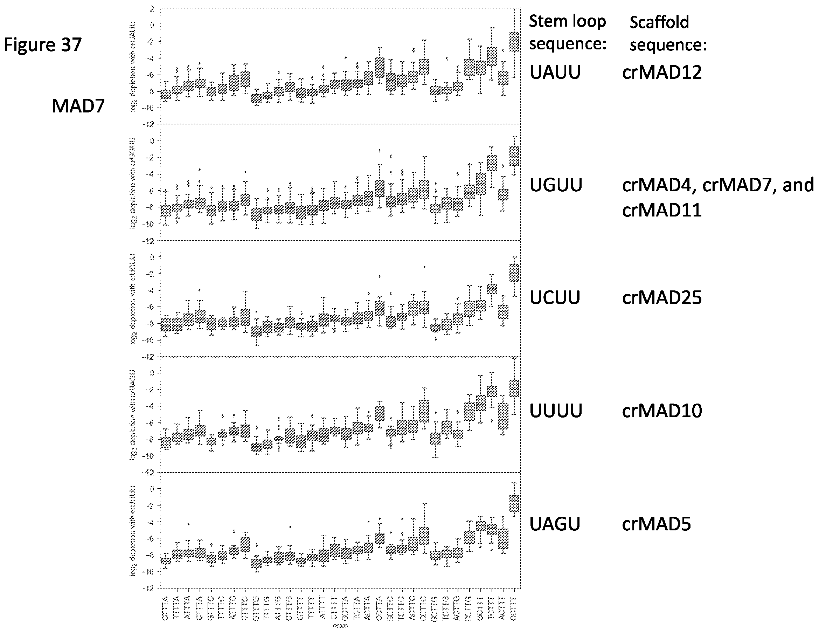

As depicted in Figure 35A, MAD7 appears to be compatible with most of the tested scaffold sequences with a 4 bp loop. There also appeared to be slightly less cleavage activity with a UAGU loop sequence when coupled with weaker PAM sequences. MAD7 activity is widely spreads over a range of PAM sequences as indicated by respective depletion scores. Combined, these results suggest MAD7 activity could be tuned by engineering of the scaffold sequence. Additional data from these MAD7 experiments is depicted in Figures 36A-36B, which shows a comparison of MAD7's compatibility with its native scaffold sequence and heterologous scaffold sequences comprising different stem loop sequences. Additional data showing the compatibility of MAD7 with various scaffold sequences with different stem loop sequences is depicted in Figure 37. As this data shows, the -1 position sequence upstream of the PAM appears to be important and the first "C" nucleotide at the -1 position adversely affects depletion. There appears to be the following preference at the -1 position: G>T>A>C, except for the situation of a CTTT PAM, in which case the preference appears to be: A>G>T>C. Pyrimidine (T/C) rich spacer sequences, especially in seed region, appeared to be slightly disfavored by both MAD7 and MAD2 while purine (A/G) rich slightly enriched Having a C upstream of PAM is also appeared detrimental.

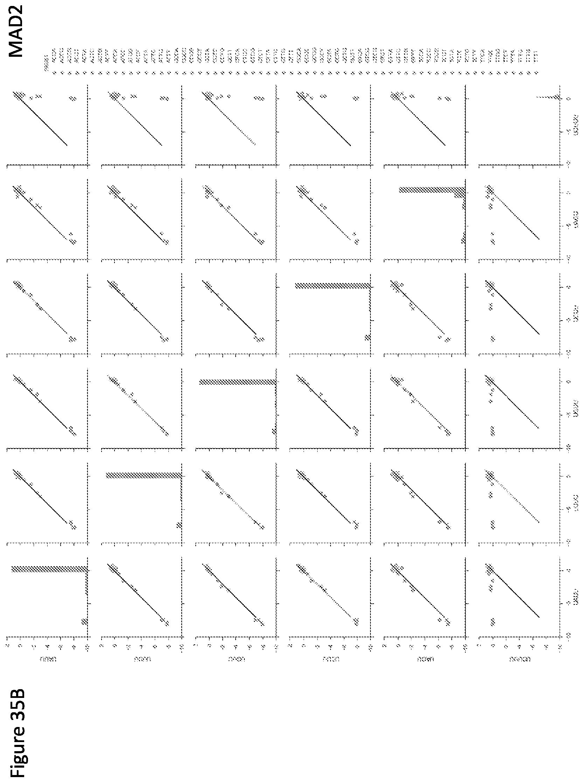

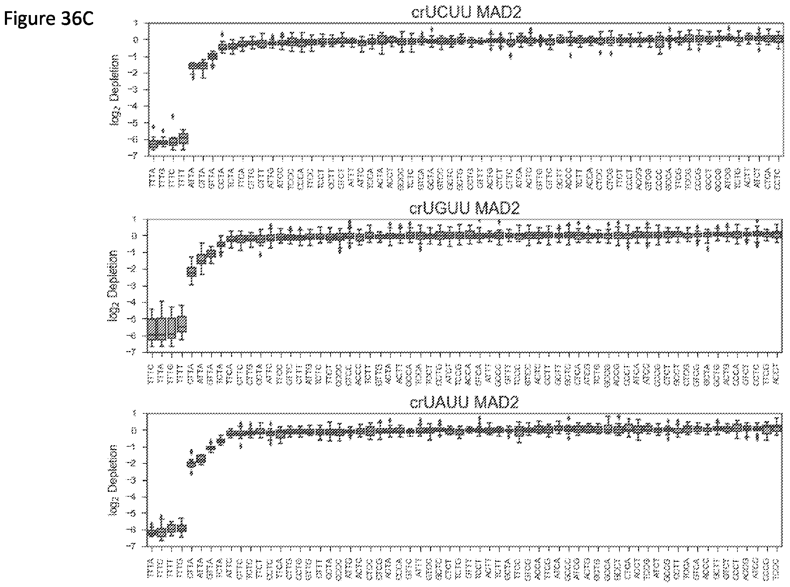

As depicted in Figure 35B, MAD2 appears to be compatible with most of the tested scaffolds with a 4 bp loop. Bimodal PAM recognition was also observed. Additional data from these experiments is depicted in Figures 36C-37D, which shows a comparison of MAD2's compatibility with its native scaffold sequence and heterologous scaffold sequences comprising different stem loop sequences.

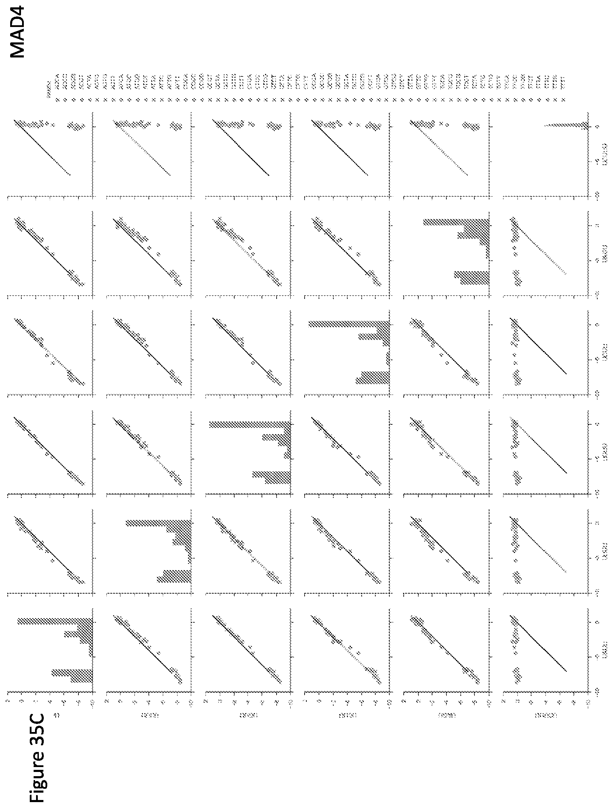

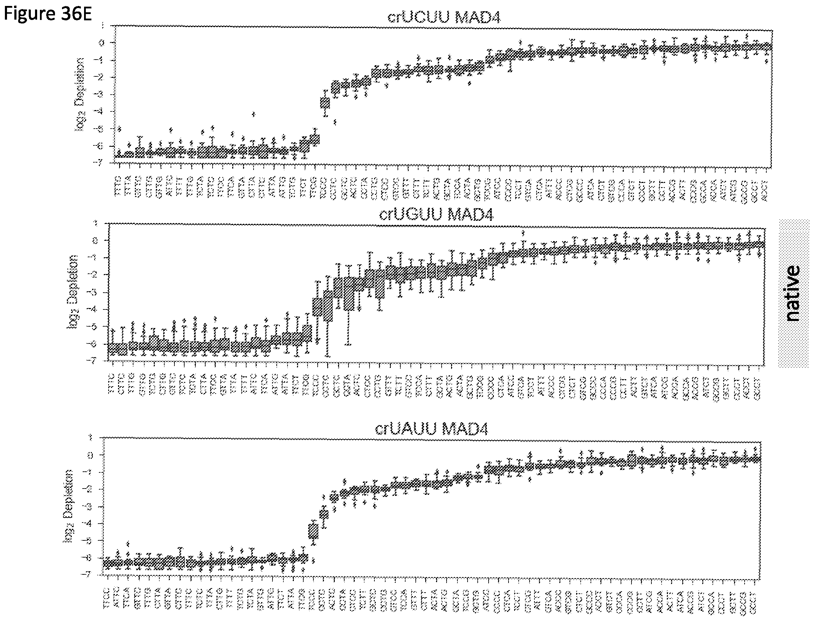

As depicted in Figure 35C, MAD4 appears to be compatible with most of the tested scaffolds with a 4 bp loop. There also appeared to be slightly less cleavage activity with a UAGU loop sequence when coupled with weaker PAM sequences. PAM recognition appeared to be slightly bimodal. Additional data from these experiments is depicted in Figures 36E-36F, which shows a comparison of MAD4's compatibility with its native scaffold sequence and heterologous scaffold sequences comprising different stem loop sequences.

Example 17. Characterization of MAD7 activity in yeast

A CAN1 selection assay was used to test a number of yeast MAD7 cassette architectures. In some example experiments, eleven different cassettes with various homology arm orientations were tested for editing efficiency of the target sequence. In general, yeast cells were transformed with one of the subject cassettes and a vector expressing the MAD7 enzyme that was codon optimized for expression in *S. cerevisiae.* The transformed cells were plated on canavanine for selection. Only cells that were successfully edited would survive the canavanine selection, indicating that the MAD7 and cassette combination was successful in effecting the desired edit. DNA was then extracted from selected cells and the desired edit was confirmed by either digestion or sequencing. Details about the tested cassettes are provided in Table 6. In Table 6, underlined nucleotides indicate potential problem sequences due potential for penta-T motifs to serve as a transcription termination signal in eukaryotes and can therefore cause expression problems. So, these potential problem sequences were altered in the various tested linker sequences by either removing or replacing one or more of the nucleotides with the nucleotides indicated in bolded italics. Data from an example experiment is summarized in Table 7. These data indicate that the 36bp T to C - F style cassette is a preferred orientation and organization.

Table 6.

| Cassette | Length (bp) | Linker sequence | Scaffold sequence |
|---|---|---|---|
| Cassette E and F (MAD12 crRNA) | 35 | GTCAAAAGACCTTTTTT (SEQ ID NO: 386) | AATTTCTACTCTTGTAGAT (SEQ ID NO: 387) |
| Cassette C and D (Alternate crRNA) | 36 | GTCTGGCCCCAAATTTT (SEQ ID NO: 388) | AATTTCTACTGTTGTAGAT (SEQ ID NO: 389) |
| gg-mini | 21 | *GG* | AATTTCTACTCTTGTAGAT (SEQ ID NO: 387) |
| Cassette A and B mini | 19 | | AATTTCTACTCTTGTAGAT (SEQ ID NO: 387) |
| E and F style (Ts to Gs) | 35 | GTCAAAAGACCT*G*T*G*T (SEQ ID NO: 390) | AATTTCTACTCTTGTAGAT (SEQ ID NO: 387) |
| E and F style (T to C) | 36 | GTCTGGCCCCAAATT*C*T (SEQ ID NO: 391) | AATTTCTACTGTTGTAGAT (SEQ ID NO: 389) |

Table 7.

| crRNA | Colonies plated on Canavanine | Colonies with growth on Canavinine | Growth Percentage | Successful PCRs from canavanine colonies | Edits observed from PCR digest | Edit percentage of PCRs |
|---|---|---|---|---|---|---|
| 35bp Ts to Gs - E style | 35 | 31 | 88.5% | 11 | 11 | 100% |
| 36bp T to C - E style | 35 | 34 | 97.1% | 13 | 13 | 100% |
| 35bp Ts to Gs - F style | 30 | 26 | 86.7% | 17 | 15 | 88.2% |
| 36bp T to C - F style | 30 | 30 | 100% | 9 | 9 | 100% |
| 21bp crRNA gg-mini | 35 | 25 | 71.4% | 17 | 10 | 58.8% |
| Cassette A | 10 | 0 | 0% | - | - | - |
| Cassette B | 10 | 0 | 0% | - | - | - |
| Cassette C | 35 | 34 | 97.1% | 25 | 25 | 100% |
| Cassette D | 34 | 32 | 94.1% | 22 | 21 | 95.4% |
| Cassette E | 35 | 35 | 100% | 26 | 23 | 88.5% |
| Cassette F | 35 | 32 | 91.4% | 18 | 14 | 77.8% |

Additional cassette arrangements were also tested, such as those depicted in Table 8. In this round of experiments, the cassettes parameters were filtered such that there were no TTTTT in the spacer (targeting sequence), and so that there were no homopolymeric regions of over 6bp in the homology arms or spacer (targeting sequence).

Table 9 summarizes the editing efficiencies of select cassettes in additional experiments, which show a range of 25-100% editing efficiency based on the expected mutations, and Table 10 summarized the sequences comprised on the indicated cassettes and other experimental details including the WT gene sequences that was targeted and the mutated sequences that was inserted following DNS cleavage with the subject MAD enzyme and crRNA complex.

Table 8.

| Cassette | crRNA scaffold | Linker sequence | Partial Scaffold sequence |
|---|---|---|---|
| Cassette G | MAD 12 | GTCTGGCCCCAAATTCT (SEQ ID NO: 391) | AATTTCTACTCTTGTAGAT (SEQ ID NO: 387) |
| Cassette H | MAD5 | GTCTGGCCCCAAATTCT (SEQ ID NO: 391) | AATTTCTACTAGTGTAGAT (SEQ ID NO: 392) |
| Cassette I | MAD 10 | GTCTGGCCCCAAATTCT (SEQ ID NO: 391) | AATTTCTACTTTTGTAGAT (SEQ ID NO: 393) |
| Cassette J | MAD4, 7, and 11 | GTCTGGCCCCAAATTCT (SEQ ID NO: 391) | AATTTCTACTGTTGTAGAT (SEQ ID NO: 389) |
| Cassette K | MAD25 | GTCTGGCCCCAAATTCT (SEQ ID NO: 391) | AATTTCTACTATTGTAGAT (SEQ ID NO: 394) |

Table 9.

| | Cassette L | Cassette M | Cassette N | Cassette O |
|---|---|---|---|---|
| Strain | 1 | 1 | 1 | 2 |
| Edit% (pipeline) | 8% | 8% | ND | 9% |
| Expected mutations / read | 7 | 8 | 6 | 5 |
| # Edited reads / # total reads | 7/85 | 22/55 | 20/119 | 6/64 |

Table 10.

| | Cassette L | Cassette M | Cassette N | Cassette O |
|---|---|---|---|---|
| crRNA targeting sequence | SEQ ID NO: 360 | SEQ ID NO: 361 | SEQ ID NO: 362 | SEQ ID NO: 363 |
| Mutated sequence inserted into cleaved site | SEQ ID NO: 364 | SEQ ID NO: 365 | SEQ ID NO: 366 | SEQ ID NO: 367 |
| WT gene sequence before mutation | SEQ ID NO: 368 | SEQ ID NO: 369 | SEQ ID NO: 370 | SEQ ID NO: 371 |
| Editing cassette | SEQ ID NO: 372 | SEQ ID NO: 373 | SEQ ID NO: 374 | SEQ ID NO: 375 |

Example 18. Cleavage of mammalian target sequences *in vitro* and *in vivo*.

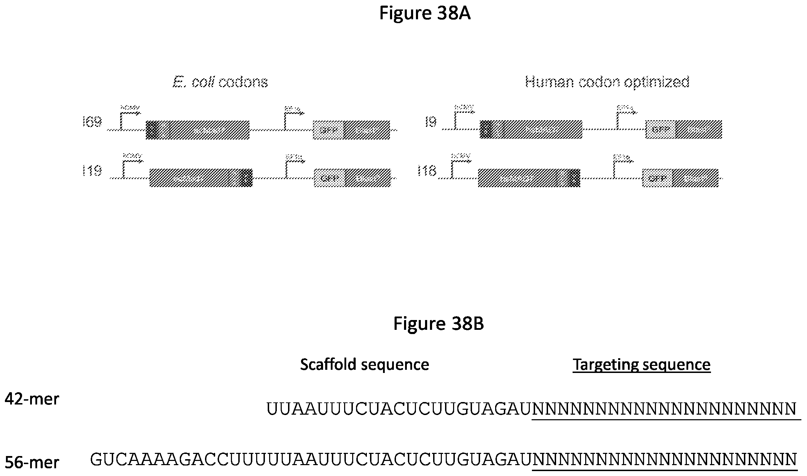

MAD7 was used to target and cleave mammalian cell targets. *E. coli* or human codon optimized MAD7 expression constructs were generated (E.g., Figure 38A). Briefly, construct 169 contained a hCMV promoter operably linked to an *E. coli* codon optimized MAD7 sequence which also contained a 5' (N-terminal) V5 epitope tag and nuclear localization signal (NLS), and the construct also contains an EF1alpha promoter driving expression of a GFP reporter and Blasticidin resistance gene. Construct 119 is similar to 169 with the exception that the NLS and V5 sequences are on the 3' end (C-terminal) of the MAD7 sequence. The 19 construct is similar to the 169 sequence with the exception that the MAD7 sequence is codon optimized for expression in human cells instead of *E. coli.* Construct 118 is similar to the 119 construct with the exception that the MAD7 sequence is codon optimized for expression in human cells instead of *E. coli.* Two guide RNA sequences were used as shown in Figure 38B.

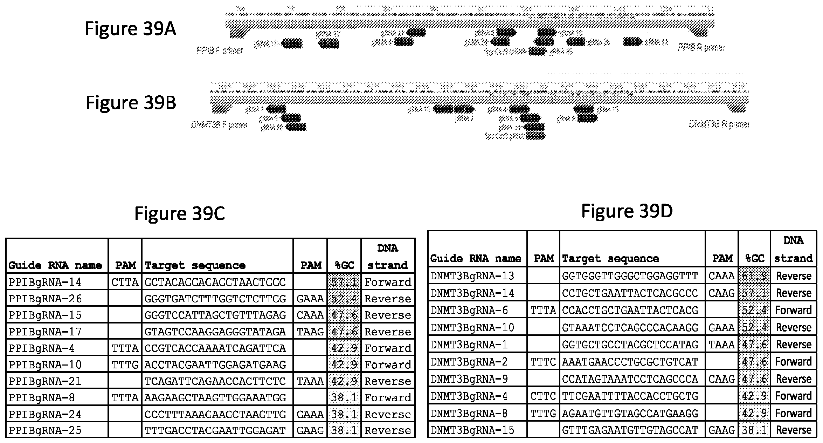

Figures 39A-39B depicts the target sites within the two target genes, PPIB (Figure 39A) and DNMT3B (Figure 39B). Figures 39C-39D summarize the PAM sequence and target sequence targeted by the indicated guide RNA sequences, as well as the CG content percentage of the target sequence and which DNA strand is targeted.

The MAD7 expression vectors were each transfected separately into mammalian cells and then cell lysates were collected. The cell lysates were assessed by western blot analysis to confirm MAD7 expression. The cell lysates were also used in an *in vitro* cutting assay to assess MAD7 protein function, which would indicate proper expression and folding of the protein expressed within mammalian cells. This assay allows for assessment of the expression and cleavage ability of MAD7 without the possibility of the cell machinery correcting the cleavage event or blocking it due to chromatin compaction of the target sequence. After the cleavage assay was run, the lysate was separated by gel electrophoresis and analyzed using ImageJ software and densitometry to determine the % cleavage (% cutting), which was calculated by dividing the cleavage product intensity by the total band intensity.

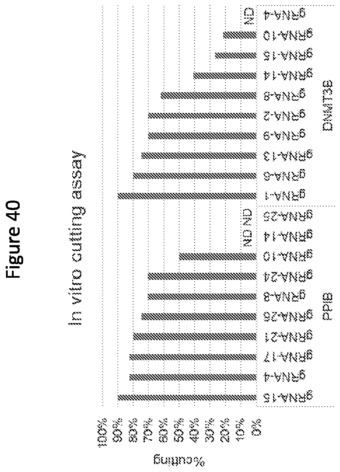

Figure 40 shows quantification of the percent cleavage (cutting efficiency) from an example experiment for the indicated PPIB and DNMT3B target sequences using the 118 construct. Little difference in cleavage was observed *in vitro* between the 42-mer and 56-mer guide RNA sequences so the average of the two are plotted in Figure 40. For PPIB, eight of the ten tested guide RNAs resulted in targeted cleavage, while gRNA-14 and gRNA-25 resulted in undetectable cleavage using this assay. gRNA-15 targeting PPIB resulted in efficient cleavage but was difficult to measure due to the small size of the generated cleavage product (approximately 70 bp difference between uncut) so the percent cleavage was estimated to be > 90%. For DNMT3B, nine of the ten tested guide RNAs resulted in targeted cleavage, while gRNA-4 resulted in undetectable cleavage using this assay. gRNA-1 targeting DNMT3B resulted in efficient cleavage but was difficult to measure due to the small size of the generated cleavage product (approximately 70 bp difference between uncut) so the percent cleavage was estimated to be > 90%.

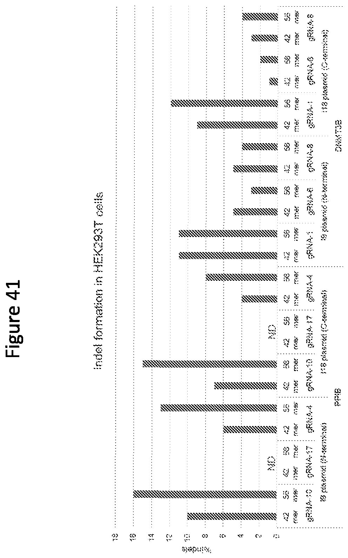

Figure 41 shows the quantification of indel (insertion or deletion) formation in mammalian cells. Briefly, a MAD7 nuclease expression vector (19 or 118; see Figure 38A) and a synthetic guide RNA were co-transfected into HEK293T cells. After approximately 72 hour incubation period, *in vivo* cleavage was assessed by detecting indel formation. Without wishing to be bound by theory, after cleavage occurs, in some cases, the cleaved DNA is repaired through non-homologous end-joining (NHEJ) mechanisms, which often results in one or more nucleotides being inserted or deleted from the cleavage site. These insertion and/or deletion events are referred to as indels. Indels were detected using a DNA mismatch detection assay using the T7EI assay. This mismatch assay detects mismatches between the expected non-altered sequences and the altered sequence comprising indels following the cleavage and NHEJ repair. In some cases, the 56-mer guide RNA resulted in higher indel formation compared to the 42-mer guide RNA. For PPIB, two of three guide RNAs resulted in detectable cleavage by this method, and three of three for the DNMT3B gene target. The other two constructs, which used *E. coli* codon optimized MAD7 nuclease also demonstrated indel formation at relatively the same efficiency as the human codon optimized constructs (data not shown). Overall, these data show successful *in vivo* cleavage activity of MAD7 in mammalian cells.

Combined, these data indicate that MAD7 is efficiently expressed and folded in mammalian cells and functions efficiently *in vitro* and *in vivo* on mammalian target sequences. The *in vitro* data also indicate that the shorter 42-mer gRNA cuts as efficiently as the longer 56-mer gRNAs, although this was not necessarily observed when measuring endogenous indel formation in mammalian cells.

Example 19. Further characterization of MAD nucleases

An editing screen was performed to further characterize the PAM preferences for select MAD nucleases. The galK gene was used as a target for this screen. Some notable observations from these data include that editing was observed with MAD7 using a GTTG and TTTC PAM and editing was observed with MAD2 when using a TTTC PAM. Some cell toxicity was observed in these experiments. Table 11 summarizes the results from these experiments.

Table 11.

| MAD nuclease | Sites cut | Sites edited | CFU count of edited cells |
|---|---|---|---|
| MAD2 | 4/7 | 2/7 | >1e5 |
| MAD4 | 5/7 | 0/7 | 0 |
| MAD7 | 5/7 | 2/7 | 1e4 |

The PAM screen assay was repeated using MAD2, MAD4, and MAD7. According to the experimental design of this assay, white colonies reflect stop codon insertions due to homologous recombination of an editing cassette containing the mutation, while a red colony would indicate that the stop codon was not inserted. MAD2 showed editing consistent with previously described PAM specificity data. MAD4 showed high cell toxicity and had fewer observable colonies compared to MAD2 and MAD7 transformed cells. MAD7 showed editing results consistent with previous PAM specificity data and showed a broader editing efficiency than MAD2.

These data show over 90% editing efficiency despite the low cell survival rates. Table 12 summarizes the results from the experiments. These data indicate that there is a lower variability between PAM sites for MAD4 (~2.3 fold) as compared to MAD2 (25 fold) or MAD7 (101 fold). This fold change was calculated as follows: Fold difference = max(editedCFU)/min(editedCFU).

Table 12.

| MAD nuclease | Sites cut | Cut sites edited | CFU count of edited cells | Fold difference between max and min edited CFU |
|---|---|---|---|---|
| MAD2 | 5/10 | 2/5 | 1.7e5 | 25 |
| MAD4 | 7/10 | 7/7 | <1.5e3 | 2.3 |
| MAD7 | 6/10 | 6/7 | 6.7e4 | 101 |

**Claims**

1. A method of modifying a target region in the genome of a cell, the method comprising:

   (a) contacting a cell with:

   a nucleic-acid-guided nuclease comprising an amino acid sequence at least 90% identical to SEQ ID NO: 7;
   an engineered guide nucleic acid capable of complexing with the nucleic acid-guided nuclease; and
   an editing sequence encoding a nucleic acid complementary to said target region having a change in sequence relative to the target region; and

   (b) allowing the nuclease, guide nucleic acid, and editing sequence to create a genome edit in the target region of the genome of the cell;

   wherein the method is not a method for modifying the germ line genetic identity of human beings.

2. A nucleic acid-guided nuclease system comprising:

   (a)

   (i) a nucleic-acid-guided nuclease comprising an amino acid sequence at least 90% identical to SEQ ID NO: 7, or
   (ii) a nucleic acid molecule encoding the nucleic-acid-guided nuclease;

   (b)

   (i) an engineered guide nucleic acid capable of complexing with the nucleic acid-guided nuclease, or
   (ii) a nucleic acid molecule encoding the engineered guide nucleic acid; and

   (c) an editing sequence encoding a nucleic acid complementary to a target region in a genome of a cell having a change in sequence relative to the sequence of the target region.

3. The method of claim 1 or system of claim 2, wherein the engineered guide nucleic acid and the editing sequence are provided as a single nucleic acid, optionally wherein the single nucleic acid further comprises a mutation in a protospacer adjacent motif (PAM) site.

4. The method of claim 1 or system of claim 2, wherein:

(a) the nucleic acid-guided nuclease is encoded by a nucleic acid with at least 85% identity to SEQ ID NO: 47, or 203-222;

(b) the nucleic acid-guided nuclease is encoded by a nucleic acid with at least 85% identity to SEQ ID NO: 133, or 183-202;

(c) the nucleic acid-guided nuclease is encoded by a nucleic acid with at least 85% identity to SEQ ID NO: 153, or 243-262; or

(d) the nucleic acid-guided nuclease is encoded by a nucleic acid with at least 85% identity to SEQ ID NO: 223-242.

5. The method of claim 1 or system of claim 2, wherein the engineered guide nucleic acid comprises the sequence of any one of SEQ ID NO: 172-182.

6. The method of claim 1 or system of claim 2, wherein the engineered guide nucleic acid comprises a loop sequence which comprises the sequence of UAUU, UUUU, UGUU, UCUU, UCUUU, or UAGU.

7. The system of claim 2, wherein the nucleic acid molecule encoding the nucleic acid-guided nuclease is codon optimized for the cell to be edited.

8. A composition comprising

   (a)

      (i) a nucleic-acid-guided nuclease comprising an amino acid sequence at least 90% identical to SEQ ID NO: 7, or
      (ii) a nucleic acid molecule encoding the nucleic-acid-guided nuclease; and

   (b)

      (i) an engineered guide nucleic acid capable of complexing with the nucleic acid-guided nuclease, or
      (ii) a nucleic acid molecule encoding the engineered guide nucleic acid, wherein the engineered guide nucleic acid comprises a loop sequence which comprises the sequence of UAUU, UUUU, UGUU, UCUU, UCUUU, or UAGU.

9. A nucleic acid-guided nuclease system comprising:

   (a)

      (i) a nucleic-acid-guided nuclease comprising an amino acid sequence at least 90% identical to SEQ ID NO: 7, or
      (ii) a nucleic acid molecule encoding the nucleic-acid-guided nuclease; and

   (b)

      (i) a heterologous engineered guide nucleic acid capable of complexing with the nucleic acid-guided nuclease, or
      (ii) a nucleic acid molecule encoding the heterologous engineered guide nucleic acid.

10. The composition of claim 8, wherein the engineered guide nucleic acid is a heterologous engineered guide nucleic acid.

11. The composition of claim 8 or system of claim 9, wherein the engineered guide nucleic acid comprises the sequence of any one of SEQ ID NO: 172-182.

12. The composition of claim 8 or system of claim 9, wherein the nuclease is encoded by a codon optimized nucleic acid sequence for:

    (a) use in cells from a particular organism;
    (b) E. coli; or

(c) S. cerevisiae; or
(d) mammalian cells; or
(e) human cells; or
(f) plant cells.

13. The system of claim 9, further comprising (c) an editing sequence encoding a nucleic acid complementary to a target region in a genome of a cell having a change in sequence relative to the sequence of the target region.

14. The system of claim 9, wherein the engineered guide nucleic acid comprises a loop sequence which comprises the sequence of UAUU, UUUU, UGUU, UCUU, UCUUU, or UAGU.

15. The method of claim 1, 3, 4, 5 or 6, or system of claim 2, 3, 4, 5, 6 or 7, wherein the nucleic-acid-guided nuclease comprises an amino acid sequence at least 95% identical to SEQ ID NO:7.

16. The method of claim 1, 3, 4, 5 or 6, or system of claim 2, 3, 4, 5, 6 or 7, wherein the nucleic-acid-guided nuclease comprises the amino acid sequence of SEQ ID NO: 7.

17. The composition of claim 8, 10, 11 or 12, or system of claim 9, 11, 12, 13 or 14, wherein the nucleic-acid-guided nuclease comprises an amino acid sequence at least 95% identical to SEQ ID NO: 7.

18. The composition of claim 8, 10, 11 or 12, or system of claim 9, 11, 12, 13 or 14, wherein the nucleic-acid-guided nuclease comprises the amino acid sequence of SEQ ID NO: 7.


**Patentansprüche**

1. Verfahren zum Modifizieren einer Zielregion im Genom einer Zelle, wobei das Verfahren Folgendes umfasst:

    (a) das Kontaktieren einer Zelle mit:

    einer Nucleinsäure-geführten Nuclease, die eine Aminosäuresequenz mit einer Identität von zumindest 90 % mit SEQ ID NO: 7 umfasst;
    einer gentechnisch hergestellten Führungsnucleinsäure, die in der Lage ist, mit der Nucleinsäure-geführten Nuclease zu komplexieren; und
    einer Bearbeitungssequenz, die für eine Nucleinsäure kodiert, die zu der Zielregion komplementär ist, die eine Sequenzveränderung in Bezug zur Zielregion aufweist; und

    (b) das Zulassen, dass die Nuclease, die Führungsnucleinsäure und die Bearbeitungssequenz eine Genombearbeitung in einer Zielregion des Genoms der Zelle erzeugen;
    wobei das Verfahren kein Verfahren zum Modifizieren der genetischen Keimbahnidentität von Menschen ist.

2. Nucleinsäure-geführtes Nucleasesystem, das Folgendes umfasst:

    (a)

    (i) eine Nucleinsäure-geführte Nuclease, die eine Aminosäuresequenz mit einer Identität von zumindest 90 % mit SEQ ID NO: 7 umfasst; oder
    (ii) ein Nucleinsäuremolekül, das für die Nucleinsäure-geführte Nuclease kodiert;

    (b)

    (i) eine gentechnisch hergestellte Führungsnucleinsäure, die in der Lage ist, mit der Nucleinsäure-geführten Nuclease zu komplexieren; oder
    (ii) ein Nucleinsäuremolekül, das für die gentechnisch hergestellte Führungsnucleinsäure kodiert; oder

    (c) eine Bearbeitungssequenz, die für eine Nucleinsäure kodiert, die zu einer Zielregion in einem Genom einer Zelle komplementär ist, die eine Sequenzveränderung in Bezug zur Sequenz der Zielregion aufweist.

**3.** Verfahren nach Anspruch 1 oder System nach Anspruch 2, wobei die gentechnisch hergestellte Führungsnucleinsäure und die Bearbeitungssequenz als eine einzelne Nucleinsäure bereitgestellt sind, wobei die einzelne Nucleinsäure gegebenenfalls außerdem eine Mutation an der Protospacer-Adjacent-Motif- (PAM-) Stelle umfasst.

**4.** Verfahren nach Anspruch 1 oder System nach Anspruch 2, wobei:

(a) für die Nucleinsäure-geführte Nuclease eine Nucleinsäure mit zumindest 85 % Identität mit SEQ ID NO: 47 oder 203-222 kodiert;
(b) für die Nucleinsäure-geführte Nuclease eine Nucleinsäure mit zumindest 85 % Identität mit SEQ ID NO: 133 oder 183-202 kodiert;
(c) für die Nucleinsäure-geführte Nuclease eine Nucleinsäure mit zumindest 85 % Identität mit SEQ ID NO: 153 oder 243-262 kodiert; oder
(d) für die Nucleinsäure-geführte Nuclease eine Nucleinsäure mit zumindest 85 % Identität mit SEQ ID NO: 223-242 kodiert.

**5.** Verfahren nach Anspruch 1 oder System nach Anspruch 2, wobei die gentechnisch hergestellte Führungsnucleinsäure eine beliebige der SEQ ID NO: 172-182 umfasst.

**6.** Verfahren nach Anspruch 1 oder System nach Anspruch 2, wobei die gentechnisch hergestellte Führungsnucleinsäure eine Schleifensequenz umfasst, die die Sequenz UAUU, UUUU, UGUU, UCUU, UCUUU oder UAGU umfasst.

**7.** System nach Anspruch 2, wobei das Nucleinsäuremolekül, das für die Nucleinsäure-geführte Nuclease kodiert, für die zu bearbeitende Zelle Codon-optimiert ist.

**8.** Zusammensetzung, die Folgendes umfasst:

(a)

(i) eine Nucleinsäure-geführte Nuclease, die eine Aminosäuresequenz mit einer Identität von zumindest 90 % mit SEQ ID NO: 7 umfasst; oder
(ii) ein Nucleinsäuremolekül, das für die Nucleinsäure-geführte Nuclease kodiert; und

(b)

(i) eine gentechnisch hergestellte Führungsnucleinsäure, die in der Lage ist, mit der Nucleinsäure-geführten Nuclease zu komplexieren, oder
(ii) ein Nucleinsäuremolekül, das für die gentechnisch hergestellte Führungsnucleinsäure kodiert, wobei die gentechnisch hergestellte Führungsnucleinsäure eine Schleifensequenz umfasst, die die Sequenz UAUU, UUUU, UGUU, UCUU, UCUUU oder UAGU umfasst.

**9.** Nucleinsäure-geführtes Nucleasesystem, das Folgendes umfasst:

(a)

(i) eine Nucleinsäure-geführte Nuclease, die eine Aminosäuresequenz mit einer Identität von zumindest 90 % mit SEQ ID NO: 7 umfasst; oder
(ii) ein Nucleinsäuremolekül, das für die Nucleinsäure-geführte Nuclease kodiert; und

(b)

(i) eine heterologe gentechnisch hergestellte Führungsnucleinsäure, die in der Lage ist, mit der Nucleinsäure-geführten Nuclease zu komplexieren, oder
(ii) ein Nucleinsäuremolekül, das für die heterologe gentechnisch hergestellte Führungsnucleinsäure kodiert.

**10.** Zusammensetzung nach Anspruch 8, wobei die gentechnisch hergestellte Führungsnucleinsäure eine heterologe gentechnisch hergestellte Führungsnucleinsäure ist.

**11.** Zusammensetzung nach Anspruch 8 oder System nach Anspruch 9, wobei die gentechnisch hergestellte Führungsnucleinsäure eine beliebige der SEQ ID NO: 172-182 umfasst.

**12.** Zusammensetzung nach Anspruch 8 oder System nach Anspruch 9, wobei eine für

    (a) die Verwendung in Zellen eines spezifischen Organismus;
    (b) E. coli; oder
    (c) S. cerevisiae; oder
    (d) Säugetierzellen; oder
    (e) menschliche Zellen; oder
    (f) Pflanzenzellen

Codon-optimierte Nucleinsäuresequenz für die Nuclease kodiert.

**13.** System nach Anspruch 9, das außerdem (c) eine Bearbeitungssequenz umfasst, die für eine Nucleinsäure kodiert, die komplementär zu einer Zielregion in einem Genom einer Zelle ist, die eine Sequenzveränderung in Bezug auf die Sequenz der Zielregion aufweist.

**14.** System nach Anspruch 9, wobei die gentechnisch hergestellte Führungsnucleinsäure eine Schleifensequenz umfasst, die die Sequenz UAUU, UUUU, UGUU, UCUU, UCUUU oder UAGU umfasst.

**15.** Verfahren nach Anspruch 1, 3, 4, 5 oder 6 oder System nach Anspruch 2, 3, 4, 5, 6 oder 7, wobei die Nucleinsäure-geführte Nuclease eine Aminosäuresequenz mit einer Identität von zumindest 95 % mit SEQ ID NO: 7 umfasst.

**16.** Verfahren nach Anspruch 1, 3, 4, 5 oder 6 oder System nach Anspruch 2, 3, 4, 5, 6 oder 7, wobei die Nucleinsäure-geführte Nuclease die Aminosäuresequenz von SEQ ID NO: 7 umfasst.

**17.** Zusammensetzung nach Anspruch 8, 10, 11 oder 12 oder System nach Anspruch 9, 11, 12, 13 oder 14, wobei die Nucleinsäure-geführte Nuclease eine Aminosäuresequenz mit einer Identität von zumindest 95 % mit SEQ ID NO: 7 umfasst.

**18.** Zusammensetzung nach Anspruch 8, 10, 11 oder 12 oder System nach Anspruch 9, 11, 12, 13 oder 14, wobei die Nucleinsäure-geführte Nuclease die Aminosäuresequenz von SEQ-ID NO: 7 umfasst.

**Revendications**

**1.** Procédé de modification d'une région cible dans le génome d'une cellule, le procédé comprenant les étapes consistant à :

    (a) mettre en contact une cellule avec :

        une nucléase guidée par acide nucléique comprenant une séquence d'acides aminés ayant au moins 90 % d'identité avec SEQ ID NO : 7 ;
        un acide nucléique-guide génétiquement modifié capable de se complexer avec la nucléase guidée par acide nucléique ; et
        une séquence d'édition codant pour un acide nucléique complémentaire de ladite région cible présentant un changement de séquence par rapport à la région cible ; et

    (b) permettre à la nucléase, à l'acide nucléique-guide et à la séquence d'édition de créer une édition de génome dans une région cible du génome de la cellule,

dans lequel le procédé n'est pas un procédé pour modifier l'identité génétique de lignée germinale d'êtres humains.

**2.** Système de nucléase guidé par acide nucléique comprenant :

    (a)

(i) une nucléase guidée par acide nucléique comprenant une séquence d'acides aminés ayant au moins 90 % d'identité avec SEQ ID NO : 7 ; ou

(ii) une molécule d'acide nucléique codant pour la nucléase guidée par acide nucléique ;

(b)

(i) un acide nucléique-guide génétiquement modifié capable de se complexer avec la nucléase guidée par acide nucléique ; ou

(ii) une molécule d'acide nucléique codant pour l'acide nucléique-guide génétiquement modifié ; et

(c) une séquence d'édition codant pour un acide nucléique complémentaire d'une région cible dans un génome d'une cellule ayant un changement de séquence par rapport à la séquence de la région cible.

3. Procédé selon la revendication 1 ou système selon la revendication 2, dans lequel l'acide nucléique-guide génétiquement modifié et la séquence d'édition sont fournis sous la forme d'un acide nucléique unique, facultativement dans lequel l'acide nucléique unique comprend en outre une mutation dans un site de motif adjacent d'espaceur (PAM).

4. Procédé selon la revendication 1 ou système de revendication 2, dans lequel :

(a) la nucléase guidée par acide nucléique est codée par un acide nucléique ayant au moins 85 % d'identité avec SEQ ID NO : 47, ou 203 à 222 ;

(b) la nucléase guidée par acide nucléique est codée par un acide nucléique ayant au moins 85 % d'identité avec SEQ ID NO : 133, ou 183 à 202 ;

(c) la nucléase guidée par acide nucléique est codée par un acide nucléique ayant au moins 85 % d'identité avec SEQ ID NO : 153, ou 243 à 262 ; ou

(d) la nucléase guidée par acide nucléique est codée par un acide nucléique ayant au moins 85 % d'identité avec SEQ ID NO : 223 à 242.

5. Procédé selon la revendication 1 ou système selon la revendication 2, dans lequel l'acide nucléique-guide génétiquement modifié comprend la séquence de l'une quelconque des SEQ ID NO : 172 à 182.

6. Procédé selon la revendication 1 ou système selon la revendication 2, dans lequel l'acide nucléique-guide génétiquement modifié comprend une séquence en boucle qui comprend la séquence de UAUU, UUUU, UGUU, UCUU, UCUUU ou UAGU.

7. Système selon la revendication 2, dans lequel la molécule d'acide nucléique codant pour la nucléase guidée par acide nucléique est optimisée par codon pour la cellule à éditer.

8. Composition comprenant :

a)

(i) une nucléase guidée par acide nucléique comprenant une séquence d'acides aminés ayant au moins 90 % d'identité avec SEQ ID NO : 7 ; ou

(ii) une molécule d'acide nucléique codant pour la nucléase guidée par acide nucléique ;

(b)

(i) un acide nucléique-guide génétiquement modifié capable de se complexer avec la nucléase guidée par acide nucléique ; ou

(ii) une molécule d'acide nucléique codant pour l'acide nucléique-guide génétiquement modifié, l'acide nucléique-guide génétiquement modifié comprenant une séquence en boucle qui comprend la séquence de UAUU, UUUU, UGUU, UCUU, UCUUU ou UAGU.

9. Système de nucléase guidé par acide nucléique comprenant :

a)

(i) une nucléase guidée par acide nucléique comprenant une séquence d'acides aminés ayant au moins 90 % d'identité avec SEQ ID NO : 7 ; ou

(ii) une molécule d'acide nucléique codant pour la nucléase guidée par acide nucléique ; et

(b)

(i) un acide nucléique-guide génétiquement modifié hétérologue capable de se complexer avec la nucléase guidée par acide nucléique, ou

(ii) une molécule d'acide nucléique codant pour l'acide nucléique-guide génétiquement modifié

10. Composition selon la revendication 8, dans laquelle l'acide nucléique-guide génétiquement modifié est un acide nucléique-guide génétiquement modifié hétérologue.

11. Composition selon la revendication 8 ou système selon la revendication 9, dans lequel l'acide nucléique-guide génétiquement modifié comprend la séquence de l'une quelconque des SEQ ID NO : 172 à 182.

12. Composition selon la revendication 8 ou système selon la revendication 9, dans lequel la nucléase est codée par une séquence d'acide nucléique optimisée par codon pour :

(a) utilisation dans des cellules d'un organisme particulier ;
(b) E. coli ; ou
(c) S. cerevisiae ; ou
(d) des cellules de mammifères ; ou
(e) des cellules humaines ; ou
(f) des cellules végétales.

13. Système selon la revendication 9, comprenant en outre (c) une séquence d'édition codant pour un acide nucléique complémentaire d'une région cible dans un génome d'une cellule ayant un changement de séquence par rapport à la séquence de la région cible.

14. Système selon la revendication 9, dans lequel l'acide nucléique-guide génétiquement modifiée comprend une séquence en boucle qui comprend la séquence de UAUU, UUUU, UGUU, UCUU, UCUUU ou UAGU.

15. Procédé selon la revendication 1, 3, 4, 5 ou 6, ou système selon la revendication 2, 3, 4, 5, ou 7, dans lequel la nucléase guidée par acide nucléique comprend une séquence d'acides aminés ayant au moins 95 % d'identité avec SEQ ID NO : 7.

16. Procédé selon la revendication 1, 3, 4, 5 ou 6, ou système selon la revendication 2, 3, 4, 5, ou 7, dans lequel la nucléase guidée par acide nucléique comprend une séquence d'acides aminés de SEQ ID NO : 7.

17. Composition selon la revendication 8, 10, 11 ou 12, ou système selon la revendication 9, 11, 12, 13 ou 14, dans lequel dans lequel la nucléase guidée par acide nucléique comprend une séquence d'acides aminés ayant au moins 95 % d'identité avec SEQ ID NO : 7.

18. Composition selon la revendication 8, 10, 11 ou 12, ou système selon la revendication 9, 11, 12, 13 ou 14, dans lequel dans lequel la nucléase guidée par acide nucléique comprend la séquence d'acides aminés de SEQ ID NO : 7.

Figure 1A

Figure 1B

Figure 2

EP 3 642 334 B1

MAD protein expression

Figure 3

Figure 4

# Screening/selection for gene edits

**Figure 5A**

## MAD nuclease vector

promoter       promoter

**Figure 5B**

## Editing cassette

Guide nucleic acid

promoter

**Figure 5C**

Cell Death (when MAD nuclease and guide nucleic acid are active)

No Editing
(when MAD nuclease and guide nucleic acid are inactive)

Editing
(when MAD nuclease and guide nucleic acid are active and gene was successfully edited)

## PAM selection assay

Figure 6A

### MAD nuclease vector

promoter    promoter

Figure 6B

### Editing cassette (self targeting)

Guide nucleic acid

promoter

Random PAMs

Figure 6C

Genome

dsDNA cleavage

Cell Death (when MAD nuclease and guide nucleic acid are active)

+ Cm/Kan

Cell survival (no active cleaving)

NGS sequencing for PAM depletion

EP 3 642 334 B1

Figure 7

EP 3 642 334 B1

Figure 8

EP 3 642 334 B1

Figure 9

Figure 10A

Figure 10B

Figure 11

MAD2

MAD7

Target codons    PAM

Figure 12

EP 3 642 334 B1

Figure 13

Figure 14A — Cas9 plasmid qPCR

Figure 14B — MAD11 template qPCR results

Figure 15

Figure 16

## Figure 17A

## Figure 17B

## Figure 17C

# Figure 18

Figure 19

Figure 20

crRNA scaffold

spacer,
guide sequence

Target

guide nucleic acid

5' PAM

3' PAM

Primer
binding
site

NNNNN

NNNNN

## Figure 21A

CFU (total, 200 uL SOP comp cells) vs Impedance (kΩ)

## Figure 21B

| | Read calculation | | |
|---|---|---|---|
| Treatment | unique variants | Target reads/variant | reads required |
| Ec110 | 512 | 1000 | 5.1E+05 |
| Ec110 | 512 | 1000 | 5.1E+05 |
| Ec83* | 512 | 1000 | 5.1E+05 |
| Ec83* | 512 | 1000 | 5.1E+05 |
| Ec83* | 512 | 1000 | 5.1E+05 |
| Ec110 | 512 | 1000 | 5.1E+05 |
| Ec110 | 512 | 1000 | 5.1E+05 |
| Ec83* | 512 | 1000 | 5.1E+05 |
| Ec83* | 512 | 1000 | 5.1E+05 |
| Ec78* | 512 | 1000 | 5.1E+05 |
| Ec78* | 512 | 1000 | 5.1E+05 |
| Input plasmid | 512 | 1000 | 5.1E+05 |
| | | Total reads | 9.6E+06 |

**Figure 22B**

**Figure 22A**

Figure 23A

Figure 23B

# Figure 23C

Figure 25

TTTN, CTTN

Figure 26A

EP 3 642 334 B1

TTTN, CTTN

| ΔG | GC |
|----|----|
| 0 | 0.35 |
| -4.1 | 0.45 |
| -7.3 | 0.50 |
| -6.1 | 0.55 |
| -5.1 | 0.60 |
| -8.4 | 0.65 |
| -9.4 | 0.70 |

MAD5

Figure 26B

EP 3 642 334 B1

Figure 26C

RTTN, YCCN

| ΔG | GC |
|------|------|
| 0 | 0.35 |
| -4.1 | 0.45 |
| -7.3 | 0.50 |
| -6.1 | 0.55 |
| -5.1 | 0.60 |
| -8.4 | 0.65 |
| -9.4 | 0.70 |

MAD7   MAD2   MAD4

log$_2$ depletion score

EP 3 642 334 B1

Figure 27A

Figure 27B

MAD7 - Target5

MAD7 - Target6

MAD7 - Target7

MAD7 - Target8

**Target GC content**

| | ΔG | GC |
|---|-----|------|
| 5 | -6.1 | 0.55 |
| 6 | -5.1 | 0.60 |
| 7 | -8.4 | 0.65 |
| 8 | -9.4 | 0.70 |

Figure 28A

MAD2 - Target1

Target GC content

|   | ΔG   | GC   |
|---|------|------|
| 1 | 0    | 0.35 |
| 3 | -4.1 | 0.45 |
| 4 | -7.3 | 0.50 |

MAD2 - Target3

MAD2 - Target4

EP 3 642 334 B1

Figure 28B

| Target GC content | | |
|---|---|---|
| | ΔG | GC |
| 5 | -6.1 | 0.55 |
| 6 | -5.1 | 0.60 |
| 7 | -8.4 | 0.65 |
| 8 | -9.4 | 0.70 |

Figure 29A

Target GC content

|   | ΔG | GC |
|---|-----|------|
| 1 | 0 | 0.35 |
| 3 | -4.1 | 0.45 |
| 4 | -7.3 | 0.50 |

EP 3 642 334 B1

EP 3 642 334 B1

Figure 29B

MAD4 - Target5

MAD4 - Target6

MAD4 - Target7

MAD4 - Target8

**Target GC content**

| | ΔG | GC |
|---|---|---|
| 5 | -6.1 | 0.55 |
| 6 | -5.1 | 0.60 |
| 7 | -8.4 | 0.65 |
| 8 | -9.4 | 0.70 |

Figure 30A

Figure 30B

Figure 31B

Figure 31A

Figure 31C

Figure 32B

Off-target deletion mutations

MAD2

MAD4

MAD7

Figure 32D

Off-target insertion mutations

MAD2

MAD4

MAD7

Figure 32A

Off-target random mutations

MAD2

MAD4

MAD7

Figure 32C

Off-target mismatch mutations

MAD2

MAD4

MAD7

Figure 32F

Figure 32E

Figure 32H

Figure 32G

Figure 32I

Figure 32J

Figure 32K

## Figure 32L

Figure 32M

Figure 32N

Figure 32O

Figure 32P

Figure 33

## Figure 34A

5' repeat          Pseudo-knot

```
                          .....................((((((.....))))))..
MAD12      -GUC-AA-AAGACCUUUUUAAUU  UCU-U       U  35
MAD52      -GUC-AAUAAGACUCAUUUAAUU  UUC-G       U  36
MAD11      --GUCUGGCCCCAAAUUUUAAUU  UGU-U       U  36
MAD6       --GUCUAGGUACUCUCUUUAAUU  UAU-U       U  36
MAD63      -GGUCUAGGUACUCUCUUUAAUU  UAU-U       U  37
MAD82      -G-UCUAAGAACUUUAAAUAAUU  UGU-U       U  36
MAD50      -G-UUGAGUAACCUUAAAUAAUU  UGU-U       U  36
MAD92      -G-UCAAAUCGUUUUUAAAAAUU  UAU-U       U  36
MAD7       -G-UUAAGUUAUAUAGAAUAAUU  UGU-U       U  36
MAD44      -G-UUAAGUAAUAUAAAAGAAUU  UAU-U       U  36
MAD89      -G-CUUAAGACUUAGAAAUAAUU  UAU-U       U  36
MAD10      -C-UCUACAACUGAUAAAGAAUU  UUU-U       U  36
MAD40      -G-UUUAAUCCAUAUAUAAAAUU  UUU-U       U  36
MAD43      -G-UCUAAUACCCAUAUAAAAUU  UUU-U       U  36
MAD28      -C-UCUAAUACCUCUAUAAGAUU  UUU-U       U  36
MAD33      -C-UCUAAAACCCCCAACAAAUU  UUU-U       U  36
MAD5       -G-UCUAAAACUCAUUCAGAAUU  UAG-U       U  36
MAD8       -G-UUUAAAACCACUUUAAAAUU  UAU-U       U  36
MAD30      -G-UUCUGCAUCCAUAUUGGAUU  UUU-U       U  36
MAD32      -G-UUUAAAAGUCCUAUUGGAUU  UUU-U       U  36
MAD4       -C-UCUAGCAGGCCUGGCAAAUU  UGU-U       U  36
MAD108     -A-UCGAACAGACUCAUAAAAUU  UGU-U       U  36
MAD41      -GUU--AAGUAACCUAAAUAAUU  UGUGU       U  36
MAD51      -GUU--AAGUAACCUAAAUAAUU  UGUGU       U  36
MAD48      AGUUUGAAUAACCUUAAAUAAUU  UU--U       U  37
MAD57      --GUUAAAUAAUAAGAAAAAAUU  UA-GU       UU 37
MAD38      --GUCUAAGACUUAAAGAUAAUU  UACUAU-U    U  36
MAD3       ----CUCAAAACUCAUUCGAAUC  UCUUU       U  35
                  ........10........20........30........4
```

## Figure 34B

```
crMAD3    --GAAUCUCUACUCUUUGUAGAU
crMAD4    -CAAAUUUCUACUGU-UGUAGAU
crMAD5    -AGAAUUUCUACUAG-UGUAGAU
crMAD7    AAUAAUUUCUACUGU-UGUAGAU
crMAD10   -AGAAUUUCUACUUU-UGUAGAU
crMAD11   -UUAAUUUCUACUGU-UGUAGAU
crMAD25   -UUAAUUUCUACUCU-UGUAGAU
crMAD12v2 -UUAAUUUCUACUAU-UGUAGAU
crMAD12   -----AAUUUCUACUAU-UGUAGAU
```

Figure 35A

Figure 35B

MAD2

EP 3 642 334 B1

MAD4

Figure 35C

Figure 36A

Figure 36B

Figure 36C

Figure 36D

Figure 36E

Figure 36F

crUAGU MAD4

crUUUU MAD4

crUCUUU MAD4

EP 3 642 334 B1

EP 3 642 334 B1

Figure 37

116

## Figure 38A

*E. coli codons*

*Human codon optimized*

## Figure 38B

Scaffold sequence     Targeting sequence

42-mer    UUAAUUUCUACUCUUGUAGAUNNNNNNNNNNNNNNNNNNNNN

56-mer    GUCAAAAGACCUUUUUAAUUUCUACUCUUGUAGAUNNNNNNNNNNNNNNNNNNNNN

## Figure 39A

## Figure 39B

## Figure 39C

| Guide RNA name | PAM | Target sequence | PAM | %GC | DNA strand |
|---|---|---|---|---|---|
| PPIBgRNA-14 | CTTA | GCTACAGGAGAGGTAAGTGGC | | 57.1 | Forward |
| PPIBgRNA-26 | | GGGTGATCTTTGGTCTCTTCG | GAAA | 52.4 | Reverse |
| PPIBgRNA-15 | | GGGTCCATTAGCTGTTTAGAG | CAAA | 47.6 | Reverse |
| PPIBgRNA-17 | | GTAGTCCAAGGAGGGTATAGA | TAAG | 47.6 | Reverse |
| PPIBgRNA-4 | TTTA | CCGTCACCAAAATCAGATTCA | | 42.9 | Forward |
| PPIBgRNA-10 | TTTG | ACCTACGAATTGGAGATGAAG | | 42.9 | Forward |
| PPIBgRNA-21 | | TCAGATTCAGAACCACTTCTC | TAAA | 42.9 | Reverse |
| PPIBgRNA-8 | TTTA | AAGAAGCTAAGTTGGAAATGG | | 38.1 | Forward |
| PPIBgRNA-24 | | CCCTTTAAAGAAGCTAAGTTG | GAAA | 38.1 | Reverse |
| PPIBgRNA-25 | | TTTGACCTACGAATTGGAGAT | GAAG | 38.1 | Reverse |

## Figure 39D

| Guide RNA name | PAM | Target sequence | PAM | %GC | DNA strand |
|---|---|---|---|---|---|
| DNMT3BgRNA-13 | | GGTGGGTTGGGCTGGAGGTTT | CAAA | 61.9 | Reverse |
| DNMT3BgRNA-14 | | CCTGCTGAATTACTCACGCCC | CAAG | 57.1 | Reverse |
| DNMT3BgRNA-6 | TTTA | CCACCTGCTGAATTACTCACG | | 52.4 | Forward |
| DNMT3BgRNA-10 | | GTAAATCCTCAGCCCACAAGG | GAAA | 52.4 | Reverse |
| DNMT3BgRNA-1 | | GGTGCTGCCTACGCTCCATAG | TAAA | 47.6 | Reverse |
| DNMT3BgRNA-2 | TTTC | AAATGAACCCTGCGCTGTCAT | | 47.6 | Forward |
| DNMT3BgRNA-9 | | CCATAGTAAATCCTCAGCCCA | CAAG | 47.6 | Reverse |
| DNMT3BgRNA-4 | CTTC | TTCGAATTTTACCACCTGCTG | | 42.9 | Forward |
| DNMT3BgRNA-8 | TTTG | AGAATGTTGTAGCCATGAAGG | | 42.9 | Forward |
| DNMT3BgRNA-15 | | GTTTGAGAATGTTGTAGCCAT | GAAG | 38.1 | Reverse |

## Figure 40

In vitro cutting assay

Figure 41

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160208243 A
- US 20140068797 A
- US 8697359 B
- US 81573004
- US 20040171156 A1
- US 5049386 A
- US 4946787 A
- US 4897355 A
- WO 9117424 A
- WO 9116024 A
- US 4186183 A
- US 4217344 A
- US 4235871 A
- US 4261975 A
- US 4485054 A
- US 4501728 A
- US 4774085 A
- US 4837028 A
- US 9405700 W
- US 4797368 A
- WO 9324641 A
- US 5173414 A
- US 61736465
- US 61721283
- US 5210015 A
- US 5445934 A
- WO 9703211 A
- WO 9639154 A

**Non-patent literature cited in the description**

- **ZETSCHE et al.** *Cell,* 22 October 2015, vol. 163 (3), 759-71
- **KLEINSTIVER B P et al.** Engineered CRISPR-Cas9 nucleases with altered PAM specificities. *Nature,* 23 July 2015, vol. 523 (7561), 481-5
- **NAKAMURA, Y. et al.** Codon usage tabulated from the international DNA sequence databases: status for the year 2000. *Nucl. Acids Res.,* 2000, vol. 28, 292
- **SHALEK et al.** *Nano Letters,* 2012
- **PARDRIDGE et al.** *Cold Spring Harb Protoc,* 2010
- **ANDERSON.** *Science,* 1992, vol. 256, 808-813
- **NABEL ; FEIGNER.** *TIBTECH,* 1993, vol. 11, 211-217
- **MITANI ; CASKEY.** *TIBTECH,* 1993, vol. 11, 162-166
- **DILLON.** *TIBTECH,* 1993, vol. 11, 167-175
- **MILLER.** *Nature,* 1992, vol. 357, 455-460
- **VAN BRUNT.** *Biotechnology,* 1988, vol. 6 (10), 1149-1154
- **VIGNE.** *Restorative Neurology and Neuroscience,* 1995, vol. 8, 35-36
- **KREMER ; PERRICAUDET.** *British Medical Bulletin,* 1995, vol. 51 (1), 31-44
- **HADDADA et al.** Current Topics in Microbiology and Immunology. 1995
- **YU et al.** *Gene Therapy,* 1994, vol. 1, 13-26
- **CRYSTAL.** *Science,* 1995, vol. 270, 404-410
- **BLAESE et al.** *Cancer Gene Ther.,* 1995, vol. 2, 291-297
- **BEHR et al.** *Bioconjugate Chem.,* 1994, vol. 5, 382-389
- **REMY et al.** *Bioconjugate Chem.,* 1994, vol. 5, 647-654
- **GAO et al.** *Gene Therapy,* 1995, vol. 2, 710-722
- **AHMAD et al.** *Cancer Res.,* 1992, vol. 52, 4817-4820
- **BUCHSCHER et al.** *J. Virol.,* 1992, vol. 66, 2731-2739
- **JOHANN et al.** *J. Virol.,* 1992, vol. 66, 1635-1640
- **SOMMNERFELT et al.** *Virol.,* 1990, vol. 176, 58-59
- **WILSON et al.** *J. Virol.,* 1989, vol. 63, 2374-2378
- **MILLER et al.** *J. Virol.,* 1991, vol. 65, 2220-2224
- **WEST et al.** *Virology,* 1987, vol. 160, 38-47
- **KOTIN.** *Human Gene Therapy,* 1994, vol. 5, 793-801
- **MUZYCZKA, J.** *Clin. Invest.,* 1994, vol. 94, 1351
- **TRATSCHIN et al.** *Mol. Cell. Biol.,* 1985, vol. 5, 3251-3260
- **TRATSCHIN et al.** *Mol. Cell. Biol.,* 1984, vol. 4, 2072-2081
- **HERMONAT ; MUZYCZKA.** *PNAS,* 1984, vol. 81, 6466-6470

- **SAMULSKI et al.** *J. Virol.,* 1989, vol. 63, 03822-3828
- **GREEN ; SAMBROOK et al.** Nonradioactive in Situ Hybridization Application Manual. Boehringer, 2014
- **CHAN-HUI et al.** *Clinical Immunology,* 2003, vol. 111, 162-174
- **GREER.** *Science,* 1985, vol. 228, 1055
- **BLUNDELL et al.** *Eur J Biochem,* 1988, vol. 172, 513
- **DEY F ; CLIFF ZHANG Q ; PETREY D ; HONIG B.** Toward a "structural BLAST": using structural relationships to infer function. *Protein Sci.,* April 2013, vol. 22 (4), 359-66
- Overview of principles of hybridization and the strategy of nucleic acid probe assay. **TIJSSEN.** Laboratory Techniques In Biochemistry And Molecular Biology-Hybridization With Nucleic Acid Probes Part I. Elsevier, 1993
- **DEVEREUX et al.** *Nucleic Acids Research,* 1984, vol. 12, 387
- **ATSCHUL et al.** *J. Mol. Biol.,* 1990, 403-410
- **DEVEREUX et al.** *Nuc. Acids Research,* 1984, vol. 12, 38
- **AUSUBEL et al.** Short Protocols in Molecular Biology. 1999
- **ALTSCHUL et al.** *J. Mol. Biol.,* 1990, 403-410
- **AUSUBEL et al.** *Short Protocols in Molecular Biology,* 1999, 7-58, 7-60
- *FEMS Microbiol Lett.,* 1999, vol. 174 (2), 247-50
- *FEMS Microbiol Lett.,* 1999, vol. 177 (1), 187-8
- **HIGGINS D G ; SHARP P M.** *Gene,* 1988, vol. 73 (1), 237-244
- **LIVINGSTONE C. D. ; BARTON G. J.** Protein sequence alignments: a strategy for the hierarchical analysis of residue conservation. *Comput. Appl. Biosci.,* 1993, vol. 9, 745-756
- **TAYLOR W. R.** The classification of amino acid conservation. *J. Theor. Biol.,* 1986, vol. 119, 205-218
- **SIMON R J et al.** *PNAS,* 1992, vol. 89 (20), 9367-9371
- **HORWELL D C.** *Trends Biotechnol.,* 1995, vol. 13 (4), 132-134
- **GREEN ; SAMBROOK.** Molecular Cloning: A Laboratory Manual. Cold Spring Harbor Laboratory Press, 2014
- CURRENT PROTOCOLS IN MOLECULAR BIOLOGY. 2017
- the series METHODS IN ENZYMOLOGY. Academic Press, Inc,
- PCR 2: A PRACTICAL APPROACH. 1995
- ANTIBODIES, A LABORATORY MANUAL. 2014
- CULTURE or ANIMAL CELLS : A MANUAL OF BASIC TECHNIQUE. 2016